# EUROPEAN PATENT SPECIFICATION

(11) **EP 2 449 488 B1**
(45) Date of publication and mention of the grant of the patent: **14.01.2015**
(21) Application number: 10726084.6
(22) Date of filing: 23.06.2010
(51) Int. Cl.: G06F 19/24

(54) **METHOD FOR NORMALIZATION IN METABOLOMICS ANALYSIS METHODS WITH ENDOGENOUS REFERENCE METABOLITES**
VERFAHREN ZUR NORMALISIERUNG BEI METABOLOMANALYSEVERFAHREN MIT ENDOGENEN REFERENZMETABOLITEN
Procédé de normalisation dans les procédés d'analyse métabolomiques avec des métabolites de référence endogène

(30) Priority: 02.07.2009 EP 09164410
(43) Date of publication of application: 09.05.2012
(73) Proprietor: BIOCRATES Life Sciences AG, 6020 Innsbruck (AT)
(72) Inventor: DEIGNER, Hans-Peter, 68623 Lampertheim (DE); KOHL, Matthias, 95326 Kulmbach (DE); KELLER, Matthias, 45219 Essen (DE); KOAL, Therese, A-6020 Innsbruck (AT); ENOT, David, F-14480 Creully (FR)
(74) Representative: Simandi, Claus
(86) International application number: PCT/EP2010/058911
(87) International publication number: WO 2011/000753

(56) References cited:
- SYSI-AHO MARKO ET AL: "Normalization method for metabolomics data using optimal selection of multiple internal standards" BMC BIOINFORMATICS, vol. 8, March 2007 (2007-03), XP021021881 ISSN: 1471-2105
- WARRACK B M ET AL: "Normalization strategies for metabonomic analysis of urine samples" JOURNAL OF CHROMATOGRAPHY B: ANALYTICAL TECHNOLOGIES IN THE BIOMEDICAL AND LIFE SCIENCES 20090215 ELSEVIER NL, vol. 877, no. 5-6, 15 February 2009 (2009-02-15), pages 547-552, XP025928077
- ENOT DAVID P ET AL: "Preprocessing, classification modeling and feature selection using flow injection electrospray mass spectrometry metabolite fingerprint data." NATURE PROTOCOLS 2008, vol. 3, no. 3, 2008, pages 446-470, XP001539671 ISSN: 1750-2799
- DIXON S J ET AL: "Pattern recognition of gas chromatography mass spectrometry of human volatiles in sweat to distinguish the sex of subjects and determine potential discriminatory marker peaks" CHEMOMETRICS AND INTELLIGENT LABORATORY SYSTEMS 20070615 NL, vol. 87, no. 2, 15 June 2007 (2007-06-15), pages 161-172, XP022081779 ISSN: 0169-7439
- ANDERSEN CLAUS LINDBJERG ET AL: "Normalization of real-time quantitative reverse transcription-PCR data: a model-based variance estimation approach to identify genes suited for normalization, applied to bladder and colon cancer data sets." CANCER RESEARCH 1 AUG 2004, vol. 64, no. 15, 1 August 2004 (2004-08-01), pages 5245-5250, XP008112436 ISSN: 0008-5472
- VANDESOMPELE JO ET AL: "Accurate normalization of real-time quantitative RT-PCR data by geometric averaging of multiple internal control genes." GENOME BIOLOGY 18 JUN 2002, vol. 3, no. 7, 18 June 2002 (2002-06-18), XP008027995 ISSN: 1465-6914

## Description

The present invention relates to a method for normalization of intensity data corresponding to amounts and/or concentrations of selected target metabolites in a biological sample of a mammalian subject, wherein said intensity data are obtained by a metabolomics analysis method with one or a plurality of endogenous reference metabolites in accordance with claim 1, and to a use of one or a plurality of compounds or derivatives thereof, wherein said compounds have a molecular mass less than 1500 Da, as endogenous reference metabolites in metabolomics analysis methods, in accordance with claim 13.

### FIELD OF THE INVENTION

The present invention relates to the field of metabolomics. More specifically, the present invention concerns a method for normalizing signals to be compared in assays for metabolites. This novel method relies on the use of endogenous metabolite concentrations as an internal standard and allows determination of the concentrations and relative abundance as well as direct comparisons between any samples.

The invention thus relates to the use of metabolite concentrations and the comparison of metabolite levels between different species, tissues and between different cells. The invention provides the identity and use of reference, control or normalization metabolites, the level of which remains consistent in individual cells, even under different conditions, as well as among body liquid, cells and tissue from different samples, species and origins.

### BACKGROUND OF THE INVENTION

In metabolomics, the term normalization refers to a data adjustment step that follows signal extraction and processing and precedes data dissemination and subsequent statistical treatment (Preprocessing, classification modeling and feature selection using flow injection electrospray mass spectrometry metabolite fingerprint data, D.P. Enot, W. Lin, M. Beckmann, D. Parker, D.P. Overy and J. Draper: Nature Protocols, 2008, 3, 446-470; Metabolomics by numbers: acquiring and understanding global metabolite data, R. Goodacre, S. Vaidyanathan, W.B. Dunn, .G. Harrigan and D.B. Kell: TRENDS in Biotechnology 2004, 22(5) 245-252). Normalization methods in metabolomics data to compare data generated at different days, on different machines, in various dilutions etc. are still a matter of debate, far from a gold standard or even from a uniform recommendation.
As the primary goal of metabolomics is the study of metabolite changes in response to environmental and genetic changes, normalization is a crucial step to make measurements comparable where obscuring and unrelated sources of variance are excluded. Typically, inter sample variability originates from sample concentration and homogeneity differences, loss of sensitivity and drift of the analytical system or sample degradation over time. It becomes a real challenge when multiple experiments are considered and when metabolite measurements are originating from several experimental procedures and from different analytical platforms (Enot et al. 2008).

Depending on the experimental design, there are some useful approaches for calculating normalization factors. For instance, one could add a number of controls in increasing but equimolar concentrations to the sample, prior or after chemical derivatization, and the sum of the intensities for these spots should be equal. Alternatively, mixtures or extracts containing constant amounts of compounds such as samples from one batch of plasma, could be added. Measured intensities for added equimolar controls should behave similarly.

More general methods make use of control samples on a plate to control variations in overall plate quality (e.g. standard batch and method of standard preparation) or measuring differences.

Applicable normalization strategies are based on some underlying assumptions regarding the data and the strategies used for each experiment. These strategies must therefore be adjusted to reflect both the system under study and the experimental design. A primary assumption is that for some added set of controls, the ratio of measured concentrations averaged over the set should be close to unity.

The prior art for normalizing metabolomics data can be summarized into three main categories:
1- A first class of strategy encapsulates methods that infers a samplewise bias factor, also known as dilution or scaling factor, from the data themselves (A note on normalization of biofluid 1D 1H-NMR data, R.J.O. Torgrip, K.M. Åberg1, E. Alm, I. Schuppe-Koistinen and J. Lindberg: Metabolomics, 2008 4(2), 114-121). The simplest approach is global normalisation where the scaling factor is the sum (or related) of all measurements across the sample (aka constant sum, integrale, total io count normalisation). Limitations of this naive approach has been widely discussed (Torgrip et al., 2008; Probabilistic quotient normalization as robust method to account for dilution of complex biological mixtures. Application in 1 H NMR metabolomics, F. Dieterle, A. Ross, G. Schlotterbeck, H. Senn: Anal Chem., 2006, 78(13), 4281-90) and alternative approaches have proposed to derive a more adequate scaling factor estimate by introducing class information (Enot et al. 2008), subselection of peak/signals (Normalization strategies for metabolomic analysis of urine samples, B. M. Warrack, S. Hnatyshyn, K.-H. Ott, M.D. Reily, M. Sanders, H. Zhang, and D.M. Drexler: Journal of Chromatography B, 2009, 877(5-6), 547-552; Normalization Regarding Non-Random Missing Values in High-Throughput Mass Spectrometry Data, P. Wang, H. Tang, H. Zhang, J. Whiteaker, A. G. Paulovich, and M. Mcintosh Pacific Symposium on Biocomputing, 2006, 11:315-326) or mapping intensity distribution to a reference sample profile (Torgrip et al. 2008, Dieterle et al. 2006). Many such methods are in the field of NMR (US Pat. No. 7,277,807 B2, Dieterle et al., 2006). Regardless of the sophistication of the technique employed and its usefulness for solving a specific biological question, data generated remain in a form of a dimensionless and experiment dependent quantity that cannot be efficiently transferred and compared between varying experimental set up or application areas.
2- A second normalization family uses information from the biological context to adjust sample concentrations (Warrack et al. 2009). As such, creatinine, urine volume or osmolality at time of sampling are commonly used in urine based studies and plant extract or tissue weight may also employ to scale sample measurements. In addition to the availability of such information in practice and inherent errors related to their measurement, the application of experimental/clinical parameter for normalization cannot be warranted when the study addresses biochemical processes that may induce dramatic changes in their estimation (e.g. kidney impairment).
3- Finally, absolute quantification of compound concentrations by means of calibration to single or multiple internal standards is the most reliable approach to both minimize inter individual variance and to compare datasets originating from multiple sites and experiments (Normalization method for metabolomics data using optimal selection of multiple internal standards, M. Sysi-Aho, M. Katajamaa, L. Yetukuri1 and M. Ore 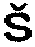 i 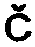 : BMC Bioinformatics 2007, 8:93). However, its implementation can become a daunting task in a high throughput metabolite profiling context due to the chemical diversity:
   i) use of a single internal standard does warrant its efficiency to a wide collection compounds
   ii) cost and commercial availability of a collection of internal standards and
   iii) matrix effects that require ad hoc analytical procedures.

Additional options in quantitative metabolomic profiling of a biological sample require a separation-molecular ID process, such as gas chromatography-mass spectrometry ('GC-MS') and the derivatization of the original sample (WO2007/008307). A data correction and validation strategy provides for a weighted average of metabolite derivatives after derivatization of an original metabolite. In this profiling method a sample is combined with a derivatizing agent to produce derivatives and a separation-molecular ID and quantification process is performed on the derivatives to obtain corresponding peak areas, comprising: measuring the peak areas of the derivatives.

### References:

1.) Normalization method for metabolomics data using optimal selection of multiple internal standards
   M. Sysi-Aho, M. Katajamaa, L. YetukurM and M. Oresic BMC Bioinformatics 2007, 8:93
   M. Sysi-Aho et al disclose normalization method for metabolomics data using optimal selection of multiple internal standards, wherein labelled internal standards are added to a sample prior to extraction and analysis in contrast to the present invention.
2.) Normalization strategies for metabolomic analysis of urine samples
   B. M. Warrack, S. Hnatyshyn, K.-H. Ott, M. D. Reily, M. Sanders, H. Zhang, and D. M. Drexler Journal of Chromatography B, 2009, 877(5-6), 547-552
   B. M. Warrack et al refer to normalization strategies for metabolomic analysis of urine samples, wherein creatinine alone is determined without relating to a plurality of housekeeper metabolites in contrast to the present invention.
3.) Preprocessing, classification modelling and feature selection using flow injection electrospray mass spectrometry metabolite fingerprint data
   D. P. Enot, W. Lin, M. Beckmann, D. Parker, D. P. Overy and J. Draper Nature Protocols, 2008, 3, 446-470
4.) Metabolomics by numbers: acquiring and understanding global metabolite data
   R. Goodacre, S. Vaidyanathan, W. B. Dunn, G. Harrigan and D. B. Kell TRENDS in Biotechnology 2004, 22(5) 245-252
5.) Normalization Regarding Non-Random Missing Values in High-Throughput Mass Spectrometry Data
   P. Wang, H. Tang, H. Zhang, J. Whiteaker, A. G. Paulovich, and M. Mcintosh Pacific Symposium on Biocomputing, 2006, 11:315-326
6.) Large-Scale Human Metabolomics Studies: A Strategy for Data (Pre-) Processing and Validation S. BijlsmaJ. Bobeldijk, E. R. Verheij, R. Ramaker, S. Kochhar, I. A. Macdonald, B. van Ommen and A.K. Smilde Anal. Chem., 2006 78 (2), 567-574
7.) A note on normalization of biofluid 1 D 1 H-NMR data
   R.J. O. Torgrip, K.M. Abergi , E. Aim, I. Schuppe-Koistinen and J. Lindberg Metabolomics, 2008 4(2), 114-121
8.) Probabilistic quotient normalization as robust method to account for dilution of complex biological mixtures. Application in 1 H NMR metabonomics.
   F. Dieterle, A. Ross, G. Schlotterbeck, H. Senn Anal Chem., 2006, 78(13), 4281-90
9.) WO2007/008307: Data correction, normalization and validation for quantitative high-throughput metabolomic profiling

To summarize, the prior art currently does not present reliable normalization methods in metabolomics analysis methods.

Thus, it is the object of the present invention, to provide a reliable normalization for metabolomics analysis methods.

This object is achieved by a method for normalization in accordance with claim 1 and a use of endogenous reference metabolites in accordance with claim 12.

In particular, the present invention relates to:
Method for normalization of intensity data corresponding to amounts and/or concentrations of selected target metabolites in a biological sample of a mammalian subject, wherein said intensity data are obtained by a metabolomics analysis method comprising the generation of intensity data for the quantitation of endogenous metabolites by mass spectrometry (MS), in particular MS-technologies such as Matrix Assisted Laser Desorption/Ionisation (MALDI), Electro Spray Ionization (ESI), Atmospheric Pressure Chemical Ionization (APCI), ¹H-, ¹³C-and/or ³¹P-Nuclear Magnetic Resonance spectroscopy (NMR), optionally coupled to MS, determination of metabolite concentrations by use of MS-technologies and/or methods coupled to separation, in particular Liquid Chromatography (LC-MS), Gas Chromatography (GC-MS), or Capillary Electrophoresis (CE-MS), with a plurality of endogenous reference metabolites, comprising
carrying out at least one in vitro metabolomics analysis method of said selected target metabolites in said biological sample;
simultaneously carrying out in the same sample a quantitative analysis of lysophosphatidylcholines (monoacylphosphatidylcholines) having from 1 to 30 carbon atoms in the acyl residue; lysophosphatidylcholines having from 3 to 30 carbon atoms in the acyl residue and having 1 to 6 double bonds in the acyl residue;
phosphatidylcholines (diacylphosphatidylcholines) having a total of from 1 to 50 carbon atoms in the acyl residues; phatidylcholines having a total from 3 to 50 carbon atoms in the acyl residues and having a total of 1 to 8 double bonds in the acyl residues;
sphingolipids, in particular sphingomyelines having a total number of carbon atoms in the acyl chains from 10 to 30; sphingomyelines having a total number of carbon atoms in the acyl chains from 10 to 30 and 1 to 5 double bonds; hydroxysphinogomyelines having a total number of carbon atoms in the acyl residues from 10 to 30; hydroxysphirigoyelines having a total number of carbon atoms in the acyl residues from 10 to 30 and 1 to 5 double bonds;
prostaglandines, namely 6-keto-prostaglandin F1 alpha, prostaglandin D2;
putrescine;
and wherein
said detected intensities of said selected target metabolites each are related to said intensities of said endogenous reference metabolites.

In a preferred method according to the present invention, the plurality of intensities of the target and endogenous reference metabolites are subjected to a mathematical preprocessing, in particular transformations such as applying logarithms, generalized logarithms, power transformations.

In a preferred embodiment of the present invention, said plurality of intensities of the endogenous reference metabolites are aggregated to one reference value.

In the latter case, it might be preferred that the plurality of intensities of the endogenous reference metabolites are aggregated to one reference value by calculation of geometric mean value, arithmetic mean value, median value, weighted arithmetic mean value.

Preferably, a ratio can be formed by each of the intensities of the target metabolites and the determined reference value in case of linear intensities, or the determined reference value is subtracted from each target metabolite intensity in case of logarithmic intensities.

In general, for the purpose of the present invention, said metabolomics analysis method comprises the generation of intensity data for the quantitation of endogenous metabolites by mass spectrometry (MS), in particular MS-technologies such as Matrix Assisted Laser Desorption/lonisation (MALDI), Electro Spray Ionization (ESI), Atmospheric Pressure Chemical Ionization (APCI), ¹H-, ¹³C- and/or ³¹P- Nuclear Magnetic Resonance spectroscopy (NMR), optionally coupled to MS, determination of metabolite concentrations by use of MS-technologies and/or methods coupled to separation, in particular Liquid Chromatography (LC-MS), Gas Chromatography (GC-MS), or Capillary Electrophoresis (CE-MS), which technologies are well known to the skilled person.

A preferred embodiment of the present invention lies in endogenous reference metabolites which are selected from the group consisting of:
15((S)-hydroxy-5Z,8Z,11Z,13E-eicosatetraenoic acid
6-keto-Prostaglandin F1 alpha
asymmetrical Dimethylarginin
Arginine
PTC-Arginine
Aspartic acid
Carnitine (free)
Decanoylcarnitine [Caprylcarnitine] (Fumarylcarnitine)
Decenoylcarnitine
Decadienoylcarnitine
Dodecanoylcarnitine [Laurylcarnitine]
Dodecenoylcarnitine
Dodecanedioylcarnitine
Tetradecanoylcarnitine
Tetradecenoylcarnitine [Myristoleylcarnitine]
3-Hydroxytetradecenoylcarnitine [3-Hydroxymyristoleylcarnitine]
3-Hydroxytetradecadienoylcarnitine
3-Hydroxytetradecanoylcarnitine [Hydroxymyristylcarnitine]
Hexadecenoylcarnitine [Palmitoleylcarnitine]
3-Hydroxyhexadecenoylcarnitine [3-Hydroxypalmitoleylcarnitine]
Hexadecadienoylcarnitine
3-Hydroxyhexadecadienoylcarnitine
3-Hydroxyhexadecanolycarnitine [3-Hydroxypalmitoylcarnitine]
Octadecanoylcarnitine [Stearylcarnitine]
Octadecenoylcarnitine [Oleylcarnitine] 3-Hydroxyoctadecenoylcarnitine [3-Hydroxyoleylcarnitine]
Acetylcarnitine
Propenoylcarnitine
Hydroxypropionylcarnitine
Butenoylcarnitine
3-Hydroxybutyrylcarnitine / Malonylcarnitine
Isovalerylcarnitine / 2-Methylbutyrylcarnitine / Valerylcarnitine
Tiglylcarnitine / 3-Methyl-crotonylcarnitine
Glutarylcarnitine / Hydroxycaproylcarnitine
Glutarylcarnitine / Hydroxycaproylcarnitine
Methylglutarylcarnitine
3-Hydroxyisovalerylcarnitine / 3-Hydroxy-2-methylbutyryl
Hexanoylcarnitine [Caproylcarnitine]
Hexenoylcarnitine
Pimelylcarnitine
Octanoylcarnitine [Caprylylcarnitine]
Octenoylcarnitine
Nonanoylcarnitine [Pelargonylcarnitine]
Citrulline
Creatinine
Glutamine
PTC-Glutamine
Glutamate
Histidine
PTC-Histidine
Kynurenine
Leucine
Lysophosphatidylcholine with acyl residue C14:0
Lysophosphatidylcholine with acyl residue C16:0
Lysophosphatidylcholine with acyl residue C16:1
Lysophosphatidylcholine with acyl residue C18:0
Lysophosphatidylcholine with acyl residue C18:1
Lysophosphatidylcholine with acyl residue C18:2
Lysophosphatidylcholine with acyl residue C20:3
Lysophosphatidylcholine with acyl residue C20:4
Lysophosphatidylcholine with acyl residue C24:0
Lysophosphatidylcholine with acyl residue C26:0
Lysophosphatidylcholine with acyl residue C26:1
Lysophosphatidylcholine with acyl residue C28:0
Lysophosphatidylcholine with acyl residue C28:1
Lysophosphatidylcholine with acyl residue C6:0
PTC-Methionine
Ornithine
PTC-Ornithine
Phosphatidylcholine with diacyl residue sum C24:0
Phosphatidylcholine with diacyl residue sum C26:0
Phosphatidylcholine with diacyl residue sum C28:1
Phosphatidylcholine with diacyl residue sum C30:0
Phosphatidylcholine with diacyl residue sum C30:2
Phosphatidylcholine with diacyl residue sum C32:0
Phosphatidylcholine with diacyl residue sum C32:1
Phosphatidylcholine with diacyl residue sum C32:2
Phosphatidylcholine with diacyl residue sum C32:3
Phosphatidylcholine with diacyl residue sum C34:1
Phosphatidylcholine with diacyl residue sum C34:2
Phosphatidylcholine with diacyl residue sum C34:3
Phosphatidylcholine with diacyl residue sum C34:4
Phosphatidylcholine with diacyl residue sum C36:0
Phosphatidylcholine with diacyl residue sum C36:1
Phosphatidylcholine with diacyl residue sum C36:2
Phosphatidylcholine with diacyl residue sum C36:3
Phosphatidylcholine with diacyl residue sum C36:4
Phosphatidylcholine with diacyl residue sum C36:5
Phosphatidylcholine with diacyl residue sum C36:6
Phosphatidylcholine with diacyl residue sum C38:0
Phosphatidylcholine with diacyl residue sum C38:1
Phosphatidylcholine with diacyl residue sum C38:3
Phosphatidylcholine with diacyl residue sum C38:4
Phosphatidylcholine with diacyl residue sum C38:5
Phosphatidylcholine with diacyl residue sum C38:6
Phosphatidylcholine with diacyl residue sum C42:1
Phosphatidylcholine with diacyl residue sum C40:3
Phosphatidylcholine with diacyl residue sum C40:4
Phosphatidylcholine with diacyl residue sum C40:5
Phosphatidylcholine with diacyl residue sum C40:6
Phosphatidylcholine with diacyl residue sum C42:0
Phosphatidylcholine with diacyl residue sum C42:1
Phosphatidylcholine with diacyl residue sum C42:2
Phosphatidylcholine with diacyl residue sum C42:4
Phosphatidylcholine with diacyl residue sum C42:5
Phosphatidylcholine with diacyl residue sum C42:6
Phosphatidylcholine with acyl-alkyl residue sum C30:0
Phosphatidylcholine with acyl-alkyl residue sum C30:1
Phosphatidylcholine with acyl-alkyl residue sum C34:0
Phosphatidylcholine with acyl-alkyl residue sum C34:1
Phosphatidylcholine with acyl-alkyl residue sum C34:2
Phosphatidylcholine with acyl-alkyl residue sum C34:3
Phosphatidylcholine with acyl-alkyl residue sum C36:0
Phosphatidylcholine with acyl-alkyl residue sum C36:1
Phosphatidylcholine with acyl-alkyl residue sum C36:2
Phosphatidylcholine with acyl-alkyl residue sum C36:3
Phosphatidylcholine with acyl-alkyl residue sum C36:4
Phosphatidylcholine with acyl-alkyl residue sum C38:0
Phosphatidylcholine with acyl-alkyl residue sum C38:1
Phosphatidylcholine with acyl-alkyl residue sum C38:2
Phosphatidylcholine with acyl-alkyl residue sum C38:3
Phosphatidylcholine with acyl-alkyl residue sum C38:4
Phosphatidylcholine with acyl-alkyl residue sum C38:5
Phosphatidylcholine with acyl-alkyl residue sum C38:6
Phosphatidylcholine with acyl-alkyl residue sum C40:0
Phosphatidylcholine with acyl-alkyl residue sum C40:1
Phosphatidylcholine with acyl-alkyl residue sum C40:2
Phosphatidylcholine with acyl-alkyl residue sum C40:3
Phosphatidylcholine with acyl-alkyl residue sum C40:4
Phosphatidylcholine with acyl-alkyl residue sum C40:5
Phosphatidylcholine with acyl-alkyl residue sum C40:6
Phosphatidylcholine with acyl-alkyl residue sum C42:0
Phosphatidylcholine with acyl-alkyl residue sum C42:1
Phosphatidylcholine with acyl-alkyl residue sum C42:3
Phosphatidylcholine with acyl-alkyl residue sum C42:4
Phosphatidylcholine with acyl-alkyl residue sum C42:5
Phosphatidylcholine with acyl-alkyl residue sum C44:5
Phosphatidylcholine with acyl-alkyl residue sum C44:6
Prostaglandin D2
Phenylalanine
PTC-Phenylalanine
Proline
PTC-Proline
Putrescine
Serine
PTC-Serine
Hydroxysphingomyeline with acyl residue sum C14:1
Hydroxysphingomyelin with acyl residue sum C16:1
Hydroxysphingomyeline with acyl residue sum C22:1
Hydroxysphingomyeline with acyl residue sum C22:2
Hydroxysphingomyelin with acyl residue sum C24:1
Sphingomyeline with acyl residue sum C16:0
Sphingomyeline with acyl residue sum C16:1
Sphingomyeline with acyl residue sum C18:0
Sphingomyeline with acyl residue sum C18:1
sphingomyelin with acyl residue sum C20:2
Sphingomyeline with acyl residue sum C22:3
Sphingomyeline with acyl residue sum C24:0
Sphingomyeline with acyl residue sum C24:1
Sphingomyeline with acyl residue sum C26:0
Sphingomyeline with acyl residue sum C26:1
Succinic acid (succinate)
Total dimethylarginine: sum ADMA + SDMA
Tryptophan
PTC-Tryptophan
Tyrosine
Valine
PTC- Valine
Leucine + Isoleucine
wherein the designation "residue Cn:m" or "residue sum Cn:m" represents the chain length of the acyl/alkyl residue(s), n represents the number of total carbon atoms in the acyl/alkyl residue(s), and m represents the number of total double bonds in the residue(s).

Furthermore, it is particularly preferred to use such metabolites as the endogenous reference metabolites which show stability in accordance with statistical stability measures being selected from the group consisting of coefficient of variation (CV) of raw intensity data, standard deviation (SD) of logarithmic intensity data, stability measure (M) of geNorm - algorithm or stability measure value (rho) of NormFinderalgorithm.

Furthermore, it might be preferred to use such metabolites as endogenous reference metabolites which show stability in accordance with at least 2 statistical stability measures being selected from the group consisting of coefficient of variation (CV) of raw intensity data, standard deviation (SD) of logarithmic intensity data, stability measure (M) of geNorm - algorithm or stability measure value (rho) of NormFinderalgorithm, and/or such endogenous reference metabolites being in particular selected from the group consisting of:
PTC-Arginine
Carnitine (free)
Decenoylcarnitine
Decanoylcarnitine [Caprylcarnitine] (Fumarylcarnitine)
Dodecenoylcarnitine
Dodecanedioylcarnitine
Dodecanoylcarhitine [Laurylcarnitine]
Tetradecanoylcarnitine
3-Hydroxytetradecanoylcarnitine [Hydroxymyristylcarnitine]
Hexadecenoylcarnitine [Palmitoleylcarnitine]
3-Hydroxyhexadecenoylcarnitine [3-Hydroxypalmitoleylcarnitine]
3-Hydroxyhexadecadienoylcarnitine
3-Hydroxyhexadecanolycarnitine [3-Hydroxypalmitoylcarnitine]
3-Hydroxyoctadecenoylcarnitine [3-Hydroxyoleylcarnitine]
Propenoylcarnitine
Hydroxypropionylcarnitine
3-Hydroxybutyrylcarnitine / Malonylcarnitine
Methylglutarylcarnitine
3-Hydroxyisovalerylcarnitine / 3-Hydroxy-2-methylbutyryl
Hexenoylcarnitine
Hexanoylcarnitine [Caproylcarnitine]
Pimelylcarnitine
Octenoylcarnitine
Octanoylcarnitine [Caprylylcarnitine]
Glutamine
PTC-Glutamine
PTC-Histidine
Lysophosphatidylcholine with acyl residue C14:0
Lysophosphatidylcholine with acyl residue C26:0
Lysophosphatidylcholine with acyl residue C26:1
Lysophosphatidylcholine with acyl residue C28:0
Lysophosphatidylcholine with acyl residue C28:1
Phosphatidylcholine with diacyl residue sum C24:0
Phosphatidylcholine with diacyl residue sum C26:0
Phosphatidylcholine with diacyl residue sum C30:0
Phosphatidylcholine with diacyl residue sum C30:2
Phosphatidylcholine with diacyl residue sum C32:2
Phosphatidylcholine with diacyl residue sum C34:2
Phosphatidylcholine with diacyl residue sum C36:0
Phosphatidylcholine with diacyl residue sum C36:2
Phosphatidylcholine with diacyl residue sum C36:4
Phosphatidylcholine with diacyl residue sum C38:0
Phosphatidylcholine with diacyl residue sum C38:1
Phosphatidylcholine with diacyl residue sum C42:1
Phosphatidylcholine with diacyl residue sum C42:0
Phosphatidylcholine with diacyl residue sum C42:5
Phosphatidylcholine with diacyl residue sum C42:6
Phosphatidylcholine with acyl-alkyl residue sum C30:1
Phosphatidylcholine with acyl-alkyl residue sum C34:1
Phosphatidylcholine with acyl-alkyl residue sum C36:0
Phosphatidylcholine with acyl-alkyl residue sum C38:1
Phosphatidylcholine with acyl-alkyl residue sum C38:4
Phosphatidylcholine with acyl-alkyl residue sum C38:6
Phosphatidylcholine with acyl-alkyl residue sum C40:0
Phosphatidylcholine with acyl-alkyl residue sum C40:1
Phosphatidylcholine with acyl-alkyl residue sum C40:5
Phosphatidylcholine with acyl-alkyl residue sum C40:6
Phosphatidylcholine with acyl-alkyl residue sum C42:0
Phosphatidylcholine with acyl-alkyl residue sum C42:5
Phosphatidylcholine with acyl-alkyl residue sum C44:6
Phenylalanine
PTC-Phenylalanine
Proline
Sphingomyeline with acyl residue sum C16:0
Sphingomyeline with acyl residue sum C16:1
Sphingomyeline with acyl residue sum C18:0
Sphingomyeline with acyl residue sum C18:1
sphingomyelin with acyl residue sum C20:2
Sphingomyeline with acyl residue sum C24:0
Sphingomyeline with acyl residue sum C24:1
Hydroxysphingomyeline with acyl residue sum C14:1
Hydroxysphingomyelin with acyl residue sum C16:1
Hydroxysphingomyeline with acyl residue sum C22:2
Hydroxysphingomyelin with acyl residue sum C24:1
Succinic acid (succinate)
PTC-Tryptophan
Valine
PTC- Valine
Leucine + Isoleucine
wherein the designation "residue Cn:m" or "residue sum Cn:m" represents the chain length of the acyl/alkyl residue(s), n represents the number of total carbon atoms in the acyl/alkyl residue(s), and m represents the number of total double bonds in the residue(s).

In a further preferred embodiment of the present invention, said selected endogenous reference metabolites show stability in accordance with at least 3 statistical stability measures being selected from the group consisting of coefficient of variation (CV) of raw intensity data, standard deviation (SD) of logarithmic intensity data, stability measure (M) of geNorm - algorithm or stability measure value (rho) of NormFinderalgorithm, and/or such endogenous reference metabolites being in particular selected from the group consisting of:
Carnitine (free)
Decanoylcarnitine [Caprylcarnitine] (Fumarylcarnitine)
Dodecanedioylcarnitine
Dodecanoylcarnitine [Laurylcarnitine]
Hexadecenoylcarnitine [Palmitoleylcarnitine]
3-Hydroxyhexadecenoylcarnitine [3-Hydroxypalmitoleylcarnitine]
3-Hydroxyhexadecanolycarnitine [3-Hydroxypalmitoylcarnitine]
Propenoylcarnitine
Hydroxypropionylcarnitine
3-Hydroxybutyrylcarnitine / Malonylcarnitine
Methylglutarylcarnitine
Hexanoylcarnitine [Caproylcarnitine]
Pimelylcarnitine
Octenoylcarnitine
Octanoylcarnitine [Caprylylcarnitine]
Glutamine
PTC-Glutamine
PTC-Histidine
Lysophosphatidylcholine with acyl residue C14:0
Lysophosphatidylcholine with acyl residue C26:0
Lysophosphatidylcholine with acyl residue C26:1
Lysophosphatidylcholine with acyl residue C28:1
Phosphatidylcholine with diacyl residue sum C26:0
Phosphatidylcholine with diacyl residue sum C32:2
Phosphatidylcholine with diacyl residue sum C36:0
Phosphatidylcholine with diacyl residue sum C38:0
Phosphatidylcholine with diacyl residue sum C42:1
Phosphatidylcholine with diacyl residue sum C42:5
Phosphatidylcholine with diacyl residue sum C42:6
Phosphatidylcholine with acyl-alkyl residue sum C38:4
Phosphatidylcholine with acyl-alkyl residue sum C40:0
Phosphatidylcholine with acyl-alkyl residue sum C42:0
Phosphatidylcholine with acyl-alkyl residue sum C42:5
Phosphatidylcholine with acyl-alkyl residue sum C44:6
Sphingomyeline with acyl residue sum C16:0
Sphingomyeline with acyl residue sum C16:1
sphingomyelin with acyl residue sum C20:2
Sphingomyeline with acyl residue sum C24:0
Hydroxysphingomyelin with acyl residue sum C24:1
Valine
PTC- Valine
wherein the designation "residue Cn:m" or "residue sum Cn:m" represents the chain length of the acyl/alkyl residue(s), n represents the number of total carbon atoms in the acyl/alkyl residue(s), and m represents the number of total double bonds in the residue(s).

A further preferred method according to the present invention uses endogenous reference metabolites which show stability in accordance with all statistical stability measures being selected from the group consisting of coefficient of variation (CV) of raw intensity data, standard deviation (SD) of logarithmic intensity data, stability measure (M) of geNorm - algorithm or stability measure value (rho) of NormFinderalgorithm, and/or such endogenous reference metabolites being in particular Phosphatidylcholine with diacyl residue sum C42:6, wherein the designation "C42:6" represents the chain length of the acyl residues, 42 represents the number of total carbon atoms in the acyl residues, and 6 represents the number of total double bonds in the residues.

Within the scope of the present invention, said plurality of endogenous reference metabolites comprises 2 to 80, in particular 2 to 60, preferably 2 to 50, preferred 2 to 30, more preferred 2 to 10, particularly preferred 2 to 10, preferably 3 to 5 endogenous reference metabolites.

A further embodiment of the present invention is use of one or a plurality of compounds or derivatives thereof wherein said compounds have a molecular mass less than 1500 Da as endogenous reference metabolites in metabolomics analysis methods, wherein said endogenous reference metabolites are selected from the group consisting of:
Amino acids, in particular, arginine, aspartic acid, citrulline, glutamic acid (glutamate), glutamine, leucine, isoleucine, histidine, ornithine, proline, phenylalanine, serine, tryptophane, tyrosine, valine, kynurenine;
phenylthio carbamyl amino acids (PTC-amino acids), in particular, PCT-arginine, PTC-glutamine, PTC-histidine, PTC-methionine, PTC-ornithine, PTC-phenylalanine, PTC-proline, PTC-serine, PTC-tryptophane, PTC-tyrosine, PTC-valine;
dimethylarginine, in particular N,N-dimethyl-L-arginine;
carboxylic acids, namely 15(S)-hydroxy-5Z,8Z,11Z,13E-eicosatetraenoic acid [(5Z,8Z,11Z,13E,15S)-15-Hydroxyicosa-5,8,11,13-tetraenoic acid], succinic acid (succinate);
carnitine; acylcarnitines having from 1 to 20 carbon atoms in the acyl residue; acylcarnitines having from 3 to 20 carbon atoms in the acyl residue and having 1 to 4 double bonds in the acyl residue; acylcarnitines having from 1 to 20 carbon atoms in the acyl residue and having from 1 to 3 OH-groups in the acyl residue; acylcarnitines having from 3 to 20 carbon atoms in the acyl residue with 1 to 4 double bonds and 1 to 3 OH-groups in the acyl residue;
phospholipides, in particular lysophosphatidylcholines (monoacylphosphatidylcholines) having from 1 to 30 carbon atoms in the acyl residue; lysophosphatidylcholines having from 3 to 30 carbon atoms in the acyl residue and having 1 to 6 double bonds in the acyl residue;
phosphatidylcholines (diacylphosphatidylcholines) having a total of from 1 to 50 carbon atoms in the acyl residues; phatidylcholines having a total from 3 to 50 carbon atoms in the acyl residues and having a total of 1 to 8 double bonds in the acyl residues;
sphingolipids, in particular sphingomyelines having a total number of carbon atoms in the acyl chains from 10 to 30; sphingomyelines having a total number of carbon atoms in the acyl chains from 10 to 30 and 1 to 5 double bonds; hydroxysphinogomyelines having a total number of carbon atoms in the acyl residues from 10 to 30; hydroxysphingoyelines having a total number of carbon atoms in the acyl residues from 10 to 30 and 1 to 5 double bonds;
prostaglandines, namely 6-keto-prostaglandin F1 alpha, prostaglandin D2;
putrescine;

As mentioned earlier, said metabolomics analysis methods comprise the quantitation of endogenous target and reference metabolites by mass spectrometry (MS), in particular MS-technologies such as Matrix Assisted Laser Desorption/Ionisation (MALDI), Electro Spray Ionization (ESI), Atmospheric Pressure Chemical Ionization (APCI), ¹H-, ¹³C- and/or ³¹P- Nuclear Magnetic Resonance spectroscopy (NMR), optionally coupled to MS, determination of metabolite concentrations by use of MS-technologies and/or methods coupled to separation, in particular Liquid Chromatography (LC-MS), Gas Chromatography (GC-MS), or Capillary Electrophoresis (CE-MS).

Particularly preferred compounds which can be used as endogenous reference metabolites in accordance with the present invention are selected from the group consisting of:
15((S)-hydroxy-5Z,8Z,11Z,13E-eicosatetraenoic acid
6-keto-Prostaglandin F1alpha
asymmetrical Dimethylarginin
Arginine
PTC-Arginine
Aspartic acid
Carnitine (free)
Decanoylcarnitine [Caprylcarnitine] (Fumarylcarnitine)
Decenoylcarnitine
Decadienoylcarnitine
Dodecanoylcarnitine [Laurylcarnitine]
Dodecenoylcarnitine
Dodecanedioylcarnitine
Tetradecanoylcarnitine
Tetradecenoylcarnitine [Myristoleylcarnitine]
3-Hydroxytetradecenoylcarnitine [3-Hydroxymyristoleylcarnitine]
3-Hydroxytetradecadienoylcarnitine
3-Hydroxytetradecanoylcarnitine [Hydroxymyristylcarnitine]
Hexadecenoylcarnitine [Palmitoleylcarnitine]
3-Hydroxyhexadecenoylcarnitine [3-Hydroxypalmitoleylcarnitine]
Hexadecadienoylcarnitine
3-Hydroxyhexadecadienoylcarnitine
3-Hydroxyhexadecanolycarnitine [3-Hydroxypalmitoylcarnitine]
Octadecanoylcarnitine [Stearylcarnitine]
Octadecenoylcarnitine [Oleylcarnitine]
3-Hydroxyoctadecenoylcarnitine [3-Hydroxyoleylcarnitine]
Acetylcarnitine
Propenoylcarnitine
Hydroxypropionylcarnitine
Butenoylcarnitine
3-Hydroxybutyrylcarnitine / Malonylcarnitine
Isovalerylcarnitine / 2-Methylbutyrylcarnitine / Valerylcarnitine
Tiglylcarnitine / 3-Methyl-crotonylcarnitine
Glutarylcarnitine / Hydroxycaproylcarnitine
Glutarylcarnitine / Hydroxycaproylcarnitine
Methylglutarylcarnitine
3-Hydroxyisovalerylcarnitine / 3-Hydroxy-2-methylbutyryl
Hexanoylcarnitine [Caproylcarnitine]
Hexenoylcarnitine
Pimelylcarnitine
Octanoylcarnitine [Caprylylcarnitine]
Octenoylcarnitine
Nonanoylcarnitine [Pelargonylcarnitine]
Citrulline
Creatinine
Glutamine
PTC-Glutamine
Glutamate
Histidine
PTC-Histidine
Kynurenine
Leucine
Lysophosphatidylcholine with acyl residue C14:0
Lysophosphatidylcholine with acyl residue C16:0
Lysophosphatidylcholine with acyl residue C16:1
Lysophosphatidylcholine with acyl residue C18:0
Lysophosphatidylcholine with acyl residue C18:1
Lysophosphatidylcholine with acyl residue C18:2
Lysophosphatidylcholine with acyl residue C20:3
Lysophosphatidylcholine with acyl residue C20:4
Lysophosphatidylcholine with acyl residue C24:0
Lysophosphatidylcholine with acyl residue C26:0
Lysophosphatidylcholine with acyl residue C26:1
Lysophosphatidylcholine with acyl residue C28:0
Lysophosphatidylcholine with acyl residue C28:1
Lysophosphatidylcholine with acyl residue C6:0
PTC-Methionine
Ornithine
PTC-Ornithine
Phosphatidylcholine with diacyl residue sum C24:0
Phosphatidylcholine with diacyl residue sum C26:0
Phosphatidylcholine with diacyl residue sum C28:1
Phosphatidylcholine with diacyl residue sum C30:0
Phosphatidylcholine with diacyl residue sum C30:2
Phosphatidylcholine with diacyl residue sum C32:0
Phosphatidylcholine with diacyl residue sum C32:1
Phosphatidylcholine with diacyl residue sum C32:2
Phosphatidylcholine with diacyl residue sum C32:3
Phosphatidylcholine with diacyl residue sum C34:1
Phosphatidylcholine with diacyl residue sum C34:2
Phosphatidylcholine with diacyl residue sum C34:3
Phosphatidylcholine with diacyl residue sum C34:4
Phosphatidylcholine with diacyl residue sum C36:0
Phosphatidylcholine with diacyl residue sum C36:1
Phosphatidylcholine with diacyl residue sum C36:2
Phosphatidylcholine with diacyl residue sum C36:3
Phosphatidylcholine with diacyl residue sum C36:4
Phosphatidylcholine with diacyl residue sum C36:5
Phosphatidylcholine with diacyl residue sum C36:6
Phosphatidylcholine with diacyl residue sum C38:0
Phosphatidylcholine with diacyl residue sum C38:1
Phosphatidylcholine with diacyl residue sum C38:3
Phosphatidylcholine with diacyl residue sum C38:4
Phosphatidylcholine with diacyl residue sum C38:5
Phosphatidylcholine with diacyl residue sum C38:6
Phosphatidylcholine with diacyl residue sum C42:1
Phosphatidylcholine with diacyl residue sum C40:3
Phosphatidylcholine with diacyl residue sum C40:4
Phosphatidylcholine with diacyl residue sum C40:5
Phosphatidylcholine with diacyl residue sum C40:6
Phosphatidylcholine with diacyl residue sum C42:0
Phosphatidylcholine with diacyl residue sum C42:1
Phosphatidylcholine with diacyl residue sum C42:2
Phosphatidylcholine with diacyl residue sum C42:4
Phosphatidylcholine with diacyl residue sum C42:5
Phosphatidylcholine with diacyl residue sum C42:6
Phosphatidylcholine with acyl-alkyl residue sum C30:0
Phosphatidylcholine with acyl-alkyl residue sum C30:1
Phosphatidylcholine with acyl-alkyl residue sum C34:0
Phosphatidylcholine with acyl-alkyl residue sum C34:1
Phosphatidylcholine with acyl-alkyl residue sum C34:2
Phosphatidylcholine with acyl-alkyl residue sum C34:3
Phosphatidylcholine with acyl-alkyl residue sum C36:0
Phosphatidylcholine with acyl-alkyl residue sum C36:1
Phosphatidylcholine with acyl-alkyl residue sum C36:2
Phosphatidylcholine with acyl-alkyl residue sum C36:3
Phosphatidylcholine with acyl-alkyl residue sum C36:4
Phosphatidylcholine with acyl-alkyl residue sum C38:0
Phosphatidylcholine with acyl-alkyl residue sum C38:1
Phosphatidylcholine with acyl-alkyl residue sum C38:2
Phosphatidylcholine with acyl-alkyl residue sum C38:3
Phosphatidylcholine with acyl-alkyl residue sum C38:4
Phosphatidylcholine with acyl-alkyl residue sum C38:5
Phosphatidylcholine with acyl-alkyl residue sum C38:6
Phosphatidylcholine with acyl-alkyl residue sum C40:0
Phosphatidylcholine with acyl-alkyl residue sum C40:1
Phosphatidylcholine with acyl-alkyl residue sum C40:2
Phosphatidylcholine with acyl-alkyl residue sum C40:3
Phosphatidylcholine with acyl-alkyl residue sum C40:4
Phosphatidylcholine with acyl-alkyl residue sum C40:5
Phosphatidylcholine with acyl-alkyl residue sum C40:6
Phosphatidylcholine with acyl-alkyl residue sum C42:0
Phosphatidylcholine with acyl-alkyl residue sum C42:1
Phosphatidylcholine with acyl-alkyl residue sum C42:3
Phosphatidylcholine with acyl-alkyl residue sum C42:4
Phosphatidylcholine with acyl-alkyl residue sum C42:5
Phosphatidylcholine with acyl-alkyl residue sum C44:5
Phosphatidylcholine with acyl-alkyl residue sum C44:6
Prostaglandin D2
Phenylalanine
PTC-Phenylalanine
Proline
PTC-Proline
Putrescine
Serine
PTC-Serine
Hydroxysphingomyeline with acyl residue sum C14:1
Hydroxysphingomyelin with acyl residue sum C16:1
Hydroxysphingomyeline with acyl residue sum C22:1
Hydroxysphingomyeline with acyl residue sum C22:2
Hydroxysphingomyelin with acyl residue sum C24:1
Sphingomyeline with acyl residue sum C16:0
Sphingomyeline with acyl residue sum C16:1
Sphingomyeline with acyl residue sum C18:0
Sphingomyeline with acyl residue sum C18:1
sphingomyelin with acyl residue sum C20:2
Sphingomyeline with acyl residue sum C22:3
Sphingomyeline with acyl residue sum C24:0
Sphingomyeline with acyl residue sum C24:1
Sphingomyeline with acyl residue sum C26:0
Sphingomyeline with acyl residue sum C26:1
Succinic acid (succinate)
Total dimethylarginine: sum ADMA + SDMA
Tryptophan
PTC-Tryptophan
Tyrosine
Valine
PTC- Valine
Leucine + Isoleucine
wherein the designation "residue Cn:m" or "residue sum Cn:m" represents the chain length of the acyl/alkyl residue(s), n represents the number of total carbon atoms in the acyl/alkyl residue(s), and m represents the number of total double bonds in the residue(s).

Another advantageous embodiment of the present invention is the use of such compounds as endogenous reference metabolites, which show stability in accordance with statistical stability measures being selected from the group consisting of coefficient of variation (CV) of raw intensity data, standard deviation (SD) of logarithmic intensity data, stability measure (M) of geNorm - algorithm or stability measure value (rho) of Norm Finder-algorithm.

Preferably, such compounds can be used as endogenous reference metabolites which show stability in accordance with at least 2 statistical stability measures being selected from the group consisting of coefficient of variation (CV) of raw intensity data, standard deviation (SD) of logarithmic intensity data, stability measure (M) of geNorm - algorithm or stability measure value (rho) of Norm Finder-algorithm, and/or such endogenous reference metabolites being in particular selected from the group consisting of:
PTC-Arginine
Carnitine (free)
Decenoylcarnitine
Decanoylcarnitine [Caprylcarnitine] (Fumarylcarnitine)
Dodecenoylcarnitine
Dodecanedioylcarnitine
Dodecanoylcarnitine [Laurylcarnitine]
Tetradecanoylcarnitine
3-Hydroxytetradecanoylcarnitine [Hydroxymyristylcarnitine]
Hexadecenoylcarnitine [Palmitoleylcarnitine]
3-Hydroxyhexadecenoylcarnitine [3-Hydroxypalmitoleylcarnitine]
3-Hydroxyhexadecadienoylcarnitine
3-Hydroxyhexadecanolycarnitine [3-Hydroxypalmitoylcarnitine]
3-Hydroxyoctadecenoylcarnitine [3-Hydroxyoleylcarnitine]
Propenoylcarnitine
Hydroxypropionylcarnitine
3-Hydroxybutyrylcarnitine / Malonylcarnitine
Methylglutarylcarnitine
3-Hydroxyisovalerylcarnitine / 3-Hydroxy-2-methylbutyryl
Hexenoylcarnitine
Hexanoylcarnitine [Caproylcarnitine]
Pimelylcarniti ne
Octenoylcarnitine
Octanoylcarnitine [Caprylylcarnitine]
Glutamine
PTC-Glutamine
PTC-Histidine
Lysophosphatidylcholine with acyl residue C14:0
Lysophosphatidylcholine with acyl residue C26:0
Lysophosphatidylcholine with acyl residue C26:1
Lysophosphatidylcholine with acyl residue C28:0
Lysophosphatidylcholine with acyl residue C28:1
Phosphatidylcholine with diacyl residue sum C24:0
Phosphatidylcholine with diacyl residue sum C26:0
Phosphatidylcholine with diacyl residue sum C30:0
Phosphatidylcholine with diacyl residue sum C30:2
Phosphatidylcholine with diacyl residue sum C32:2
Phosphatidylcholine with diacyl residue sum C34:2
Phosphatidylcholine with diacyl residue sum C36:0
Phosphatidylcholine with diacyl residue sum C36:2
Phosphatidylcholine with diacyl residue sum C36:4
Phosphatidylcholine with diacyl residue sum C38:0
Phosphatidylcholine with diacyl residue sum C38:1
Phosphatidylcholine with diacyl residue sum C42:1
Phosphatidylcholine with diacyl residue sum C42:0
Phosphatidylcholine with diacyl residue sum C42:5
Phosphatidylcholine with diacyl residue sum C42:6
Phosphatidylcholine with acyl-alkyl residue sum C30:1
Phosphatidylcholine with acyl-alkyl residue sum C34:1
Phosphatidylcholine with acyl-alkyl residue sum C36:0
Phosphatidylcholine with acyl-alkyl residue sum C38:1
Phosphatidylcholine with acyl-alkyl residue sum C38:4
Phosphatidylcholine with acyl-alkyl residue sum C38:6
Phosphatidylcholine with acyl-alkyl residue sum C40:0
Phosphatidylcholine with acyl-alkyl residue sum C40:1
Phosphatidylcholine with acyl-alkyl residue sum C40:5
Phosphatidylcholine with acyl-alkyl residue sum C40:6
Phosphatidylcholine with acyl-alkyl residue sum C42:0
Phosphatidylcholine with acyl-alkyl residue sum C42:5
Phosphatidylcholine with acyl-alkyl residue sum C44:6
Phenylalanine
PTC-Phenylalanine
Proline
Sphingomyeline with acyl residue sum C16:0
Sphingomyeline with acyl residue sum C16:1
Sphingomyeline with acyl residue sum C18:0
Sphingomyeline with acyl residue sum C18:1
sphingomyelin with acyl residue sum C20:2
Sphingomyeline with acyl residue sum C24:0
Sphingomyeline with acyl residue sum C24:1
Hydroxysphingomyeline with acyl residue sum C14:1
Hydroxysphingomyelin with acyl residue sum C16:1
Hydroxysphingomyeline with acyl residue sum C22:2
Hydroxysphingomyelin with acyl residue sum C24:1
Succinic acid (succinate)
PTC-Tryptophan
Valine
PTC- Valine
Leucine + Isoleucine
wherein the designation "residue Cn:m" or "residue sum Cn:m" represents the chain length of the acyl/alkyl residue(s), n represents the number of total carbon atoms in the acyl/alkyl residue(s), and m represents the number of total double bonds in the residue(s).

Another advantageous use according to the invention is the use of such endogenous reference metabolites which show stability in accordance with at least 3 statistical stability measures being selected from the group consisting of coefficient of variation (CV) of raw intensity data, standard deviation (SD) of logarithmic intensity data, stability measure (M) of geNorm - algorithm or stability measure value (rho) of NormFinder-algorithm, and/or such endogenous reference metabolites being in particular selected from the group consisting of:
Carnitine (free)
Decanoylcarnitine [Caprylcarnitine] (Fumarylcarnitine)
Dodecanedioylcarnitine
Dodecanoylcarnitine [Laurylcarnitine]
Hexadecenoylcarnitine [Palmitoleylcarnitine]
3-Hydroxyhexadecenoylcarnitine [3-Hydroxypalmitoleylcarnitine]
3-Hydroxyhexadecanolycarnitine [3-Hydroxypalmitoylcarnitine]
Propenoylcarnitine
Hydroxypropionylcarnitine
3-Hydroxybutyrylcarnitine / Malonylcarnitine
Methylglutarylcarnitine
Hexanoylcarnitine [Caproylcarnitine]
Pimelylcarnitine
Octenoylcarnitine
Octanoylcarnitine [Caprylylcarnitine]
Glutamine
PTC-Glutamine
PTC-Histidine
Lysophosphatidylcholine with acyl residue C14:0
Lysophosphatidylcholine with acyl residue C26:0
Lysophosphatidylcholine with acyl residue C26:1
Lysophosphatidylcholine with acyl residue C28:1
Phosphatidylcholine with diacyl residue sum C26:0
Phosphatidylcholine with diacyl residue sum C32:2
Phosphatidylcholine with diacyl residue sum C36:0
Phosphatidylcholine with diacyl residue sum C38:0
Phosphatidylcholine with diacyl residue sum C42:1
Phosphatidylcholine with diacyl residue sum C42:5
Phosphatidylcholine with diacyl residue sum C42:6
Phosphatidylcholine with acyl-alkyl residue sum C38:4
Phosphatidylcholine with acyl-alkyl residue sum C40:0
Phosphatidylcholine with acyl-alkyl residue sum C42:0
Phosphatidylcholine with acyl-alkyl residue sum C42:5
Phosphatidylcholine with acyl-alkyl residue sum C44:6
Sphingomyeline with acyl residue sum C16:0
Sphingomyeline with acyl residue sum C16:1
sphingomyelin with acyl residue sum C20:2
Sphingomyeline with acyl residue sum C24:0
Hydroxysphingomyelin with acyl residue sum C24:1
Valine
PTC- Valine
wherein the designation "residue Cn:m" or "residue sum Cn:m" represents the chain length of the acyl/alkyl residue(s), n represents the number of total carbon atoms in the acyl/alkyl residue(s), and m represents the number of total double bonds in the residue(s).

A further preferred embodiment of the present invention to use such compounds as endogenous reference metabolites which show stability in accordance with all statistical stability measures being selected from the group consisting of coefficient of variation (CV) of raw intensity data, standard deviation (SD) of logarithmic intensity data, stability measure (M) of geNorm - algorithm or stability measure value (rho) of NormFinder-algorithm, and/or such endogenous reference metabolites being in particular Phosphatidylcholine with diacyl residue sum C42:6, wherein the designation "C42:6" represents the chain length of the acyl residues, 42 represents the number of total carbon atoms in the acyl residues, and 6 represents the number of total double bonds in the residues.

Furthermore, the use of a plurality of 2 to 80, in particular 2 to 60, preferably 2 to 50, preferred 2 to 30, more preferred 2 to 10, particularly preferred 2 to 10, preferably 3 to 5, as endogenous reference metabolites, is preferred.

The need for good methods of normalization for metabolomics data can not be overstated and is a prerequisite for applications requiring quantitative data or comparison to reference values, such as required in diagnostics. There are numerous sources of technical variations such as charge effects of reagents, batch effects, dilution effects, unreproducible distribution of internal standards after addition to tissue and unnoticed changes in experimental conditions. Ideally, normalization should allow also a direct comparison of data obtained from different sample types, of different species and determined on different assay platforms and by different laboratories. Additional, more specifically mass-spectrometry-related unsolved problems comprise matrix-specific signal suppression or ion suppression, detrimental to cross-matrix, cross- tissue and cross-species comparison of data.

The above procedures must be carried out to transform raw signal information to present the data in a format suitable for subsequent data analysis and interpretation. However, even with control spots or external control samples, undesired experimental variation can contaminate data. It is also possible that some or all of the physical normalization techniques are missing from the experiment, in which case it is even more important to find additional means of normalization.

Theoretically, an ideal endogenous standard for metabolomics would be a compound or a set of compounds whose concentration levels do not vary during the cell cycle, between cell types, between various states of a disease, between various states of health or in response to experimental treatments that one wishes to examine. Additionally, for an endogenous standard (that is endogenous metabolites) to be valid in metabolomics it is crucial that it be of a similar relative abundance as the test and reference (or target) metabolite in the metabolite assay. Such molecules have not been described so far, nor a method utilizing these molecules in the context of quantitative multiparametric metabolomics and determination of metabolite levels using mass spectroscopy or any other method affording quantitative or relative concentration data.

Using an exogenously added standard has the advantage of giving the user control over the amount of compound added, with low variation between samples. Using an exogenous standard does not, however, control differences in the quality of the starting material or the tissue for workup then subjected to analysis. If there are differences in the levels of integrity of the metabolites between otherwise identical samples, the extraction yield will reflect this variation, although the external standards will still appear identical.

An endogenous standard -not to be mixed up with internal standards which are also added externally- might in theory circumvent many of the above listed problems in metabolomics experiments. However, although the concept of endogenous housekeeping genes is known in the context of gene expression and transcriptomics, the generation, identification and application of housekeeping metabolites or combination and combinatorial use of these is unknown in metabolomics and, while numerous studies exist describing the application of metabolic data in various contexts, the application of control-, housekeeping or endogenous reference metabolites in metabolomics has not been described.
However, since metabolite concentrations are notably varying in-between species, in response to physiological and environmental conditions, due to variations in nutrition or amount of food intake the identification and use of endogenous compounds or metabolites as normalization tools has been assessed as highly unlikely. An identification of endogenous compounds with limited variation among different tissues, different species, let alone in-between various states of health or in subjects with distinct diseases is even more unlikely and in any case not obvious. In particular, it is surprising for the person having average skill in the art, that the compound being identified in the present application, can be used as endogenous reference metabolites. The experimental results as described in the examples below, further show that a significantly improved prediction power was achieved in metabolomics experiments as shown in the examples with the endogenous reference metabolites compared to without using endogenous reference metabolite normalization.

Despite of the obstacles from the prior art, the inventors investigated whether it will be possible to identify a plurality of metabolites with minimal changes of concentrations or "constant" levels which can be used for normalization, thus prerequisite for the identification, determination, quantitation and application of disease associated concentration changes of other, variable metabolites and the application of these reference or control metabolites, in conjunction with a determination of variable metabolites, in diagnostic tools.

In the present approach, the invention shows that subsets of endogenous reference metabolites or housekeeper metabolites actually exist and that for these metabolites the distribution of levels remains -within defined limits-constant or have some mean values and standard deviations that surprisingly are independent of any particular sample, physiological conditions of a subject, sample workup, and tissue.

Accordingly, the present invention provides a general method and a novel solution to the problem of normalizing metabolite data, a general method to generate endogenous reference metabolites, a method to use endogenous reference metabolites as well as a list of endogenous reference compounds and metabolites.

A further object of the present invention is to address and resolve the above outlined problems in metabolomics data normalization and data applications by generating and providing a list of novel endogenous standards for normalizing the relative intensities of signals in metabolite and metabolomics assays, a method for the application of these standards in normalization and thus in quantitative metabolite determination and diagnosis.

The invention provides a solution to the comparison, quantification and normalization and thus application of metabolomics data, generated by quantitation of endogenous metabolites by, but not limited to, mass spectrometry (MS), in particular MS-technologies such as MALDI, ESI, atmospheric pressure pressure chemical ionization (APCI), and other methods, determination of metabolite concentrations by use of MS-technologies or alternative methods coupled to separation (LC-MS, LC-NMR, GC-MS, CE-MS), subsequent feature selection and /or the combination of features to classifiers including molecular data of at least two molecules or at least tow analytes of the same or different molecules and to the comparison of these data obtained from different subjects, different species, different time points of sampling, processed by different people and under varying experimental conditions.

We demonstrate that endogenous reference metabolite panels surprisingly show minimal variations in concentrations among samples from different species, different tissues and work-up conditions. We further demonstrate applications in characterizing diseased states or in diagnosis.
This invention thus provides robust control metabolites, surprisingly displaying almost unchanged levels among various species, animal model, conditions of health, sample type, sample workup and experimental settings, tissue type or body liquid, sample processing and assay conditions as well as analytical determination of contents, allowing normalization, identification and application of metabolites characteristic for distinct biological states or associated with distinct diseases, respectively the grade of such diseases and their response to therapy in man or in mammalian model systems, irrespective of the experimental set-up and the assay platform.

The invention further relates to the use of metabolite measurements, such as to diagnose, classify or identify a disease, to prognose future development, to distinguish various states of disease, control treatment regimens or lengths of treatment, control consequences of drug intake or consequences of varying endogenous drug concentrations or any consequences on the levels of endogenous metabolites due to experimental conditions. It provides a reference point and appropriate underlying values to identify and recognize disease-associated metabolic changes and thus applications in diagnosis and therapeutic monitoring.
The use of metabolite measurements relies upon the ability to detect or measure differential concentration of metabolites in body liquids, cells and tissue. Metabolites analyzed include those which are differentially detected in a manner that is relevant to the biological phenotype of interest. The metabolite level(s) of one or more differentially concentrated metabolites is determined in the cell(s), tissues or body liquids of a sample or subject, after which the metabolite concentration level(s) of the one or more analytes are used directly or, in the case of determination of multiple analytes, in comparison to each other. One non-limiting example of the latter case is where the metabolite concentration levels of two samples are determined and then used as a ratio of one to the other.
The comparison of metabolite levels in a cell, tissue, body liquid, as well as between specimen of different samples and/or origins and across experiments or dates of experimental measurement is improved when the levels are normalized to a reference. This can be viewed as normalization to a single scale. The use of normalization, such as, but not limited to, where metabolomics or metabolomics kits with multiple probes are used to determine metabolite levels, allows for direct assay to assay or in case of assays on multi-well plates, plate to plate comparisons.
"Mass Spectrometry" (MS) is a technique for measuring and analyzing molecules that involves fragmenting a target molecule, then analyzing the fragments, based on their mass/charge ratios, to produce a mass spectrum that serves as a "molecular fingerprint". Determining the mass/charge ratio of an object is done through means of determining the wavelengths at which electromagnetic energy is absorbed by that object. There are several commonly used methods to determine the mass to charge ratio of an ion, some measuring the interaction of the ion trajectory with electromagnetic waves, others measuring the time an ion takes to travel a given distance, or a combination of both. The data from these fragment mass measurements can be searched against databases to obtain definitive identifications of target molecules. Mass spectrometry is also widely used in other areas of chemistry, like petrochemistry or pharmaceutical quality control, among many others.

An "ion" is a charged object formed by adding electrons to or removing electrons from an atom.

A "mass spectrum" is a plot of data produced by a mass spectrometer, typically containing m/z values on x-axis and intensity values on y-axis. The term "m/z" refers to the dimensionless quantity formed by dividing the mass number of an ion by its absolute charge number. It has long been called the "mass-to-charge" ratio.

As used here, the term "metabolite" denotes endogenous organic compounds of a cell, an organism, a tissue or being present in body liquids and in extracts obtained from the aforementioned sources with a molecular weight typically below 1500 Dalton. Typical examples of metabolites are carbohydrates, lipids, phospholipids, sphingolipids and sphingophospholipids, amino acids, cholesterol, steroid hormones and oxidized sterols and other compounds such as collected in the Human Metabolite database [Wishart DS et al., HMDB: the Human Metabolome Database. Nucleic Acids Res. 2007 Jan;35(Database issue):D521-6(see http://www.hmdb.ca/)*]* and other databases and literature. This includes any substance produced by metabolism or by a metabolic process and any substance involved in metabolism.

The term "metabolism" refers to the chemical changes that occur within the tissues of an organism, including "anabolism" and "catabolism". Anabolism refers to biosynthesis or the buildup of molecules and catabolism refers to the breakdown of molecules.

"Metabolomics" as understood within the scope of the present invention designates the comprehensive quantitative measurement of several (2-thousands) metabolites by, but not limited to, methods such as mass spectroscopy, coupling of liquid chromatography, gas chromatography and other separation methods chromatography with mass spectroscopy or nuclear magnetic resonance (NMR).

Matrix denotes the sample type for analysis or sample work-up such as body liquids (plasma, blood) different tissues (eg. brain and liver), different parts from an organism such as leaves compared to roots obtained from plants etc. Different matrices may also include the same type of tissue or body liquid but from different species such as eg. rat plasma compared to human plasma.

A biomarker in this context is a characteristic, comprising data of at least one metabolite that is measured and evaluated as an indicator of biologic processes, pathogenic processes, or responses to a therapeutic intervention associated with a diseased state or related treatment. A combined biomarker as used here may be selected from at least two small endogenous molecules and metabolites.
Analyte denotes the chemical entity or mixtures of compounds as analysed. Given limitations to structural resolution of the techniques (mass spectroscopy) that is position of double bond(s), structural isomers varying in configuration (e.g. positions of fatty acid residues in phospholipids or triglycerides) one analyte can thus formally include more than 1 metabolite.
The terms housekeeping metabolite or analyte, reference or control metabolites are exchangeable in the invention and denote metabolites and analytes with small variations in amounts and concentration levels in-between several samples when different samples of different subjects with various physiological conditions, various states of health or therapy or pre-treatment are compared. A control-, reference- or housekeeping metabolite thus is a metabolite with minimal changes of concentration along several samples and which, therefore, can be used as endogenous reference metabolite. These endogenous reference metabolites thus can compensate for variability associated with technical set up, sample handling, experimental work up, and random fluctuation in metabolomics.
A endogenous reference metabolite is preferably not differentially present at a level that is statistically significant (e.g., a p-value less than 0.05 and/or a q-value of less than 0.05). Exemplary asphyxia-specific endogenous reference metabolites are described in the detailed description and experimental sections below.
As used herein, the term "disease specific endogenous reference metabolite" refers to a metabolite that is not differentially present or differentially concentrated in diseased organisms compared to non-diseased organisms. For instance the term "asphyxia specific endogenous reference metabolite" refers to a metabolite that has the same concentration level in asphyctic organisms compared to non-asphyctic organisms with a low variability.
As used herein, the term "target metabolite" refers to a metabolite that is differentially present or differentially concentrated under different conditions or different treatment such as eg. diseased organisms compared to non-diseased organisms, in treated organisms compared to non-treated organisms etc.. For instance the term "asphyxia specific metabolite" refers to a metabolite that is differentially present or differentially concentrated in asphyctic organisms compared to non-asphyctic organisms.
The term "sample" in the present specification and claims is used in its broadest sense. On the one hand it is meant to include a specimen or culture. On the other hand, it is meant to include both biological and environmental samples. A sample may include a specimen of synthetic origin.
Biological samples may be animal, including human, fluid, solid (e.g., stool) or tissue, as well as liquid and solid food and feed products and ingredients such as dairy items, vegetables, meat and meat by-products, and waste. Biological samples may be obtained from all of the various families of domestic animals, as well as feral or wild animals, including, but not limited to, such animals as ungulates, bear, fish, rodents, etc.

A biological sample may contain any biological material suitable for detecting the desired biomarkers, and may comprise cellular and/or non-cellular material from a subject. The sample can be isolated from any suitable biological tissue or fluid such as, for example, tissue, blood, blood plasma, urine, or cerebral spinal fluid (CSF), plant extract or processed biological material (e.g. food).
As used herein, the term "cell" refers to any eukaryotic or prokaryotic cell (e.g., bacterial cells such as E. coli, yeast cells, mammalian cells, avian cells, amphibian cells, plant cells, fish cells, and insect cells), whether located in vitro or in vivo.
As used herein, the terms "detect", "detecting", or "detection" may describe either the general act of discovering or discerning or the specific observation of a detectably labeled or unlabeled composition.

Normalization: Normalization as understood in this context denotes a procedure to make metabolomics data of different assays comparable by reduction or elimination of technical variability. In this kind of comparative analysis, normalization is essential to compensate for variations in isolation techniques, initial quantification errors, tube to tube variation in preceding chemical or enzymatic modification and other experimental variations.

A schematic view of the method of the present invention is shown below:

| |
|---|
| **Step 1:** |
| Biological sample obtained |
| **Step 2:** |
| **Measurement of raw data (concentrations of biomolecules) and deposit in data base** |
| **Step 3:** |
| **Preprocessing of raw data from data base** |
| **Step 4:** |
| **Selection of most stable housekeeping metabolites** |
| **Step 5:** |
| **Normalization of target metabolites** |
| **Step 6:** |
| **Statistical Analyses and dissemination** |

**First,** a biological sample obtained from a subject or an organism is obtained.

**Second,** the amounts of biomolecules are measured from the biological sample and stored as raw data in a database or any electronic device.

**Third,** the raw data from the database are preprocessed. This step is optional, but one often uses the log-transformed analyte concentrations for further analysis, where the log-transformation is used to stabilize variance and to obtain at least approximately normal distributed data, Of course, one could also use other transformations like for instance Box-Cox power transformations [Box G.E.P. and Cox D.R. (1964) An analysis of transformations (with discussion). Journal of the Royal Statistical Society B, 26, 211-252.], the generalized log-transformation via vsn [Huber W., von Heydebreck A., Sueltmann H., Poustka A., Vingron M. (2002). Variance stabilization applied to microarray data calibration and to the quantification of differential expression. Bioinformatics 18 Suppl.1 S96-S104.] or vst [Lin S.M., Du P., Huber W., Kibbe, W.A. (2008). Model-based variance-stabilizing transformation for Illumina microarray data. Nucleic Acids Research, Vol. 36, No. 2 e11.] or some other normalization procedure which is well-known from microarray normalization in this step. A comparative survey of normalization procedures in case of Affymetrix microarray data is given in Cope et al. (2004) [L.M. Cope et al. (2004). A Benchmark for Affymetrix GeneChip Expression Measures, Bioinformatics 20(3):323-331] or Irizarry et al. (2006) [R.A. Irizarry et al. (2006). Comparison of Affymetrix GeneChip Expression Measures, Bioinformatics 22(7):789-794].

**Fourth,** at least one most stable metabolite is chosen as housekeeping metabolite where stability can be defined in terms of coefficient of variation, standard deviation, pairwise variability, inter- and intra group variability, etc. As the technical variability may be different for different analyte classes, one can also distinguish between analyte classes during this selection process.

**Fifth,** the target metabolites are normalized via the chosen housekeeping metabolites. This can be done by considering raw or preprocessed concentration ratios or differences. If there is more than one housekeeping metabolite, the raw or preprocessed concentrations of these metabolites are aggregated using geometric mean, arithmetic mean, median or some other aggregation procedure and then the concentrations of the target metabolites are related to this aggregated reference value. The normalization can also take the analyte classes into account by using different housekeeping metabolites for different analyte classes.

**Sixth,** the housekeeper normalized concentrations of the target metabolites are used for the statistical analyses which can be uni- or multivariate tests, regression or classification analyses, etc..

The present invention provides a solution to the comparison, quantification and normalization and thus application of metabolomics data, generated by quantitation of endogenous metabolites by, but not limited to mass spectrometry (MS), in particular MS-technologies such as MALDI, ESI, atmospheric pressure pressure chemical ionization (APCI), and other methods, determination of metabolite concentrations by use of MS-technologies or alternative methods coupled to separation (LC-MS, GC-MS, CE-MS), subsequent feature selection and /or the combination of features to classifiers including molecular data of at least two molecules and to the application and comparison of these data obtained from different subjects, different species, different time points of sampling, processed by different people and under varying experimental conditions.

The reference analyte concentrations can be used to determine variable concentrations of other analytes and metabolites varying along with change of experimental conditions, disease, state of disease. pre-treatment, therapy, therapy control and prognosis.

An "endogenous reference metabolite" or an" endogenous reference analyte" means a level of the metabolite that is constant across the experimental conditions and the relative values of the target metabolites referring to the endogenous reference metabolites as standards may be indicative of a particular disease state, phenotype, or lack thereof, as well as combinations of disease states, phenotypes, or lack thereof.

For the purpose of the present invention stability of the endogenous reference metabolites means that
CV is < 0.50, < 0.25, < 0.10, < 0.05 and/or
SD is < 1.0, < 0.5, < 0.25, < 0.10 (on log scale) and/or
M is < 0.5, < 0.25, < 0.10, < 0.05 and/or
rho is < 0.5, < 0.25, < 0.10, < 0.05.

A reference level of an endogenous reference metabolite may be an absolute or relative amount or concentration of the metabolite, a range of amount or concentration of the metabolite, a mean amount or concentration of the endogenous reference metabolite, and/or a median amount or concentration of the endogenous reference metabolite; and, in addition, "reference levels" of combinations of endogenous reference metabolites may also be ratios of absolute or relative amounts or concentrations of two or more metabolites with respect to each other or a composed value / score obtained by classification.
Appropriate reference levels of endogenous reference metabolites for a particular disease state, phenotype, or lack thereof may be determined by measuring levels of desired metabolites in one or more appropriate subjects, and compare to the levels of the endogenous reference metabolites. Metabolite levels as well as reference levels of endogenous reference metabolites may be tailored to specific populations of subjects (e.g., a level may be age-matched so that comparisons may be made between metabolite levels in samples from subjects of a certain age and endogenous reference metabolite levels for a particular disease state, phenotype, or lack thereof in a certain age group). Such metabolite levels as well as reference levels of endogenous reference metabolites may also be tailored to specific techniques that are used to measure levels of metabolites in biological samples (e.g., LC-MS, GC-MS, ELISA, enzymatic tests etc.), where the levels of metabolites or the levels of the endogenous reference metabolites may differ based on the specific technique that is used.
Further features and advantages of the present invention will become evident from the description of examples together with the accompanying drawings.
- Fig. 1: shows a mean-SD-Plot of log-transformed analyte concentrations for piglet data according to example 1;
- Fig. 2: shows a Venn diagram of significant analytes for comparison of start of asphyxia vs. end of asphyxia via log-transformed and housekeeper normalized log-transformed piglet data;
- Fig. 3: shows a Venn diagram of significant analytes for comparison of end of asphyxia vs. end of resuscitation via log-transformed and housekeeper normalized log-transformed piglet data;
- Fig. 4: shows a Venn diagram of significant analytes for comparison of start of asphyxia vs. end of resuscitation via log-transformed and housekeeper normalized log-transformed piglet data;
- Fig. 5: shows a mean-SD-Plot of log-transformed analyte concentrations for sheep plasma data according to example 2;
- Fig. 6: shows a Venn diagram with number of analytes with significant treatment effect for log-transformed and housekeeper normalized log-transformed sheep data;
- Fig. 7: shows a Venn diagram with number of analytes with significant time points effect for log-transformed and housekeeper normalized log-transformed sheep data;
- Fig. 8: shows a Venn diagram with number of analytes with significant interaction between treatment and time points for log-transformed and housekeeper normalized log-transformed sheep data;
- Fig. 9: shows a mean-SD-Plot of log-transformed analyte concentrations for rat brain data according to example 3;
- Fig. 10: shows a Venn diagram with number of analytes with significant differences between at least one of the three groups (OP, Sham, Control) and housekeeper normalized raw rat data;
- Fig. 11: shows a mean-SD-Plot of log-transformed analyte concentrations for human plasma data according to example 4; and
- Fig. 12: shows a Venn diagram with number of analytes with significant differences between at least one of the three groups (Pneumonia, mixed Sepsis, Control) and housekeeper normalized log-transformed human data.

### EXAMPLES

The following examples are provided in order to demonstrate and further illustrate certain preferred embodiments and aspects of the present invention and are not to be construed as limiting the scope thereof.

### General Analytics:

Sample preparation and metabolomic analyses were performed at BIOCRATES Life Sciences AG, Innsbruck, Austria. We used a multi-parametric, highly robust, sensitive and high-throughput targeted metabolomic platform consisting of flow injection analysis (FIA)-MS/MS and LC-MS/MS methods for the simultaneous quantification of a broad range of endogenous intermediates namely acylcarnitines, sphingomyelins, hexoses, glycerophospholipids, amino acids, biogenic amines, bile acids, eicosanoids, and small organic acids (energy metabolism) oxysterols, in plasma and brain samples. All procedures (sample handling, analytics) were performed by co-workers blinded to the groups.

### Sample preparation

### Plasma and serum samples were prepared by standard procedures

Brain samples were thawed on ice for 1 hour and homogenates were prepared by adding phosphate -buffer (phosphate buffered saline, 0.1 µmol/L; Sigma Aldrich, Vienna, Austria) to tissue sample, ratio 3:1 *(w*/*v),* followed by homogenization with a Potter S homogeniser (Sartorius, Goettingen, Germany) at 9 *g* on ice for 1 minute. To enable analysis of all samples simultaneously within one batch, samples were frozen again (-70°C), thawed on ice (1 h) on the day of analysis and centrifuged at *18000 g* at 2°C for 5 min. All tubes were prepared with 0.001% BHT (butylated hydroxytoluene; Sigma-Aldrich, Vienna, Austria) to prevent artificial formation of prostaglandins caused by autooxidation .

### Acylcarnitines, Sphingomyelins, Hexoses, Glycerophospholipids (FIA-MS/MS)

To determine the concentration of acylcarnitines, sphingomyelins and glycerophospholipids in brain homogenates and in plasma the Absolute*IDQ* kit p150 (Biocrates Life Sciences AG) was prepared as described in the manufacturer's protocol. In brief, 10 µL of brain homogenate was added to the center of the filter on the upper 96-well kit plate, and the samples were dried using a nitrogen evaporator (VLM Laboratories). Subsequently, 20 µL of a 5 % solution of phenyl-isothiocyanate was added for derivatization. After incubation, the filter spots were dried again using an evaporator. The metabolites were extracted using 300 µL of a 5 mM ammonium acetate solution in methanol. The extracts were obtained by centrifugation into the lower 96-deep well plate followed by a dilution step with 600 µL of kit MS running solvent. Mass spectrometric analysis was performed on an API4000 QTrap® tandem mass spectrometry instrument (Applied Biosystems/MDS Analytical Technologies) equipped with an electro-spray ionization (ESI)-source using the analysis acquisition method as provided in the Absolute*IDQ* kit. The standard FIA-MS/MS method was applied for all measurements with two subsequent 20 µL injections (one for positive and one for negative mode analysis). Multiple reaction monitoring (MRM) detection was used for quantification applying the spectra parsing algorithm integrated into the MetIQ software (Biocrates Life Sciences AG). Concentration values for 148 metabolites (all analytes determined with the metabolomics kit besides of the amino acids, which were determined by a different method) obtained by internal calibration were exported for comprehensive statistical analysis.

### Amino acids, Biogenic amines (LC-MS/MS)

Amino acids and biogenic amines were quantitatively analyzed by reversed phase LC-MS/MS to obtain chromatographic separation of isobaric (same MRM ion pairs) metabolites for individual quantitation performed by external calibration and by use of internal standards. 10 µL sample volume (plasma, brain homogenate) is required for the analysis using the following sample preparation procedure. Samples were added on filter spots placed in a 96- solvinert well plate (internal standards were placed and dried down under nitrogen before), fixed above a 96 deep well plate (capture plate). 20 µL of 5% phenyl-isothiocyanate derivatization reagent was added. The derivatized samples were extracted after incubation by aqueous methanol into the capture plate. Sample extracts were analyzed by LC-ESI-MS/MS in positive MRM detection mode with an API4000 QTrap® tandem mass spectrometry instrument (Applied Biosystems/MDS Analytical Technologies). The analyzed individual metabolite concentrations (Analyst 1.4.2 software, Applied Biosystems) were exported for comprehensive statistical analysis.

### Bile acids (LC-MS/MS)

A highly selective reversed phase LC-MS/MS analysis method in negative MRM detection mode was applied to determine the concentration of bile acids in plasma samples. Samples were extracted via dried filter spot technique in 96 well plate format, which is well suitable for high throughput analysis. For highly accurate quantitation internal standards and external calibration were applied. In brief, internal standards and 20 µL sample volume placed onto filter spots were extracted and simultaneously protein precipitated with aqueous methanol. These sample extracts were measured by LC-ESI-MS/MS with an API4000 QTrap® tandem mass spectrometry instrument (Applied Biosystems/MDS Analytical Technologies). Data of bile acids were quantified with Analyst 1.4.2 software (Applied Biosystems) and finally exported for comprehensive statistical analysis.

### Prostanoids, oxidized fatty acids (LC-MS/MS)

Prostanoids - a term summarizing prostaglandins (PG), thromboxanes (TX) and prostacylines - and oxidised fatty acid metabolites were analyzed in plasma extracts by LC-ESI-MS/MS *[*Unterwurzacher at al. Clin Chem Lab Med 2008; 46 (11):1589-1597*]* and in brain homogenate extracts by online solid phase extraction (SPE)-LC-MS/MS *[Unterwurzacher et al. Rapid Commun Mass Spec submitted]* with an API4000 QTrap® tandem mass spectrometry instrument (Applied Biosystems/MDS Analytical Technologies) in negative MRM detection mode. The sample preparation was the same for both, plasma and brain homogenates. In brief, filter spots in a 96 well plate were spiked with internal standard; 20 µL of plasma or tissue homogenates were added and extracted with aqueous methanol, the individual extracts then were analysed. Data of prostanoids and oxidized fatty acids were quantified with Analyst 1.4.2 software (Applied Biosystems) and finally exported for statistical analysis.

### Energy metabolism (Organic Acids) (LC-MS/MS)

For the quantitative analysis of energy metabolism intermediates (glycolysis, citrate cycle, pentose phosphate pathway, urea cycle) hdyrophilic interaction liquid chromatography (HILIC)-ESI-MS/MS method in highly selective negative MRM detection mode was used. The MRM detection was performed using an API4000 QTrap® tandem mass spectrometry instrument (Applied Biosystems/MDS Analytical Technologies). 20 µL sample volume (plasma, brain homogenate) was protein precipitated and extracted simultaneously with aqueous methanol in a 96 well plate format. Internal standards (ratio external to internal standard) and external calibration were used for highly accurate quantitation. Data were quantified with Analyst 1.4.2 software (Applied Biosystems) and finally exported for statistical analysis.

### Detailed Examples

Due to the many possibilities for analyzing metabolomics data, we choose several strategies to select the most stable analytes. On the one hand, we use straight forward approaches based on coefficient of variation (CV) and standard deviation (SD) for the selection. One the other hand, we adapt algorithms from real-time quantitative RT-PCR and demonstrate that these methods work well in the context of metabolomics, too. In the framework of real-time quantitative RT-PCR the use of housekeeping genes for normalization is well established and there exist quite a number of procedures which can be used for housekeeping gene (reference gene) selection; confer Vandesompele et al. (2009) [Vandesompele J., Kubista M. and Pfaffl M.W. (2009). Reference Gene Validation Software for Improved Normalization. In: Real-Time PCR: Current Technology and Applications. Caister Academic Press. Editor: Logan J., Edwards K. and Saunders N.].
After the selection of housekeeping analytes, we in a second step demonstrate that the normalization of metabolomics data by housekeeping analytes works well and improves the power of statistical analyses.

**Table 1 a: Common and Systematic Name of Target Metabolites**

| **BC Code** | **Common Name** | **Systematic Name** |
|---|---|---|
| Suc.EM | Succinic acid (succinate) | Butanedioic acid |
| Lac.EM | Lactate | Propanoic acid, 2-hydroxy- |
| C4.K1 | Butyrylcarnitine / Isobutyrylcarnitine | 3-butanoyloxy-4-trimethylammonio-butanoate (L) |
| Fum.EM | Fumaric acid | 2-Butenedioic acid (E)- |
| GCA.BA | Glycocholic Acid | N-(3-alpha,7-alpha,12-alpha-Trihydroxycholan-24-oyl)glycine |
| C16:2.K1 | Hexadecadienoylcarnitine | chain length and number of double bonds is determined by the measured mass, but position and cis-trans-isomerie of double bonds is not specified (generally double bonds are cis) |
| Putrescine.K2 | Putrescine | Putrescine (1,4-Butanediamine) |
| Glu/Gln | | |
| C16:1.K1 | Hexadecenoylcarnitine [Palmitoleylcarnitine] | chain length and number of double bonds is determined by the measured mass, but position and cis-trans-isomerie of double bonds is not specified (generally double bonds are cis) |
| C10:2.K1 | Decadienoylcarnitine | chain length and number of double bonds is determined by the measured mass, but position and cis-trans-isomerie of double bonds is not specified (generally double bonds are cis) |
| TCDCA.BA | Taurochenodeoxycholic Acid | Ethanesulfonic acid, 2-(((3alpha,5beta,7alpha)-3,7-dihydroxy-24-oxocholan-24-yl)amino)- |
| GCDCA.BA | Glycochenodeoxycholic Acid | Glycine, N-((3alpha,5beta,7alpha)-3,7-dihydroxy-24-oxocholan-24-yl)- |
| Spermidine.K2 | Spermidine | 1,4-Butanediamine, N-(3-aminopropyl)- |
| C18:2.K1 | Octadecadienoylcarnitine [Linoleylcarnitine] | chain length and number of double bonds is determined by the measured mass, but position and (Z)-(E)-isomerie of double bonds is not specified |
| CA.BA | Cholic Acid | Cholan-24-oic acid, 3,7,12-trihydroxy-, (3alpha,5beta,7alpha,12alpha)- |
| C5.K1 | Isovalerylcarnitine/2-Methylbutyrylcarmitine / Valerylcarnitine | 3 C5-fatty acids with the same mass as residues (branched/unbranched) |
| Spermine.K2 | Spermine | 1,4-Butanediamine, N,N'-bis(3-aminopropyl)- |
| Pyr + OAA. EM | Pyruvate + Oxaloacetate | |
| C5:1-DC.K1 | Tiglylcarnitine / 3-Methyl-crotonylcarnitine | 2 C5 fatty acids with one double bond as residues |
| C3.K1 | Propionylcarnitine | Propionylcarnitine |
| Lys.K2 | Lysine | L-Lysine |
| alpha-KGA.EM | alpha-Ketoglutaric acid | Pentanedioic acid, 2-oxo- |
| C18:1.K1 | Octadecenoylcarnitine [Oleylcarnitine] | chain length and number of double bonds is determined by the measured mass, but position and cis-trans-isomerie of double bonds is not specified (generally double bonds are cis) |
| C14:2.K1 | Tetradecadienoylicarnitine | chain length and number of double bonds is determined by the measured mass, but position and cis-trans-isomerie of double bonds is not specified (generally double bonds are cis) |
| Asp/Asn | | |
| Putrescine/Orn | | |
| Gln.K2 | Glutamine | L-Glutamine |
| UDCA.BA | Ursodeoxycholic Acid | Cholan-24-oic acid, 3,7-dihydroxy-, (3alpha,5beta,7beta)- |
| PC ae C40:3.K1 | | Glycerophosphocholine with estimated chemical composition (2 residues) |
| Serotonin.K2 | Serotonin | Indol-5-ol, 3-(2-aminoethyl)- |
| Orn/Cit | | |
| Ala.K2 | Alanine | L-Alanine |
| C14.K1 | Tetradecanoylcarnitine | requirement: unbranched fatty acid: only Myristic acid (CAS-Nr: 544-63-8) is possible |
| Ala/BCAA | | |
| His.K2 | Histidine | L-Histidine |
| lysoPC a C16:0.K1 | | Glycerophosphocholine with estimated chemical composition (1 acyl residue) |
| IysoPC a C17:0.K1 | | Glycerophosphocholine with estimated chemical composition (1 acyl residue) |
| C12.K1 | Dodecanoylcarnitine [Laurylcarnitine] | requirement: unbranched fatty acid: only Lauric acid (CAS-Nr: 143-07-7) is possible |
| Pro.K2 | Proline | L-Proline |
| Serotonin/Trp | Serotonin/Tryptophan | |
| C14:2-OH.K1 | 3-Hydroxytetradecadienoylcarnitine | chain length and number of double bonds is determined by the measured mass, but positionof the OH-group and position and cis-trans-isomerie of double bonds is not specified |
| Glu.K2 | Glutamate | L-Glutamic acid |
| C16:2-OH.K1 | 3-Hydroxyhexadecadienoylcarnitine | chain length and number of double bonds is determined by the measured mass, but positionof the OH-group and position and cis-trans-isomerie of double bonds is not specified |
| Histamine.K2 | Histamine | 2-Imidazol-4-ethylamine |
| PC ae C30:0.K1 | | Glycerophosphocholine with estimated chemical composition (2 residues) |
| C14:1.K1 | Tetradecenoylcarnitine [Myristoleylcarnitine] | chain length and number of double bonds is determined by the measured mass, but position and cis-trans-isomerie of double bonds is not specified (generally double bonds are cis) |
| SumLyso | | |
| Phe.K2 | Phenylalanine | L-Phenylalanine |
| lysoPC a C18:0.K1 | | Glycerophosphocholine with estimated chemical composition (1 acyl residue) |
| PC aa C42:4.K1 | | Glycerophosphocholine with estimated chemical composition (2 acyl residues) |
| PC ae C38:4.K1 | | Glycerophosphocholine with estimated chemical composition (2 residues) |
| PC aa C40:3.K1 | | Glycerophosphocholine with estimated chemical composition (2 acyl residues) |
| AA.PA | Arachidonic acid | 5,8,11,14-Eicosatetraenoic acid, (all-Z)- |
| C5:1.K1 | Tiglylcarnitine / 3-Methyl-crotonylcarnitine | 2 C5 fatty acids with one double bond as residues |
| Met-SO.K2 | Methionine-Sulfoxide | Butyric acid, 2-amino-4-(methylsulfinyl)- |
| Spermine/Spermidine | | |
| C16+C18/C0 | | |
| C16.K1 | Hexadecanoylcarnitine [Palmitoylcarnitine] | Palmitylcarnitine (requirement: unbranched fatty acid) |
| lysoPC a C16:1.K1 | | Glycerophosphocholine with estimated chemical composition (1 acyl residue) |
| PC aa C40:4.K1 | | Glycerophosphocholine with estimated chemical composition (2 acyl residues) |
| Asn.K2 | Asparagine | L-Asparagine |
| C9.K1 | Nonanoylcarnitine [Pelargonylcarnitine] | Nonanoylcarnitine (requirement: unbranched fatty acid) |
| PC ae C42:5.K1 | | Glycerophosphocholine with estimated chemical composition (2 residues) |
| C6 (C4:1-DC).K1 | Hexanoylcarnitine [Caproylcarnitine] | mass of possible residues: Caproic acid and Fumaric acid ist similar (116.1) |
| PC aa C40:6.K1 | | Glycerophosphocholine with estimated chemical composition (2 acyl residues) |
| Val.K2 | Valine | L-Valine |
| SumSFA | | |
| PC ae C40:5.K1 | | Glycerophosphocholine with estimated chemical composition (2 residues) |
| PC ae C42:4.K1 | | Glycerophosphocholine with estimated chemical composition (2 residues) |
| PC ae C40:6.K1 | | Glycerophosphocholine with estimated chemical composition (2 residues) |
| Leu.K2 | Leucine | L-Leucine |
| C2.K1 | Acetylcarn itine | 1-Propanaminium, 2-(acetyloxy)-3-carboxy-N,N,N-trimethyl-, inner salt |
| PC aa C40:5.K1 | | Glycerophosphocholine with estimated chemical composition (2 acyl residues) |
| PC ae C42:3.K1 | | Glycerophosphocholine with estimated chemical composition (2 residues) |
| PC ae C40:4.K1 | | Glycerophosphocholine with estimated chemical composition (2 residues) |
| Asp.EM | Aspartic acid | L-Aspartic acid |
| CDCA.BA | Chenodeoxycholic Acid | Cholan-24-oic acid, 3,7-dihydroxy-, (3alpha,5beta,7alpha)- |
| PC aa C38:6.K1 | | Glycerophosphocholine with estimated chemical composition (2 acyl residues) |
| SM (OH) C16:1.K1 | Hydroxysphingomyelin with acyl residue sum C16:1 | Hydroxysphingomyelin with estimated chemical composition |
| PC ae C38:5.K1 | | Glycerophosphocholine with estimated chemical composition (2 residues) |
| C18:1-OH.K1 | 3-Hydroxyoctadecenoylcarnitine [3-Hydroxyoleylcarnitine] | chain length and number of double bonds is determined by the measured mass, but positionof the OH-group and position and cis-trans-isomerie of double bond is not specified |
| Orn/Arg | | |
| C16:1-OH.K1 | 3-Hydroxyhexadecenoylcarnitine [3-Hydroxypalmitoleylcarnitine] | chain length and number of double bonds is determined by the measured mass, but positionof the OH-group and position and cis-trans-isomerie of double bond is not specified |
| C18.K1 | Octadecanoylcarnitine [Stearylcarnitine] | Stearylcarnitine (requirement: unbranched fatty acid) |
| PC aa C36:6.K1 | | Glycerophosphocholine with estimated chemical composition (2 acyl residues) |
| SM C26:1.K1 | | Hydroxysphingomyelin with estimated chemical composition |
| PC aa C42:5.K1 | | Glycerophosphocholine with estimated chemical composition (2 acyl residues) |
| C14:1-OH.K1 | 3-Hydroxytetradecenoylcarnitine [3-Hydroxymyristoleylcarnitine] | chain length and number of double bonds is determined by the measured mass, but position and cis-trans-isomerie of double bonds is not specified (generally double bonds are cis) |
| PC aa C38:4.K1 | | Glycerophosphocholine with estimated chemical composition (2 acyl residues) |
| SumPC+Lyso | | |
| PC ae C36:4.K1 | | Glycerophosphocholine with estimated chemical composition (2 residues) |
| Hex.EM | Hexose | sum of aldohexoses and ketohexoses |
| LCA.BA | Lithocholic acid | Cholan-24-oic acid, 3-hydroxy-, (3alpha,5beta)- |
| PC aa C36:4.K1 | | Glycerophosphocholine with estimated chemical composition (2 residues) |
| SumPC | | |
| SumPUFA | | |
| PC aa C40:2.K1 | | Glycerophosphocholine with estimated chemical composition (2 acyl residues) |
| Met.K2 | Methionine | L-Methionine |
| PC ae C38:3.K1 | | Glycerophosphocholine with estimated chemical composition (2 residues) |
| PC ae C32:2.K1 | | Glycerophosphocholine with estimated chemical composition (2 residues) |
| Cit.K2 | Citrulline | L-Citrulline (L-Ornithine, N5-(aminocarbonyl)-) |
| PC ae C30:1.K1 | | Glycerophosphocholine with estimated chemical composition (2 residues) |
| PC ae C42:2.K1 | | Glycerophosphocholine with estimated chemical composition (2 residues) |
| Kyn/Trp | | |
| Orn.K2 | Ornithine | L-Ornithine |
| PC ae C38:6.K1 | | Glycerophosphocholine with estimated chemical composition (2 residues) |
| lysoPC a C20:4.K1 | | Glycerophosphocholine with estimated chemical composition (1 acyl residue) |
| PC ae C40:1.K1 | | Glycerophosphocholine with estimated chemical composition (2 residues) |
| Asp.K2 | Aspartate | L-Aspartic acid |
| PC aa C30:2.K1 | | Glycerophosphocholine with estimated chemical composition (2 acyl residues) |
| PC aa C28:1.K1 | | Glycerophosphocholine with estimated chemical composition (2 acyl residues) |
| Gly/BCAA | | |
| PC aa C38:5.K1 | | Glycerophosphocholine with estimated chemical composition (2 acyl residues) |
| PC ae C34:1.K1 | | Glycerophosphocholine with estimated chemical composition (2 residues) |
| PC aa C38:3.K1 | | Glycerophosphocholine with estimated chemical composition (2 acyl residues) |
| C16-OH.K1 | 3-Hydroxyhexadecanolycarnitine [3-Hydroxypalmitoylcarnitine] | chain length is determined by the measured mass, but positionof the OH-group is not specified |
| SM (OH) C14:1.K1 | | Hydroxysphingomyelin with estimated chemical composition |
| PC ae C44:4.K1 | | Glycerophosphocholine with estimated |
| PC ae C38:1.K1 | | chemical composition (2 residues) Glycerophosphocholine with estimated chemical composition (2 residues) |
| SumSM | | |
| SumMUFA | | |
| PC ae C40:2.K1 | | Glycerophosphocholine with estimated chemical composition (2 residues) |
| lysoPC a C24:0.K1 | | Glycerophosphocholine with estimated chemical composition (1 acyl residue) |
| OH-Kyn.K2 | Hydroxykynurenine | 3-Hydroxykynurenine |
| SM (OH) C22:1.K1 | | Hydroxysphingomyelin with estimated chemical composition |
| PC ae C32:1.K1 | | Glycerophosphocholine with estimated chemical composition (2 residues) |
| SM C16:1.K1 | | chain length and number of double bonds is determined by the measured mass, but position and cis-trans-isomerie of double bonds is not specified (generally double bonds are cis) |
| PC ae C30:2.K1 | | Glycerophosphocholine with estimated chemical composition (2 residues) |
| Ala/Lys | | |
| Tyr. K2 | Tyrosine | L-Tyrosine |
| PC ae C34:0.K1 | | Glycerophosphocholine with estimated chemical composition (2 residues) |
| PC ae C36:0.K1 | | Glycerophosphocholine with estimated chemical composition (2 residues) |
| SM (OH) C22:2.K1 | | Hydroxysphingomyelin with estimated chemical composition |
| lysoPC a C28:0.K1 | | Glycerophosphocholine with estimated chemical composition (1 acyl residue) |
| C12:1.K1 | Dodecenoylcarnitine | chain length and number of double bonds is determined by the measured mass, but position and cis-trans-isomerie of double bonds is not specified (generally double bonds are cis) |
| PC aa C34:4.K1 | | Glycerophosphocholine with estimated chemical composition (2 acyl residues) |
| SM C26:0.K1 | sphingomyelin with acyl residue sum C26:0 | Sphingomyelin with estimated chemical composition |
| SM (OH) C24:1.K1 | Hydroxysphingomyelin with acyl residue sum C24:1 | Hydroxysphingomyelin with estimated chemical composition |
| SM C16:0.K1 | sphingomyelin with acyl residue sum C16:0 | Sphingomyelin with estimated chemical composition |
| Ile.K2 | Isoleucine | L-Isoleucine |
| C5-DC (C6-OH).K1 | | Acylcarnitine with estimated composition: mass of 2 possible residues is similar |
| PC aa C38:0.K1 | | Glycerophosphocholine with estimated |
| PC aa C34:2.K1 | | chemical composition (2 acyl residues) Glycerophosphocholine with estimated chemical composition (2 acyl residues) |
| Cit/Arg | | |
| Ser.K2 | Serine | L-Serine |
| PC ae C36:5.K1 | | Glycerophosphocholine with estimated chemical composition (2 residues) |
| PC ae C34:2.K1 | | Glycerophosphocholine with estimated chemical composition (2 residues) |
| PC ae C36:3.K1 | | Glycerophosphocholine with estimated chemical composition (2 residues) |
| C3-OH.K1 | Hydroxypropionylcarnitine | chain length is determined by the measured mass, but positionof the OH-group is not specified |
| PC ae C42:1.K1 | | Glycerophosphocholine with estimated chemical composition (2 residues) |
| H1.K1 | | sum of aldohexoses and ketohexoses |
| PC ae C36:2.K1 | | Glycerophosphocholine with estimated chemical composition (2 residues) |
| SM C22:3.K1 | sphingomyelin with acyl residue sum C22:3 | Sphingomyelin with estimated chemical composition |
| SM C24:1.K1 | sphingomyelin with acyl residue sum C24:1 | Sphingomyelin with estimated chemical composition |
| C4-OH (C3-DC).K1 | 3-Hydroxybutyrylcarnitine | Acylcarnitine with estimated composition: mass of 2 possible residues is similar |
| PC aa C40:1.K1 | | Glycerophosphocholine with estimated chemical composition (2 acyl residues) |
| PC aa C32:3.K1 | | Glycerophosphocholine with estimated chemical composition (2 acyl residues) |
| PC aa C42:1.K1 | | Glycerophosphocholine with estimated chemical composition (2 acyl residues) |
| PC aa C36:5.K1 | | Glycerophosphocholine with estimated chemical composition (2 acyl residues) |
| PC aa C42:6.K1 | | Glycerophosphocholine with estimated chemical composition (2 acyl residues) |
| DHA.PA | Docosahexaenoic acid | 4,7,10,13,16,19-Docosahexaenoic acid, (all-Z)- |
| SM C20:2.K1 | sphingomyelin with acyl residue sum C20:2 | Sphingomyelin with estimated chemical composition |
| C4:1.K1 | Butenoylcarnitine | chain length is determined by the measured mass, but positionof the double bond is not specified |
| SM C24:0.K1 | sphingomyelin with acyl residue sum C24:0 | Sphingomyelin with estimated chemical composition |
| PC ae C38:0.K1 | | Glycerophosphocholine with estimated chemical composition (2 residues) |
| Kyn/OHKyn | | |
| Arg.K2 | Arginine | L-Arginine |
| total DMA.K2 | | |
| PC aa C34:3.K1 | | Glycerophosphocholine with estimated chemical composition (2 acyl residues) |
| PC ae C38:2.K1 | | Glycerophosphocholine with estimated chemical composition (2 residues) |
| PUFA/MUFA | | |
| PC aa C42:0.K1 | | Glycerophosphocholine with estimated chemical composition (2 acyl residues) |
| SM C18:1.K1 | | Sphingomyelin with estimated chemical composition |
| PC aa C32:2.K1 | | Glycerophosphocholine with estimated chemical composition (2 residues) |
| lysoPC a C18:2.K1 | | Glycerophosphocholine with estimated chemical composition (1 acyl residue) |
| PC ae C44:3.K1 | | Glycerophosphocholine with estimated chemical composition (2 residues) |
| PC ae C40:0.K1 | | Glycerophosphocholine with estimated chemical composition (2 residues) |
| XIe.K2 | Leucine + Isoleucine | |
| PC aa C24:0.K1 | | Glycerophosphocholine with estimated chemical composition (2 acyl residues) |
| PC aa C38:1.K1 | | Glycerophosphocholine with estimated chemical composition (2 acyl residues) |
| SM C18:0.K1 | | Sphingomyelin with estimated chemical composition |
| PC aa C42:2.K1 | | Glycerophosphocholine with estimated chemical composition (2 acyl residues) |
| lysoPC a C20:3.K1 | | Glycerophosphocholine with estimated chemical composition (1 acyl residue) |
| PC ae C36:1.K1 | | Glycerophosphocholine with estimated chemical composition (2 residues) |
| C3:1.K1 | Propenoylcarnitine | |
| lysoPC a C18:1.K1 | | Glycerophosphocholine with estimated chemical composition (1 acyl residue) |
| C8:1.K1 | Octenoylcarnitine | chain length and number of double bonds is determined by the measured mass, but position and cis-trans-isomerie of double bonds is not specified (generally double bonds are cis) |
| C8.K1 | Octanoylcarnitine [Caprylylcarnitine] | |
| PC aa C30:0.K1 | | Glycerophosphocholine with estimated chemical composition (2 acyl residues) |
| PC ae C44:5.K1 | | Glycerophosphocholine with estimated chemical composition (2 residues) |
| lysoPC a C14:0.K1 | | Glycerophosphocholine with estimated chemical composition (1 acyl residue) |
| Creatinine.K2 | Creatinine | 4H-Imidazol-4-one, 2-amino-1,5-dihydro-1-methyl- |
| C0.K1 | Carnitine (free) | (3-Carboxy-2-hydroxypropyl)trimethylammonium hydroxide inner salt |
| Thr.K2 | Threonine | L-Threonine ((2S,3R)-2-amino-3-hydroxybutanoic acid) |
| Phe/Tyr | | |
| Gly.K2 | Glycine | Glycine |
| PC aa C26:0.K1 | | Glycerophosphocholine with estimated chemical composition (2 acyl residues) |
| C5-OH (C3-DC-M).K1 | 3-Hydroxyisovalerylcarnitine / 3-Hydroxy-2-methylbutyryl | Acylcarnitine with estimated composition: mass of 2 possible residues is similar |
| PC aa C34:1.K1 | | Glycerophosphocholine with estimated chemical composition (2 acyl residues) |
| lysoPC a C28:1.K1 | | Glycerophosphocholine with estimated chemical composition (1 acyl residue) |
| Met-SO/Met | | |
| C10:1.K1 | Decenoylcarnitine | chain length and number of double bonds is determined by the measured mass, but position and cis-trans-isomerie of double bonds is not specified (generally double bonds are cis) |
| PC aa C36:0.K1 | | Glycerophosphocholine with estimated chemical composition (2 acyl residues) |
| SDMA.K2 | Symmetrical Dimethylarginine | N,N'-Dimethyl-L-arginine |
| Trp.K2 | Tryptophan | L-Tryptophan |
| PC ae C34:3.K1 | | Glycerophosphocholine with estimated chemical composition (2 residues) |
| PC aa C36:2.K1 | | Glycerophosphocholine with estimated chemical composition (2 acyl residues) |
| PC aa C36:1.K1 | | Glycerophosphocholine with estimated chemical composition (2 acyl residues) |
| Kyn.K2 | Kynurenine | alpha-2-Diamino-gamma-oxobenzenebutyric acid |
| PC aa C32:1.K1 | | Glycerophosphocholine with estimated chemical composition (2 residues) |
| C7-DC.K1 | Pimelylcarnitine | |
| SDMA/ADMA | | |
| PUFA/SFA | | |
| Arg.EM | Arginine | L-Arginine |
| PC aa C36:3.K1 | | Glycerophosphocholine with estimated chemical composition (2 acyl residues) |
| 13S-HODE.PA | 13(S)-hydroxy-9Z,11 E-octadecadienoic acid | |
| PC ae C44:6.K1 | | Glycerophosphocholine with estimated chemical composition (2 residues) |
| lysoPC a C6:0.K1 | | Glycerophosphocholine with estimated chemical composition (1 acyl residue) |
| ADMA.K2 | asymmetrical Dimethylarginin | N,N-Dimethyl-L-arginine |
| PC aa C32:0.K1 | | Glycerophosphocholine with estimated chemical composition (2 residues) |
| SumSMOH/SumSM MUFA/SFA | | |
| PC ae C42:0.K1 | | Glycerophosphocholine with estimated chemical composition (2 residues) |
| C6:1.K1 | Hexenoylcarnitine | chain length and number of double bonds is determined by the measured mass, but position and cis-trans-isomerie of double bonds is not specified (generally double bonds are cis) |
| lysoPC a C26:1.K1 | | Glycerophosphocholine with estimated chemical composition (1 acyl residue) |
| C12-DC.K1 | Dodecanedioylcarnitine | |
| C10.K1 | Decanoylcarnitine [Caprylcarnitine] (Fumarylcarnitine) | chain length is determined by the measured mass, condition unbranched fatty acid: Capric acid as residue |

**Table 1b: CAS-Numbers and Target Metabolites Species With The Same Structure**

| **BC Code** | **CAS Registry Number** | **Species with the same structure:** |
|---|---|---|
| Suc.EM | 110-15-6 | |
| Lac.EM | 50-21-5 | (s)-2-Hydroxypropanoic acid, CAS-NR:79-33-4; (r)-2-Hydroxypropanoic acid, CAS-Nr: 10326-41-7 |
| C4.K1 | 25576-40-3 | 3-butanoyloxy-4-trimethylammonio-butanoate (D) CAS-Nr: 25518-46-1 |
| Fum.EM | 110-17-8 | (Z)-2-Butenedioic acid (Maleic acid), CAS-Nr: 110-16-7 |
| GCA.BA | 475-31-0 | |
| C16:2.K1 | | |
| Putrescine.K2 | 110-60-1 | |
| Glu/Gln | | |
| C16:1.K1 | | |
| C10:2.K1 | | |
| TCDCA.BA | 516-35-8 | Tauroursodeoxycholic acid (Ethanesulfonic acid, 2-(((3-alpha,5-beta,7-beta)-3,7-dihydroxy-24-oxocholan-24-yl)amino)-), CAS-Nr: 14605-22-2 |
| GCDCA.BA | 640-79-9 | Glycine, N-((3alpha,5beta,7beta)-3,7-dihydroxy-24-oxocholan-24-yl)-, CAS-Nr: 2273-95-2 |
| Spermidine.K2 | 124-20-9 | |
| C18:2.K1 | | |
| CA.BA | 81-25-4 | (11 stereo centers = 2048 isomers, one is natural cholic acid), Allocholic acid CAS-Nr: 2464-18-8; Ursocholic acid Cas-Nr: 2955-27-3 |
| C5.K1 | | |
| Spermine.K2 | 71-44-3 | |
| Pyr + OAA.EM | | |
| C5:1-DC.K1 | | |
| C3.K1 | | |
| Lys.K2 | 56-87-1 | DL-Lysine CAS-Nr: 70-54-2; D-Lysine CAS-Nr: 923-27-3 |
| alpha-KGA.EM | 328-50-7 | |
| C18:1.K1 | | |
| C14:2.K1 | | |
| Asp/Asn | | |
| Putrescine/Orn | | |
| Gln.K2 | 56-85-9 | DL-Glutamine CAS-Nr: 6899-04-3; D-Glutamine CAS-Nr: 5959-95-5 |
| UDCA.BA | 128-13-2 | Chenodiol (Cholan-24-oic acid, 3,7-dihydroxy-, (3alpha,5beta,7alpha)-) CAS-Nr: 474-25-9;Cholan-24-oic acid, 3,7-dihydroxy-,(3beta,5beta,7alpha)- CAS-Nr: 566-24-5; Isoursodeoxycholic acid (Cholan-24-oic acid, 3,7-dihydroxy-, (3beta,5beta,7beta)-) CAS-Nr: 78919-26-3 |
| PC ae C40:3.K1 | | |
| Serotonin.K2 | 50-67-9 | |
| Orn/Cit | | |
| Ala.K2 | 56-41-7 | DL-Alanine CAS-Nr: 302-72-7; D-Alanine CAS-Nr: 338-69-2; beta-Alanine CAS-Nr: 107-95-9 |
| C14.K1 | | |
| Ala/BCAA | | |
| His.K2 | 71-00-1 | DL-Histidine CAS-NR: 4998-57-6;D-Histidine CAS-Nr: 351-50-8 |
| lysoPC a C16:0.K1 | | Position and character of residue (a/e) is not clear! (m(lysoPC a C16.0) = m(lysoPC e C17.0)) |
| lysoPC a C17:0.K1 | | |
| C12.K1 | | |
| Pro.K2 | 147-85-3 | |
| Serotonin/Trp | | |
| C14:2-OH.K1 | | |
| Glu.K2 | 56-86-0 | DL-Glutamic acid CAS-Nr: 617-65-2; D-Glutamic acid CAS-Nr: 6893-26-1 |
| C16:2-OH.K1 | | |
| Histamine.K2 | 51-45-6 | |
| PC ae C30:0.K1 | | |
| C14:1.K1 | | |
| SumLyso | | |
| Phe.K2 | 63-91-2 | DL-Phenylalanine CAS-Nr: 150-30-1; D-Phenylalanine CAS-Nr: 673-06-3 |
| lysoPC a C18:0.K1 | | |
| PC aa C42:4.K1 | | |
| PC ae C38:4.K1 | | |
| PC aa C40:3.K1 | | |
| AA.PA | 506-32-1 | 8,11,14,17-Eicosatetraenoic acid CAS-Nr: 2091-26-1; 5,11,14,17-Eicosatetraenoic acid CAS-Nr: 2271-31-0; 5,8,11,14-Eicosatetraenoic acid CAS-Nr: 7771-44-0 ; theorretically as combination of E and Z double bonds is possible |
| C5:1.K1 | | |
| Met-SO.K2 | 62697-73-8 | Methionine S-oxide (L-Methionine sulfoxide) CAS-Nr: 3226-65-1; Butanoic acid, 2-amino-4-(methylsulfinyl)-, (S-(R*,S*))- CAS-NR:50896-98-5 |
| Spermine/Spermidine | | |
| C16+C18/C0 | | |
| C16.K1 | 1935-18-8 | Palmitoyl-D(+)-carnitin CAS-Nr: 2364-66-1; Palmitoyl-L-(-)-carnitin CAS-Nr: 2364-67-2 |
| IysoPC a C16:1.K1 | | |
| PC aa C40:4.K1 | | |
| Asn.K2 | 70-47-3 | DL-Asparagine CAS-Nr: 3130-87-8; D-Asparagine CAS-Nr: 2058-58-4 |
| C9.K1 | | |
| PC ae C42:5.K1 | | |
| C6 (C4:1-DC).K1 | | |
| PC aa C40:6.K1 | | |
| Val.K2 | 72-18-4 | DL-Valine CAS-Nr: 516-06-3; D-Valine CAS-Nr: 640-68-6 |
| SumSFA | | |
| PC ae C40:5.K1 | | |
| PC ae C42:4.K1 | | |
| PC ae C40:6.K1 | | |
| Leu.K2 | 61-90-5 | DL-Leucine CAS-Nr: 328-39-2; D-Leucine CAS-Nr: 328-38-1 |
| C2.K1 | 14992-62-2 | R-Acetylcarnitine CAS-Nr:3040-38-8 |
| PC aa C40:5.K1 | | |
| PC ae C42:3.K1 | | |
| PC ae C40:4.K1 | | |
| Asp.EM | 56-84-8 | Aspartic acid CAS-Nr: 617-45-8; D-Aspartic acid CAS-Nr: 1783-96-6 |
| CDCA.BA | 474-25-9 | 8 possible isomers (3a,5a,7a; 3a,5a,7b; 3a,5b,7a; 3a,5b,7b; 3b,5a,7a; 3b,5a,7b; 3b,5b,7a; 3b,5b,7b); Found with CAS-numbers: Cholan-24-oic acid, 3,7-dihydroxy-, (3alpha,5beta,7beta)- (Ursodeoxycholic acid) CAS-Nr: 128-13-2; Cholan-24-oic acid, 3,7-dihydroxy-, (3beta,5beta,7alpha)- CAS-Nr: 566-24-S;Cholan-24-oic acid, 3,7-dihydroxy-, (3beta,5beta,7beta)-(Isoursodeoxycholic acid) CAS-Nr: 78919-26-3 |
| PC aa C38:6.K1 | | |
| SM (OH) C16:1.K1 | | |
| PC ae C38:5.K1 | | |
| C18:1-OH.K1 | | |
| Orn/Arg | | |
| C16:1-OH.K1 | | |
| C18.K1 | | |
| PC aa C36:6.K1 | | |
| SM C26:1.K1 | | |
| PC aa C42:5.K1 | | |
| C14:1-OH.K1 | | |
| PC aa C38:4.K1 | | |
| SumPC+Lyso | | |
| PC ae C36:4.K1 | | |
| Hex.EM | | 8 Aldohexoses (D-Form) most common in nature: D-Glucose, D-Galactose und D-Mannose, 4 Ketohexoses (D-Form): D-Psicose, D-Fructose, D-Sorbose, D-Tagatose |
| LCA.BA | 434-13-9 | Cholan-24-oic acid, 3-hydroxy-, (3beta,5beta)-(Isolithocholic acid) CAS-Nr: 1534-35-6;Cholan-24-oic acid, 3-hydroxy-, (3beta,5alpha)- (9CI) (Alloisolithocholic acid) CAS-Nr:2276-93-9 |
| PC aa C36:4.K1 | | |
| SumPC | | |
| SumPUFA | | |
| PC aa C40:2.K1 | | |
| Met.K2 | 63-68-3 | DL-Methionine CAS-Nr:59-51-8; D-Methionine CAS-Nr: 348-67-4 |
| PC ae C38:3.K1 | | |
| PC ae C32:2.K1 | | |
| Cit.K2 | 372-75-8 | DL-2-Amino-5-ureidovaleric acid CAS-Nr:627-77-0 |
| PC ae C30:1.K1 | | |
| PC ae C42:2.K1 | | |
| Kyn/Trp | | |
| Orn.K2 | 70-26-8 | DL-Ornithine CAS-NR: 616-07-9; D-Ornithine CAS-Nr: 348-66-3 |
| PC ae C38:6.K1 | | |
| lysoPC a C20:4.K1 | | |
| PC ae C40:1.K1 | | |
| Asp.K2 | 56-84-8 | D,L-Aspartic acid CAS-Nr: 617-45-8; D-Aspartic acid CAS-Nr: 1783-96-6 |
| PC aa C30:2.K1 | | |
| PC aa C28:1.K1 | | |
| Gly/BCAA | | |
| PC aa C38:5.K1 | | |
| PC ae C34:1.K1 | | |
| PC aa C38:3.K1 | | |
| C16-OH.K1 | | |
| SM (OH) C14:1.K1 | | |
| PC ae C44:4.K1 | | |
| PC ae C38:1.K1 | | |
| SumSM | | |
| SumMUFA | | |
| PC ae C40:2.K1 | | |
| lysoPC a C24:0.K1 | | |
| OH-Kyn.K2 | 484-78-6 | L-3-Hydroxykynurenine CAS-Nr: 606-14-4; 5-Hydroxykynurenine CAS-Nr: 720-00-3 |
| SM (OH) C22:1.K1 | | |
| PC ae C32:1.K1 | | |
| SM C16:1.K1 | | |
| PC ae C30:2. K1 | | |
| Ala/Lys | | |
| Tyr.K2 | 60-18-4 | DL-Tyrosine CAS-Nr: 556-03-6; D-Tyrosine CAS-Nr: 556-02-5 |
| PC ae C34:0.K1 | | |
| PC ae C36:0.K1 | | |
| SM (OH) C22:2.K1 | | |
| lysoPC a C28:0.K1 | | |
| C12:1.K1 | | |
| PC aa C34:4.K1 | | |
| SM C26:0.K1 | | |
| SM (OH) C24:1.K1 | | |
| SM C16:0.K1 | | |
| Ile.K2 | 73-32-5 | DL-Isoleucine CAS-Nr: 443-79-8; Allo-L-Isoleucine CAS-Nr: 1509-34-8; Allo-D-Isoleucine CAS-Nr: 1509-35-9; Allo-DL-Isoleucine CAS-Nr: 3107-04-8 |
| C5-DC (C6-OH).K1 | | |
| PC aa C38:0.K1 | | |
| PC aa C34:2.K1 | | |
| Cit/Arg | | |
| Ser.K2 | 56-45-1 | DL-Serine CAS-Nr: 302-84-1; D-Serine CAS-Nr: 312-84-5 |
| PC ae C36:5.K1 | | |
| PC ae C34:2.K1 | | |
| PC ae C36:3.K1 | | |
| C3-OH.K1 | | |
| PC ae C42:1.K1 | | |
| H1.K1 | | 8 Aldohexoses (D-Form) most common in nature: D-Glucose, D-Galactose und D-Mannose, 4 Ketohexoses (D-Form): D-Psicose, D-Fructose, D-Sorbose, D-Tagatose |
| PC ae C36:2.K1 | | |
| SM C22:3.K1 | | |
| SM C24:1.K1 | | |
| C4-OH (C3-DC).K1 | | |
| PC aa C40:1.K1 | | |
| PC aa C32:3.K1 | | |
| PC aa C42:1.K1 | | |
| PC aa C36:5.K1 | | |
| PC aa C42:6.K1 | | |
| DHA.PA | 6217-54-5 | cis-trans-isomerie of double bonds is not specified: CAS-Nr: 25167-62-8; |
| SM C20:2.K1 | | |
| C4:1.K1 | | |
| SM C24:0.K1 | | |
| PC ae C38:0.K1 | | |
| Kyn/OHKyn | | |
| Arg.K2 | 74-79-3 | DL-Arginine CAS-Nr: 7200-25-1; D-Arginine CAS-Nr:157-06-2 |
| total DMA.K2 | | |
| PC aa C34:3.K1 | | |
| PC ae C38:2.K1 | | |
| PUFA/MUFA | | |
| PC aa C42:0.K1 | | |
| SM C18:1.K1 | | |
| PC aa C32:2.K1 | | |
| lysoPC a C18:2.K1 | | |
| PC ae C44:3.K1 | | |
| PC ae C40:0.K1 | | |
| Xle.K2 | | |
| PC aa C24:0.K1 | | |
| PC aa C38:1.K1 | | |
| SM C18:0.K1 | | |
| PC aa C42:2.K1 | | |
| IysoPC a C20:3.K1 | | |
| PC ae C36:1.K1 | | |
| C3:1.K1 | | |
| IysoPC a C18:1.K1 | | |
| C8:1.K1 | | |
| C8.K1 | | |
| PC aa C30:0.K1 | | |
| PC ae C44:5.K1 | | |
| lysoPC a C14:0.K1 | | |
| Creatinine.K2 | 60-27-5 | |
| C0.K1 | 461-06-3 | DL-Carnitine CAS-Nr: 406-76-8; D-Carnitine CAS-Nr: 541-14-0 |
| Thr.K2 | 72-19-5 | DL-Threonine CAS-Nr: 80-68-2; D-Threonine CAS-Nr: 632-20-2; Allo-DL-Threonine ((2S,3S)-2-amino-3-hydroxybutanoic acid) CAS-Nr: 144-98-9 |
| Phe/Tyr | | |
| Gly.K2 | 56-40-6 | |
| PC aa C26:0.K1 | | |
| C5-OH (C3-DC- | | |
| M).K1 | | |
| PC aa C34:1.K1 | | |
| lysoPC a C28:1.K1 | | |
| Met-SO/Met | | |
| C10:1.K1 | | |
| PC aa C36:0.K1 | | |
| SDMA.K2 | 30344-00-4 | |
| Trp.K2 | 73-22-3 | DL-Tryptophan CAS-Nr: 54-12-6; D-Tryptophan CAS-Nr: 153-94-6 |
| PC ae C34:3.K1 | | |
| PC aa C36:2.K1 | | |
| PC aa C36:1.K1 | | |
| Kyn.K2 | 343-65-7 | |
| PC aa C32:1.K1 | | |
| C7-DC.K1 | | |
| SDMA/ADMA | | |
| PUFA/SFA | | |
| Arg.EM | 74-79-3 | DL-Arginine CAS-Nr: 7200-25-1; D-Arginine CAS-Nr:157-06-2 |
| PC aa C36:3.K1 | | |
| 13S-HODE.PA | | 13-Hydroxyoctadecadienoic acid CAS-Nr: 5204-88-6; 9,11-Octadecadienoic acid, 13-hydroxy-, (R-(E,Z))- CAS-Nr: 10219-69-9 |
| PC ae C44:6.K1 | | |
| lysoPC a C6:0.K1 | | |
| ADMA.K2 | 30315-93-6 | N,N-Dimethyl-L-arginine CAS-Nr: 102783-24-4 |
| PC aa C32:0.K1 | | |
| SumSMOH/SumSM | | |
| MUFA/SFA | | |
| PC ae C42:0.K1 | | |
| C6:1.K1 | | |
| lysoPC a C26:1.K1 | | |
| C12-DC.K1 | | |
| C10.K1 | | |
| lysoPC a C26:0.K1 | | |

Table 1 a and 1b summarize analyzed target metabolites and respective abbreviations which are valid also for the following tables; Glycerophospholipids are further differentiated with respect to the presence of ester (a) and ether (e) bonds in the glycerol moiety, where two letters (aa, ea, or ee) denote that the first and the second position of the glycerol scaffold are bound to a fatty acid residue, whereas a single letter (a or e) indicates a bond with only one fatty acid residue; e.g. PC_ea_33:1 denotes a plasmalogen phosphatidylcholine with 33 carbons in the two fatty acid side chains and a single double bond in one of them. The designation ".K1 or .K2" at the end of a compound designation is an internal designation used by the Applicant, which has no chemical meaning.

### 1. Asphyxia

Piglets were subjected to asphyxia. To "mimic" birth asphyxia we exposed the whole body to hypoxia by ventilating piglets with 8% oxygen and added CO2 to achieve hypercarbia. Hypotension was used to cause ischaemic damage and occurred as a result of the hypercarbic hypoxia.

### Experimental procedure:

The National Animal Research Authority, (NARA), approved the experimental protocol. The animals were cared for and handled in accordance with the European Guidelines for Use of Experimental Animals. The Norwegian Council for Animal Research approved the experimental protocol. The animals were cared for and handled in accordance with the European Guidelines for Use of Experimental Animals, by certified FELASA (Federation of European Laboratory Animals Science Association). Thirty-two newborn Noroc (LYxLD) pigs (12-36 h old) were included in the study. In addition we had a reference group consisting of 6 newborn pigs going through all procedures.

### Surgical preparation and anesthesia.

Anesthesia was induced by giving Sevofluran 5% (Sevorane, Abbott); an ear vein was cannulated, the piglets were given Pentobarbital sodium 15 mg/kg and Fentanyl 50 µg/kg intravenously as a bolus injection. The piglets were orally intubated then placed on their back and washed for sterile procedures. Anesthesia was maintained by continuous infusion of Fentanyl (50 µg/kg/h) and Midazolam (0.25 mg/kg/h) in mixtures giving 1 ml/kg/h for each drug applied by IVAC P2000 infusion pump. When necessary, a bolus of Fentanyl (10 µg/kg), Midazolam (1 mg/kg) or Pentobarbital (2.5 mg/kg) was added (need for medication being defined as shivering, trigging on the respirator, increased tone assessed by passive movements of the limbs, increase in blood pressure and/or pulse). A continuous IV Infusion (Salidex: saline 0.3% and glucose 3.5%, 10 mL/kg/h) was given until hypoxia and from 15 min after start of resuscitation and throughout the experiment.
The piglets were ventilated with a pressure-controlled ventilator (Babylog 8000+; Dragerwerk, Lübeck, Germany). Normoventilation (arterial carbon dioxide tension (PaCO₂) 4.5-5.5 kPa) and a tidal volume of 6-8 mL/kg were achieved by adjusting the peak inspiratory pressure or ventilatory rate. Ventilatory rate was 15-40 respirations/min. Inspiratory time of 0.4 s and positive end-expiratory pressure of 4.5 cm H₂O was kept constant throughout the experiment. Inspired fraction of O₂ and end-tidal CO₂ was monitored continuously (Datex Normocap Oxy; Datex, Helsinki, Finland).
The left femoral artery was cannulated with polyethylene catheters (Porex PE-50, inner diameter 0.58mm; Porex Ltd Hythe, Kent, UK). Mean arterial bloodpressure (MABP) was measured continuously in the left femoral artery using BioPac systems MP150-CE. Rectal temperature was maintained between 38.5 and 39.5°C with a heating blanket and a radiant heating lamp. One hour of stabilization was allowed after surgery. At the end of the experiment, the piglets were given an overdose of 150 mg/kg pentobarbital intravenously. (Eye enucleation at 15 (30 Gr 3) and 60min)

### Experimental protocol:

Hypoxemia was achieved by ventilation with a gas mixture of 8% O₂ in N₂ until either mean arterial blood pressure decreased to 20 mm Hg or base excess (BE) reached -20 mM. CO₂ was added during hypoxemia aiming at a PaCO₂ of 8.0-9.5 kPa, to imitate perinatal asphyxia. Before start of resuscitation, the hypoxic piglets were block-randomized for resuscitation with 21% or 100% oxygen for 15 min and then ventilation with room air for 45 min (group 1 and 2), or to receive 100% oxygen for 60 min (group 3). After initiating the reoxygenation, the piglets were kept normocapnic (PaCO₂ 4.5-5.5 kPa). Throughout the whole experiment there was a continuous surveillance of blood pressure, saturation, pulse, temperature and blood gas measurements. Hemoglobin was measured on a HemoCue Hb 201+ (HemoCue AB, Angelholm, Sweden) at baseline and at the end. Temperature-corrected arterial acid/base status and glucose were measured regularly throughout the experiment on a Blood Gas Analyzer 860 (Ciba Corning Diagnostics, Midfield, Mass., USA). Blood samples for Metabolomics were drawn before initiating the hypoxia, at the end of hypoxia and 60 min after initiating reoxygenation and handled according to the Biocrates protocol. Plasma or serum were prepared according to a standard protocol and then stored at minus 70 °C until subsequent analysis. All blood samples obtained from the femoral artery catheter were replaced by saline 1.5 x the volume drawn. One hour after the end of hypoxia the animals were given an overdose of pentobarbital (150 mg/kg iv). The study staff and the laboratory personnel were blinded to the percentage oxygen administered by resuscitation.

### Determination and quantification of oxysterols in biological samples by LC-MS/MS

Oxysterols are determined by HPLC-Tandem mass spectrometer (HPLC-API-MS/MS) in positive detection mode using Multiple Reaction Mode (MRM).
20 µL samples, calibrators or internal standard mixture were placed into a capture plate and were protein precipitated by addition of 200 µL acetonitrile and centrifugation. 180 µL of the appropriate supernatants were transferred on a new filter plate with 7 mm filter spots and dried under a nitrogen stream. The analytes were hydrolyzed by addition of 100 µL 0.35 M KOH in 95 % EtOH followed by a 2 h incubation in the dark. The reaction mixture was dried and washed three times with 200 µL water. The oxysterols were extracted with 100 µL 90 % aqueous MeOH. 20 µL of the extracted sample are injected onto the HPLC-MS/MS system. Chromatographic separation and detection is performed by using a Zorbax Eclipse XDB C18, 150 x 2.0 mm, 3.5 µm HPLC-Column at a flow rate of 0.3 mL/min followed by electrospray ionization on the API4000 tandem mass spectrometer. For the quantitation the Analyst Quantitation software from Applied Bioystems was used.

### Selection of Housekeeping Analytes

We use data of 32 piglets for each of the time points SA (Start of Asphyxia), EA (End of Asphyxia) and ER (End of Resuscitation).
In a first step, we use the raw data and sort the analytes by the coefficient of variation (CV) which is defined as the ratio of the standard deviation (SD) to the mean; i.e., CV = SD/mean. Table 2 shows the top 20 analytes with smallest CV. In addition we give SD and mean of the raw concentrations.

**Table 2: Top 20 analytes with smallest CV (based on raw data).**

| **Nr** | **Analyte** | **CV** | **SD** | **mean** |
|---|---|---|---|---|
| 1 | lysoPC a C26:1.K1 | 0,03 | 0,0571 | 1,6451 |
| 2 | PC aa C26:0.K1 | 0,0380 | 0,0240 | 0,6314 |
| 3 | C12-DC.K1 | 0,0585 | 0,0089 | 0,1530 |
| 4 | lysoPC a C26:0.K1 | 0,0703 | 0,0316 | 0,4492 |
| 5 | C8.K1 | 0,1008 | 0,0157 | 0,1555 |
| 6 | C8:1.K1 | 0,1079 | 0,0108 | 0,1005 |
| 7 | PC ae C40:0.K1 | 0,1161 | 0,5832 | 5,0218 |
| 8 | PC ae C42:0.K1 | 0,1171 | 0,0558 | 0,4760 |
| 9 | lysoPC a C28:1.K1 | 0,1177 | 0,0353 | 0,2996 |
| 10 | PC aa C40:1.K1 | 0,1213 | 0,0575 | 0,4741 |
| 11 | C3-OH.K1 | 0,1279 | 0,0089 | 0,0696 |
| 12 | C10.K1 | 0,1313 | 0,0361 | 0,2751 |
| 13 | lysoPC a C28:0.K1 | 0,1317 | 0,0521 | 0,3954 |
| 14 | C6 (C4:1-DC).K1 | 0,1339 | 0,0188 | 0,1402 |
| 15 | C6:1.K1 | 0,1414 | 0,0097 | 0,0688 |
| 16 | C3:1.K1 | 0,1420 | 0,0062 | 0,0435 |
| 17 | C10:1.K1 | 0,1452 | 0,0218 | 0,1502 |
| 18 | C5-OH (C3-DC-M).K1 | 0,1497 | 0,0286 | 0,1911 |
| 19 | PCaeC42:5.K1 | 0,1500 | 0,1105 | 0,7367 |
| 20 | PC aa C24:0.K1 | 0,1570 | 0,0215 | 0,1369 |

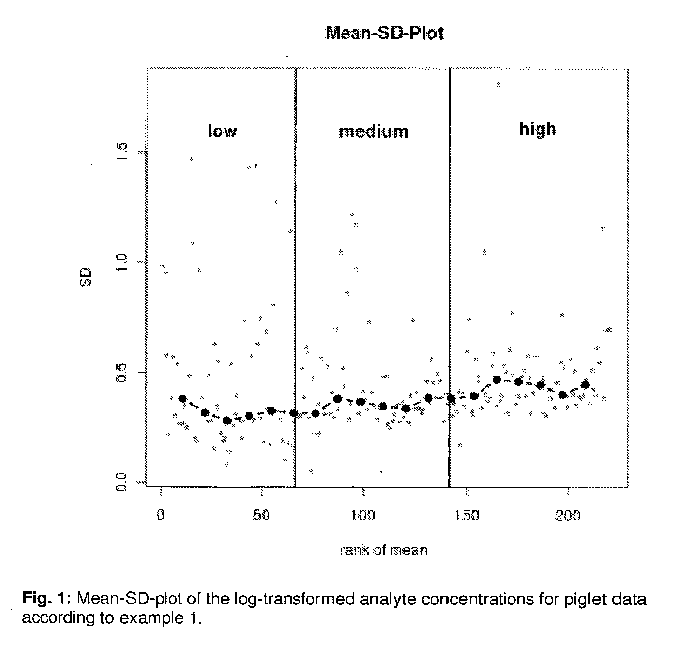

As one often uses the log-transformed analyte concentrations for further analysis, where the log-transformation is used to stabilize variance and to obtain at least approximately normal distributed data, we in a second step compute mean and SD for the log-transformed data. Of course, one could also use other transformations like for instance Box-Cox power transformations [Box G.E.P. and Cox D.R. (1964) An analysis of transformations (with discussion). Journal of the Royal Statistical Society B, 26, 211-252.], the generalized log-transformation via vsn [Huber W., von Heydebreck A., Sueltmann H., Poustka A., Vingron M. (2002). Variance stabilization applied to microarray data calibration and to the quantification of differential expression. Bioinformatics 18 Suppl.1 S96-S104.] or vst [Lin S.M., Du P., Huber W., Kibbe, W.A. (2008). Model-based variance-stabilizing transformation for Illumina microarray data. Nucleic Acids Research, Vol. 36, No. 2 e11.] or some other normalization procedure which is well-known from microarray normalization in this step. A comparative survey of normalization procedures in case of Affymetrix microarray data is given in Cope et al. (2004) [L.M. Cope et al. (2004). A Benchmark for Affymetrix GeneChip Expression Measures, Bioinformatics 20(3):323-331] or Irizarry et al. (2006) [R.A. Irizarry et al. (2006). Comparison of Affymetrix GeneChip Expression Measures, Bioinformatics 22(7):789-794]. Figure 1 shows that the variance stabilization via the log-transformation works well but not perfectly. Consequently, lower mean log-concentrations tend to have smaller SDs. Since we are looking for analytes with the smallest variability in terms of SD, we split the log-concentration in three parts (low, medium, high) to avoid getting only analytes with low concentrations. Analytes are classified as low concentrated if their mean concentration is smaller than 0.5 (i.e., mean log2-concentration < -1), as medium concentrated if their mean concentration is between 0.5 and 4 (i.e., mean log2-concentration between -1 and 2), and as high concentrated if their mean concentration is larger than 4 (i.e., mean log2-concentration > 2). This leads to three approximately equally large groups as shown in Figure 1. Of course, the choice of the groups is rather arbitrary and one could use other cut-off values and more groups, respectively. Choosing the 10 top ranked analytes, i.e., with lowest SD values, for low, medium and high concentrated analytes, respectively, we obtain the analytes depicted in Table 3.

**Table 3: 10 top ranked analytes for each case; i.e., with smallest SD for low, medium and high concentrated analytes, respectively (based on log-transformed data).**

| **Nr** | **Analyte** | **group** | **SD** | **mean** |
|---|---|---|---|---|
| 1 | C12-DC.K1 | low | 0,084 | -2,711 |
| 2 | lysoPC a C26:0.K1 | low | 0,102 | -1,158 |
| 3 | C8.K1 | low | 0,144 | -2,692 |
| 4 | C8:1.K1 | low | 0,157 | -3,323 |
| 5 | PC ae C42:0.K1 | low | 0,172 | -1,081 |
| 6 | lysoPC a C28:1.K1 | low | 0,172 | -1,749 |
| 7 | PCaaC40:1.K1 | low | 0,177 | -1,087 |
| 8 | C10.K1 | low | 0,185 | -1,874 |
| 9 | C3-OH.K1 | low | 0,187 | -3,856 |
| 10 | C6 (C4:1-DC).K1 | low | 0,191 | -2,847 |
| 11 | lysoPC a C26:1.K1 | medium | 0,050 | 0,717 |
| 12 | PC aa C26:0.K1 | medium | 0,055 | -0,664 |
| 13 | PC ae C42:5.K1 | medium | 0,221 | -0,457 |
| 14 | SM C20:2.K1 | medium | 0,222 | -0,628 |
| 15 | SM (OH) C22:2.K1 | medium | 0,245 | 0,744 |
| 16 | IysoPC a C16:1.K1 | medium | 0,268 | 0,740 |
| 17 | PC aa C30:0.K1 | medium | 0,272 | 1,085 |
| 18 | SM (OH) C16:1.K1 | medium | 0,275 | 0,752 |
| 19 | PC aa C32:2.K1 | medium | 0,276 | 1,017 |
| 20 | SM (OH) C14:1.K1 | medium | 0,277 | 0,977 |
| 21 | PC ae C40:0.K1 | high | 0,172 | 2,318 |
| 22 | PC ae C34:1.K1 | high | 0,304 | 2,132 |
| 23 | Met-PTC.K1 | high | 0,305 | 4,931 |
| 24 | PC aa C32:1.K1 | high | 0,310 | 2,756 |
| 25 | Phe.K2 | high | 0,314 | 4,697 |
| 26 | Phe-PTC.K1 | high | 0,317 | 4,915 |
| 27 | Trp-PTC.K1 | high | 0,317 | 3,889 |
| 28 | PC ae C36:2.K1 | high | 0,327 | 2,176 |
| 29 | C0.K1 | high | 0,328 | 2,741 |
| 30 | IysoPC a C18:1.K1 | high | 0,341 | 3,040 |

Beside the above straight forward approaches we use two algorithms which are known to work well for identifying housekeeping genes (reference genes) in case of real-time quantitative RT-PCR data. First, we apply the method of Vandesompele et al. [Vandesompele et al. (2002). Accurate normalization of real-time quantitative RT-PCR data by geometric averaging of multiple internal control genes. Genome Biology, 3(7):research0034.1-0034.11.] which is called geNorm. That is, we rank the analytes by the stability measure M introduced by Vandesompele et al. and in each step remove the analyte with the largest M value, i.e. the lowest stability. As the geNorm procedure is based on analyte ratios, the two most stable analytes cannot be ranked. geNorm ranks the analytes according to the similarity of the concentration profiles. Hence, the results are quite distinct from the results of the other selection methods (cf. Table 6) and indicate that there are two groups of analytes which have very similar concentration profiles and hence dominate the selection process; confer Table 4 where the 20 top ranked analytes are depicted.

**Table 4: 20 top ranked analytes using geNorm.**

| **Nr** | **Analyte** | **Rank** | **mean M** |
|---|---|---|---|
| 1 | SM (OH) C14:1.K1 | 1 | 0,087 |
| 2 | PC ae C30:1.K1 | 1 | 0,087 |
| 3 | SM (OH) C16:1.K1 | 3 | 0,103 |
| 4 | PC aa C28:1.K1 | 4 | 0,113 |
| 5 | PC ae C40:2.K1 | 5 | 0,123 |
| 6 | PC ae C38:3.K1 | 6 | 0,130 |
| 7 | PC ae C38:2.K1 | 7 | 0,132 |
| 8 | SM (OH) C22:1.K1 | 8 | 0,140 |
| 9 | PC ae C38:1.K1 | 9 | 0,145 |
| 10 | PC ae C34:1.K1 | 10 | 0,148 |
| 11 | PC ae C36:3.K1 | 11 | 0,151 |
| 12 | PC ae C34:2.K1 | 12 | 0,153 |
| 13 | PC ae C40:3.K1 | 13 | 0,156 |
| 14 | PC ae C40:4.K1 | 14 | 0,160 |
| 15 | PC ae C40:5.K1 | 15 | 0,163 |
| 16 | PC ae C42:4.K1 | 16 | 0,165 |
| 17 | SM (OH) C22:2.K1 | 17 | 0,168 |
| 18 | SM (OH) C24:1.K1 | 18 | 0,17 |
| 19 | SM C18:1.K1 | 19 | 0,172 |
| 20 | PC ae C38:4.K1 | 20 | 0,174 |

Finally, we use the method introduced by Andersen et al. (2004) [Andersen et al. (2004). Normalization of Real-Time Quantitative Reverse Transcription-PCR Data: A Model-Based Variance Estimation Approach to Identify Genes Suited for Normalization, Applied to Bladder and Colon Cancer Data Sets. Cancer Research 64:5245-5250.] which is called NormFinder. That is, we rank the analytes by the stability measure rho introduced by Andersen et al. where in each step the analyte with the smallest rho value, i.e. the highest stability, given the previously selected analytes is added. The NormFinder procedure takes the inter and intra group variability of the analyte concentrations into account where we consider the groups (time points) SA (Start of Asphyxia), EA (End of Asphyxia) and ER (End of Resuscitation).
Table 5 shows the 50 top ranked analytes.

**Table 5: 50 top ranked analytes using NormFinder.**

| **Nr** | **Analyte** | **Rank** | **rho** |
|---|---|---|---|
| 1 | C8.K1 | 1 | 0,0729 |
| 2 | C6 (C4:1-DC).K1 | 2 | 0,0441 |
| 3 | PC aa C26:0.K1 | 3 | 0,0374 |
| 4 | C16-OH.K1 | 4 | 0,0307 |
| 5 | C14:2-OH.K1 | 5 | 0,0318 |
| 6 | C4-OH (C3-DC).K1 | 6 | 0,0282 |
| 7 | lysoPC a C26:1.K1 | 7 | 0,0281 |
| 8 | C12:1.K1 | 8 | 0,0254 |
| 9 | C6:1.K1 | 9 | 0,0224 |
| 10 | C7-DC.K1 | 10 | 0,0230 |
| 11 | C16:2-OH.K1 | 11 | 0,0226 |
| 12 | C4:1.K1 | 12 | 0,0214 |
| 13 | Arg-PTC.K1 | 13 | 0,0216 |
| 14 | C18:1-OH.K1 | 14 | 0,0205 |
| 15 | Val-PTC.K1 | 15 | 0,0201 |
| 16 | C3-OH.K1 | 16 | 0,0191 |
| 17 | C16:1-OH.K1 | 17 | 0,0193 |
| 18 | C10:1.K1 | 18 | 0,0182 |
| 19 | C9.K1 | 19 | 0,0187 |
| 20 | C2.K1 | 20 | 0,0170 |
| 21 | C12-DC.K1 | 21 | 0,0175 |
| 22 | Val. K2 | 22 | 0,0172 |
| 23 | PC aa C24:0.K1 | 23 | 0,0162 |
| 24 | lysoPC a C6:0.K1 | 24 | 0,0162 |
| 25 | C14:1-OH.K1 | 25 | 0,0166 |
| 26 | Arg.K2 | 26 | 0,0171 |
| 27 | C10.K1 | 27 | 0,0163 |
| 28 | lysoPC a C26:0.K1 | 28 | 0,0168 |
| 29 | Orn-PTC.K1 | 29 | 0,0153 |
| 30 | lysoPC a C28:0.K1 | 30 | 0,0151 |
| 31 | Tyr.K2 | 31 | 0,0155 |
| 32 | PC aa C40:1.K1 | 32 | 0,0156 |
| 33 | C12.K1 | 33 | 0,0150 |
| 34 | C3:1.K1 | 34 | 0,0140 |
| 35 | Arg.EM | 35 | 0,0149 |
| 36 | SM C26:0.K1 | 36 | 0,0148 |
| 37 | C18.K1 | 37 | 0,0148 |
| 38 | lysoPC a C28:1.K1 | 38 | 0,0141 |
| 39 | Phe.K2 | 39 | 0,0149 |
| 40 | PC ae C44:5.K1 | 40 | 0,0129 |
| 41 | C5-DC (C6-OH).K1 | 41 | 0,0139 |
| 42 | Orn.K2 | 42 | 0,0140 |
| 43 | SM C18:1.K1 | 43 | 0,0128 |
| 44 | C5-OH (C3-DC-M).K1 | 44 | 0,0139 |
| 45 | C14.K1 | 45 | 0,0135 |
| 46 | SM C24:1.K1 | 46 | 0,0117 |
| 47 | PC ae C44:6.K1 | 47 | 0,0129 |
| 48 | Phe-PTC.K1 | 48 | 0,0122 |
| 49 | C8:1.K1 | 49 | 0,0120 |
| 50 | His-PTC.K1 | 50 | 0,0129 |

Table 6 shows a summary of all analytes which were chosen by at least one of the four different methods where the selection is indicated by TRUE. There are several analytes which were chosen by two or even three methods simultaneously.

**Table 6: Summary of the selected analytes. TRUE indicates that an analyte was chosen by the corresponding algorithm.**

| **Nr** | **Analyte** | **CV of raw data** | **SD of log data** | **geNorm** | **NormFinder** |
|---|---|---|---|---|---|
| 1 | Arg.EM | | | | TRUE |
| 2 | Arg.K2 | | | | TRUE |
| 3 | Arg-PTC.K1 | | | | TRUE |
| 4 | C0.K1 | | TRUE | | |
| 5 | C10:1.K1 | TRUE | | | TRUE |
| 6 | C10.K1 | TRUE | TRUE | | TRUE |
| 7 | C12:1.K1 | | | | TRUE |
| 8 | C12-DC.K1 | TRUE | TRUE | | TRUE |
| 9 | C12.K1 | | | | TRUE |
| 10 | C14:1-OH.K1 | | | | TRUE |
| 11 | C14:2-OH.K1 | | | | TRUE |
| 12 | C14.K1 | | | | TRUE |
| 13 | C16:1-OH.K1 | | | | TRUE |
| 14 | C16:2-OH.K1 | | | | TRUE |
| 15 | C16-OH.K1 | | | | TRUE |
| 16 | C18:1-OH.K1 | | | | TRUE |
| 17 | C18.K1 | | | | TRUE |
| 18 | C2.K1 | | | | TRUE |
| 19 | C3:1.K1 | TRUE | | | TRUE |
| 20 | C3-OH.K1 | TRUE | TRUE | | TRUE |
| 21 | C4:1.K1 | | | | TRUE |
| 22 | C4-OH (C3-DC).K1 | | | | TRUE |
| 23 | C5-DC (C6-OH).K1 | | | | TRUE |
| 24 | C5-OH (C3-DC-M).K1 | TRUE | | | TRUE |
| 25 | C6:1.K1 | TRUE | | | TRUE |
| 26 | C6 (C4:1-DC).K1 | TRUE | TRUE | | TRUE |
| 27 | C7-DC.K1 | | | | TRUE |
| 28 | C8:1.K1 | TRUE | TRUE | | TRUE |
| 29 | C8.K1 | TRUE | TRUE | | TRUE |
| 30 | C9.K1 | | | | TRUE |
| 31 | His-PTC.K1 | | | | TRUE |
| 32 | IysoPC a C16:1.K1 | | TRUE | | |
| 33 | IysoPC a C18:1.K1 | | TRUE | | |
| 34 | lysoPC a C26:0.K1 | TRUE | TRUE | | TRUE |
| 35 | lysoPC a C26:1.K1 | TRUE | TRUE | | TRUE |
| 36 | lysoPC a C28:0.K1 | TRUE | | | TRUE |
| 37 | lysoPC a C28:1.K1 | TRUE | TRUE | | TRUE |
| 38 | lysoPC a C6:0.K1 | | | | TRUE |
| 39 | Met-PTC.K1 | | TRUE | | |
| 40 | Orn.K2 | | | | TRUE |
| 41 | Orn-PTC.K1 | | | | TRUE |
| 42 | PC aa C24:0.K1 | TRUE | | | TRUE |
| 43 | PC aa C26:0.K1 | TRUE | TRUE | | TRUE |
| 44 | PC aa C28:1.K1 | | | TRUE | |
| 45 | PC aa C30:0.K1 | | TRUE | | |
| 46 | PC aa C32:1.K1 | | TRUE | | |
| 47 | PC aa C32:2.K1 | | TRUE | | |
| 48 | PC aa C40:1.K1 | TRUE | TRUE | | TRUE |
| 49 | PC ae C30:1.K1 | | | TRUE | |
| 50 | PC ae C34:1.K1 | | TRUE | TRUE | |
| 51 | PC ae C34:2.K1 | | | TRUE | |
| 52 | PC ae C36:2.K1 | | TRUE | | |
| 53 | PC ae C36:3.K1 | | | TRUE | |
| 54 | PC ae C38:1.K1 | | | TRUE | |
| 55 | PC ae C38:2.K1 | | | TRUE | |
| 56 | PC ae C38:3.K1 | | | TRUE | |
| 57 | PC ae C38:4.K1 | | | TRUE | |
| 58 | PC ae C40:0.K1 | TRUE | TRUE | | |
| 59 | PC ae C40:2.K1 | | | TRUE | |
| 60 | PC ae C40:3.K1 | | | TRUE | |
| 61 | PC ae C40:4.K1 | | | TRUE | |
| 62 | PC ae C40:5.K1 | | | TRUE | |
| 63 | PC ae C42:0.K1 | TRUE | TRUE | | |
| 64 | PC ae C42:4.K1 | | | TRUE | |
| 65 | PC ae C42:5.K1 | TRUE | TRUE | | |
| 66 | PC ae C44:5.K1 | | | | TRUE |
| 67 | 67 PC ae C44:6.K1 | | | | TRUE |
| 68 | Phe.K2 | | TRUE | | TRUE |
| 69 | Phe-PTC.K1 | | TRUE | | TRUE |
| 70 | 70 SM C18:1.K1 | | | TRUE | TRUE |
| 71 | SM C20:2.K1 | | TRUE | | |
| 72 | 72 SM C24:1.K1 | | | | TRUE |
| 73 | SM C26:0.K1 | | | | TRUE |
| 74 | SM (OH) C14:1.K1 | | TRUE | TRUE | |
| 75 | SM (OH) C16:1.K1 | | TRUE | TRUE | |
| 76 | SM (OH) C22:1.K1 | | | TRUE | |
| 77 | SM (OH) C22:2.K1 | | TRUE | TRUE | |
| 78 | SM (OH) C24:1.K1 | | | TRUE | |
| 79 | Trp-PTC.K1 | | TRUE | | |
| 80 | Tyr.K2 | | | | TRUE |
| 81 | Val.K2 | | | | TRUE |
| 82 | Val-PTC.K1 | | | | TRUE |

### Statistical Analysis

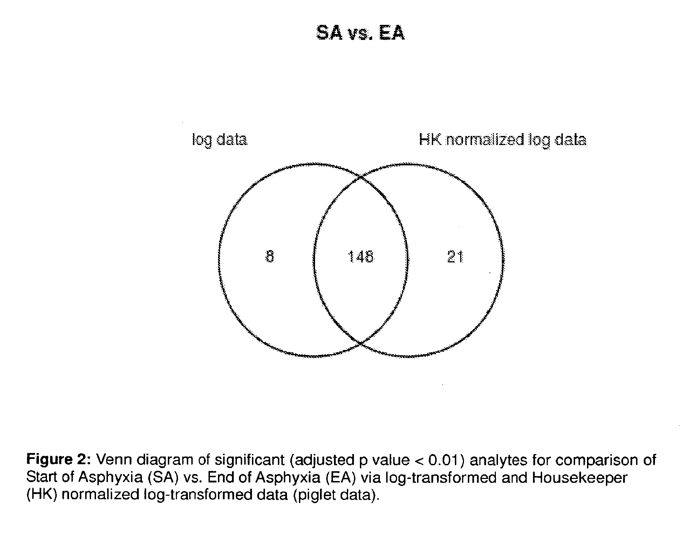

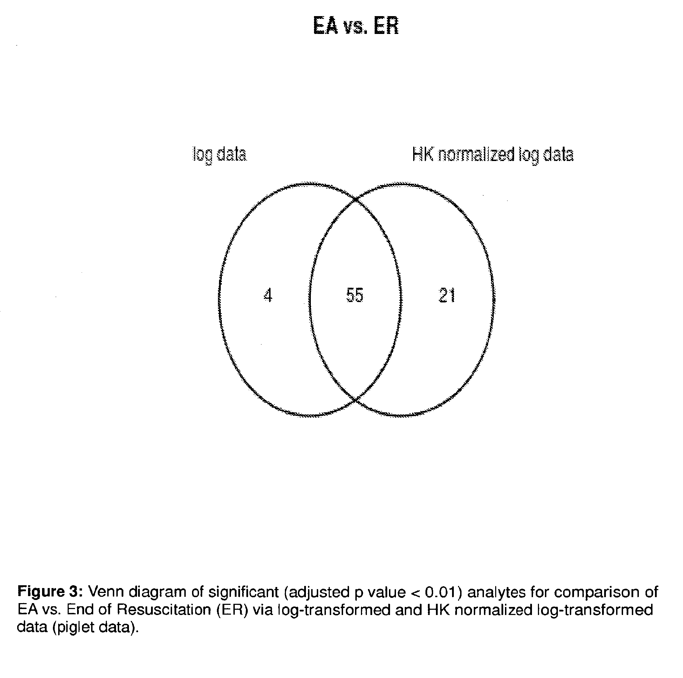

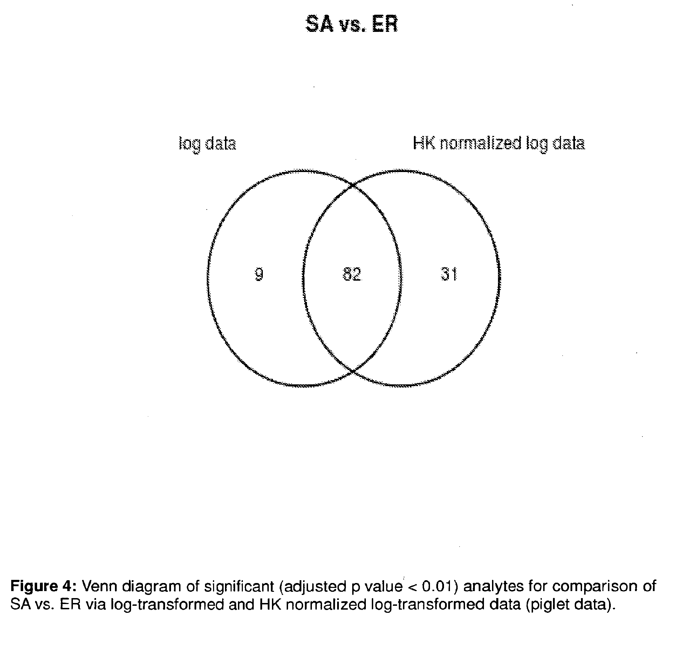

We perform paired t-tests for the comparison of the three groups (time points) SA (Start of Asphyxia), EA (End of Asphyxia) and ER (End of Resuscitation) and adjust the p value of these three pairwise comparisons via the method of Bonferroni-Holm [Holm, S. (1979). A simple sequentially rejective multiple test procedure. Scandinavian Journal of Statistics 6:65-70]. The obtained adjusted p values are further adjusted for testing multiple analytes via the method of Benjamini and Hochberg (1995) [Benjamini Y., and Hochberg Y. (1995). Controlling the false discovery rate: a practical and powerful approach to multiple testing. Journal of the Royal Statistical Society Series B 57:289-300]. As housekeeping analytes we used C8.K1, C6 (C4:1-DC).K1, PC aa C26:0.K1 which were top ranked by the NormFinder algorithm. We compare the results for log-transformed and housekeeper (HK) normalized log-transformed data. The normalization by means of housekeeping analytes is performed by subtracting the mean of log-transformed concentrations of the three selected housekeepers from the log-transformed concentrations of the other analytes; i.e., in terms of the raw concentration this means that we use the logarithm of the ratio between the raw concentration of all analytes (except the housekeepers) and the geometric mean of the housekeepers for the statistical analysis. The Venn diagrams in Figures 2-4 are based on all metabolites having adjusted p values smaller than 0.01 and show that there is a large agreement between the results for the three pairwise comparisons. However, in all cases more significant differences are detected in case of the HK normalized log-transformed data indicating that the use of the HK normalized log-transformed data leads to an increased power of the statistical analysis.
The assumption of an increased power is confirmed by area under curve (AUC) computations; i.e., for each of the pairwise comparisons and each of the analytes we compute the AUC which is a measure for the predictive power of the single analytes. From the 216 analytes included in this analysis 142 (SA vs. EA), 158 (EA vs. ER) and 131 (SA vs. ER) have a larger AUC in case of the HK normalized log-transformed data. The corresponding proportions 65.7% (p < 0.001), 73.1% (p < 0.001) and 60.6% (p = 0.002), respectively, are significantly different (i.e., larger) from 50%. At the same time the maximum AUC stays about the same 0.915 (log data) vs. 0.912 (HK), 0.881 (log data) vs. 0.867 (HK) and 0.889 (log data) vs. 0.887 (HK) for SA vs. EA, EA vs. ER and SA vs. ER, respectively. Hence, this again speaks for an increased power of the statistical analysis by using the HK normalized log-transformed data instead of the log-transformed data.

### 2. Lipopolysaccharides (LPS)

Host response to pathogens was investigated with LPS vs. control, a well known animal model for mammalian immune response, at various timepoints 0h, 2h, ..., 240h - in sheep plasma.

Twelve fetal sheep were instrumented at 97-99 days gestation (term = 147 days) under general anesthesia (1.5% isofluorane in O₂) using sterile techniques (Mallard et al., 2003). Stesolid (0.1-0.2mg/kg, iv) was used as pre-medication and anaesthesia was induced by Pentothal Sodium (13mg/kg, iv) followed by intubation. Temgesic (0.01 mg/kg, iv) was administered at the time of surgery and during the first 2 post-operative days. A small hysterectomy incision was made over the fetal head through the uterine wall, parallel to any vessels. Polyvinyl catheters were placed in both axillary arteries, one axillary vein and in the amniotic cavity. The fetal scalp overlying the parasagittal cortex was exposed and two bilateral holes drilled through the skull but avoiding the dura at 5 and 15 mm anterior of bregma and 0.5 mm lateral of midline. Two pairs of EEG electrodes (P/N Ag7/40T, Leico Ind, Medwire® MT. Vernon, NY) were inserted through the burr holes and secured to the skull with a small rubber disk glued with cyanoacrylate and skin flaps glued back over the electrodes. A reference electrode was placed subcutaneously anterior to the EEG electrodes and one ground electrode subcutaneously in the neck. At the end of the operation, catheters were filled with 50E/ml heparin in saline. The uterus was closed in two layers and catheters and electrodes exteriorized. One catheter was placed in the tarsal vein of the ewe. After surgery, ewes were housed in individual cages with free access to food and water. Animals were allowed to recover from surgery for at least four days before experimentation, during which time intravenous (i.v.) antibiotics (Gentamycin, 5 mg/kg) were administered to the ewe once daily. These studies were approved by the Animal Ethical Committee of the University of Göteborg (ethical number: 307-2006).

### Experimental procedure

At 101-106 days (term=145 days) chronically instrumented fetal sheep were randomly assigned to receive a bolus vehicle (saline, n=5) or lipopolysaccharide (LPS, Sigma O55:B5, 200ng, n=7) injection, iv. Continuous mean fetal arterial blood pressure (MAP) and amnion cavity pressure were recorded on BIOPAC Systems (MPA150) for at least 12 hours prior to injection and for 10 days after injection. EEG data were recorded before and after LPS injection using a clinical BRM2 BrainZ monitor (New Zealand). Blood samples (1 ml) were collected on ice for blood gas analysis (pH, pCO₂ kPa, SO₂%, glucose mmol/l and lactate mmol/l, Radiometer ABL 725, Copenhagen, Denmark). Remaining blood was centrifuged and serum immediately frozen and stored at -80C for further analysis as described below.

### Selection of Housekeeping Analytes

We use data of twelve sheeps where data are obtained at 0h, 2h, 6h, 24h, 48h, 72h, 96h, 120h, 144h, 168h, 192h, 216h, and 240h after injection.
In a first step, we use the raw data and sort the analytes by the coefficient of variation (CV) which is defined as the ratio of the standard deviation (SD) to the mean; i.e., CV = SD/mean. Table 7 shows the top 20 analytes with smallest CV. In addition we give SD and mean of the raw concentrations.

**Table 7: Top 20 analytes with smallest CV (based on raw data).**

| **Nr** | **Analyte** | **CV** | **SD** | **mean** |
|---|---|---|---|---|
| 1 | PC aa C26:0.K1 | 0,080 | 0,039 | 0,489 |
| 2 | C12-DC.K1 | 0,118 | 0,006 | 0,054 |
| 3 | PC ae C40:0.K1 | 0,131 | 0,775 | 5,921 |
| 4 | PC aa C40:1.K1 | 0,132 | 0,037 | 0,278 |
| 5 | lysoPC a C26:1.K1 | 0,147 | 0,332 | 2,254 |
| 6 | C5-M-DC.K1 | 0,154 | 0,005 | 0,032 |
| 7 | C16:1.K1 | 0,154 | 0,003 | 0,021 |
| 8 | C12.K1 | 0,158 | 0,003 | 0,021 |
| 9 | C6 (C4:1-DC).K1 | 0,165 | 0,004 | 0,027 |
| 10 | C8.K1 | 0,171 | 0,008 | 0,049 |
| 11 | Suc.EM | 0,172 | 1,247 | 7,243 |
| 12 | lysoPC a C26:0.K1 | 0,179 | 0,057 | 0,321 |
| 13 | PC ae C44:6.K1 | 0,179 | 0,013 | 0,070 |
| 14 | lysoPC a C28:0.K1 | 0,193 | 0,038 | 0,196 |
| 15 | C18:1-OH.K1 | 0,199 | 0,002 | 0,009 |
| 16 | C16:2-OH.K1 | 0,201 | 0,002 | 0,010 |
| 17 | C14.K1 | 0,202 | 0,004 | 0,020 |
| 18 | PC aa C42:0.K1 | 0,203 | 0,010 | 0,049 |
| 19 | lysoPC a C28:1.K1 | 0,214 | 0,046 | 0,213 |
| 20 | Val.K2 | 0,217 | 51,534 | 237,556 |

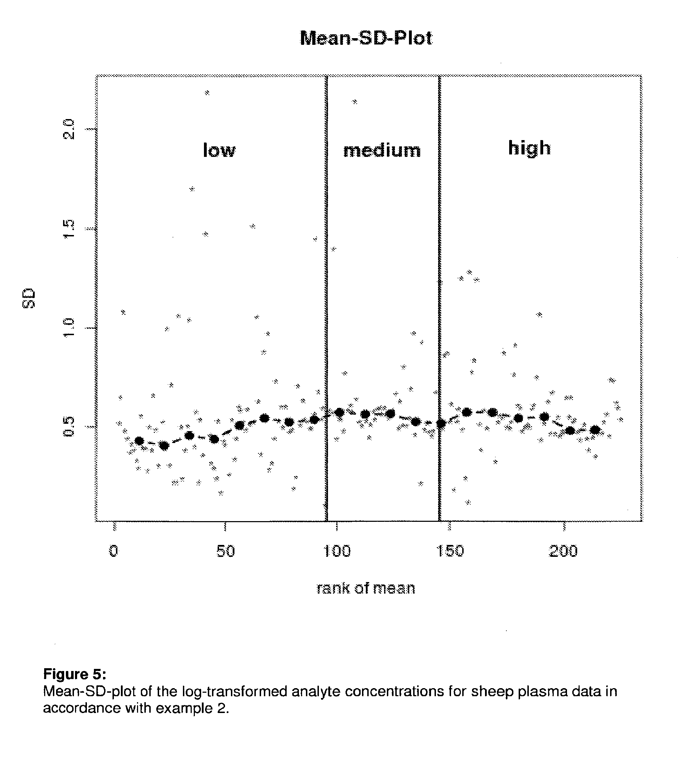

As one often uses the log-transformed analyte concentrations for further analysis, where the log-transformation is used to stabilize variance and to obtain at least approximately normal distributed data, we in a second step compute mean and SD for the log-transformed data. Of course, one could also use other transformations like for instance Box-Cox power transformations [Box, G. E. P. and Cox, D. R. (1964) An analysis of transformations (with discussion). Journal of the Royal Statistical Society B, 26, 211-252.], the generalized log-transformation via vsn [Huber W., von Heydebreck A., Sueltmann H., Poustka A., Vingron M. (2002). Variance stabilization applied to microarray data calibration and to the quantification of differential expression. Bioinformatics 18 Suppl.1 S96-S104.] or vst [Lin S.M., Du P., Huber W., Kibbe, W.A. (2008). Model-based variance-stabilizing transformation for Illumina microarray data. Nucleic Acids Research, Vol. 36, No. 2 e11.] or some other normalization procedure which is well-known from microarray normalization in this step. A comparative survey of normalization procedures in case of Affymetrix microarray data is given in Cope et al. (2004) [L.M. Cope et al. (2004). A Benchmark for Affymetrix GeneChip Expression Measures, Bioinformatics 20(3):323-331] or Irizarry et al. (2006) [R.A. Irizarry et al. (2006). Comparison of Affymetrix GeneChip Expression Measures, Bioinformatics 22(7):789-794]. Figure 5 shows that the variance stabilization works well but not perfectly. Consequently, lower mean log-concentrations tend to have smaller SDs. Since we are interested in the analytes with the smallest SDs, we split the log-concentration in three parts (low, medium, high) to avoid getting only analytes with low concentrations. Analytes are classified as low concentrated if their mean concentration is smaller than 0.5 (i.e., mean log2-concentration < -1), as medium concentrated if their mean concentration is between 0.5 and 4 (i.e., mean log2-concentration between -1 and 2), and as high concentrated if their mean concentration is larger than 4 (i.e., mean log2-concentration > 2). Of course, the choice of the groups is rather arbitrary and one could use other cut-off values and more groups, respectively. Choosing the 10 top ranked analytes, i.e., with lowest SD values, for low, medium and high concentrated analytes, respectively, we obtain the analytes depicted in Table 8.

**Table 8: 10 top ranked analytes for each case, i.e., with smallest SD for low, medium and high concentrated analytes, respectively (based on log-transformed data).**

| **Nr** | **Analyte** | **group** | **SD** | **mean** |
|---|---|---|---|---|
| 1 | PC aa C26:0.K1 | low | 0,107 | -1,037 |
| 2 | C12-DC.K1 | low | 0,168 | -4,230 |
| 3 | PC aa C40:1.K1 | low | 0,189 | -1,860 |
| 4 | C16:1.K1 | low | 0,222 | -5,559 |
| 5 | C5-M-DC.K1 | low | 0,223 | -4,975 |
| 6 | C12.K1 | low | 0,224 | -5,568 |
| 7 | C8.K1 | low | 0,239 | -4,365 |
| 8 | C6 (C4:1-DC).K1 | low | 0,243 | -5,252 |
| 9 | lysoPC a C26:0.K1 | low | 0,247 | -1,659 |
| 10 | PC ae C44:6.K1 | low | 0,261 | -3,855 |
| 11 | lysoPC a C26:1.K1 | medium | 0,213 | 1,157 |
| 12 | PC aa C38:0.K1 | medium | 0,443 | -0,817 |
| 13 | PC aa C30:2.K1 | medium | 0,447 | -0,335 |
| 14 | SM C16:1.K1 | medium | 0,454 | 1,483 |
| 15 | PC aa C38:1.K1 | medium | 0,463 | 0,975 |
| 16 | PC aa C36:0.K1 | medium | 0,471 | 1,448 |
| 17 | SM C18:1.K1 | medium | 0,478 | 1,494 |
| 18 | PC ae C38:1.K1 | medium | 0,481 | -0,793 |
| 19 | PC aa C30:0.K1 | medium | 0,484 | 1,378 |
| 20 | PC aa C32:2.K1 | medium | 0,494 | 0,483 |
| 21 | PC ae C40:0.K1 | high | 0,183 | 2,554 |
| 22 | Suc.EM | high | 0,243 | 2,836 |
| 23 | Val. K2 | high | 0,352 | 7,854 |
| 24 | Xle.K2 | high | 0,380 | 7,449 |
| 25 | Asp.EM | high | 0,386 | 3,177 |
| 26 | Trp-PTC.K1 | high | 0,434 | 5,820 |
| 27 | Pro. K2 | high | 0,437 | 6,850 |
| 28 | Arg-PTC.K1 | high | 0,439 | 7,395 |
| 29 | Val-PTC.K1 | high | 0,449 | 7,811 |
| 30 | Gln.K2 | high | 0,450 | 8,430 |

Beside the above straight forward approaches we use two algorithms which are known to work well for identifying housekeeping genes (reference genes) in case of real-time quantitative RT-PCR data. First, we apply the method of Vandesompele et al. [Vandesompele et al. (2002). Accurate normalization of real-time quantitative RT-PCR data by geometric averaging of multiple internal control genes. Genome Biology, 3(7):research0034.1-0034.11.] which is called geNorm. That is, we rank the analytes by the stability measure M introduced by Vandesompele et al. and in each step remove the analyte with the largest M value, i.e. the lowest stability. As the geNorm procedure is based on analyte ratios, the two most stable analytes cannot be ranked. geNorm ranks the analytes according to the similarity of the concentration profiles. Hence, the results are quite distinct from the results of the other selection methods (cf. Table 11) and indicate that there are three groups of analytes which have very similar concentration profiles and hence dominate the selection process; confer Table 9 where the 20 top ranked analytes are depicted.

**Table 9: 20 top ranked analytes using geNorm.**

| **Nr** | **Analyte** | **Rank** | **mean M** |
|---|---|---|---|
| 1 | SM C16:0.K1 | 1 | 0,121 |
| 2 | PC aa C38:1.K1 | 1 | 0,121 |
| 3 | SM (OH) C22:2.K1 | 3 | 0,138 |
| 4 | SM (OH) C14:1.K1 | 4 | 0,147 |
| 5 | SM (OH) C16:1.K1 | 5 | 0,154 |
| 6 | PC aa C28:1.K1 | 6 | 0,162 |
| 7 | PC ae C38:1.K1 | 7 | 0,168 |
| 8 | PC ae C40:1.K1 | 8 | 0,176 |
| 9 | PC ae C40:2.K1 | 9 | 0,183 |
| 10 | PC ae C38:2.K1 | 10 | 0,188 |
| 11 | PC aa C30:0.K1 | 11 | 0,194 |
| 12 | PC ae C30:1.K1 | 12 | 0,199 |
| 13 | SM C18:0.K1 | 13 | 0,206 |
| 14 | PC aa C38:0.K1 | 14 | 0,211 |
| 15 | PC aa C32:2.K1 | 15 | 0,216 |
| 16 | PC aa C36:0.K1 | 16 | 0,222 |
| 17 | PC ae C38:6.K1 | 17 | 0,227 |
| 18 | PC ae C34:0.K1 | 18 | 0,231 |
| 19 | PC ae C36:1.K1 | 19 | 0,235 |
| 20 | PC aa C36:2.K1 | 20 | 0,238 |

Finally, we use the method introduced by Andersen et al. (2004) [Andersen et al. (2004). Normalization of Real-Time Quantitative Reverse Transcription-PCR Data: A Model-Based Variance Estimation Approach to Identify Genes Suited for Normalization, Applied to Bladder and Colon Cancer Data Sets. Cancer Research 64:5245-5250.] which is called Norm Finder. That is, we rank the analytes by the stability measure rho introduced by Andersen et al. where in each step the analyte with the smallest rho value, i.e. the highest stability, given the previously selected analytes is added. The NormFinder procedure takes the inter and intra group variability of the analyte concentrations into account where we consider the groups LPS (LPS treated animals) and control at time points 0h, 2h, 6h, 24h, 48h, 72h, 96h, 120h, 144h, 168h, 192h, 216h, 240h and obtain 26 different groups. Table 10 shows the 50 top ranked analytes.

**Table 10: 50 top ranked analytes using NormFinder.**

| **Nr** | **Analyte** | **Rank** | **rho** |
|---|---|---|---|
| 1 | PC aa C38:0.K1 | 1 | 0,0817 |
| 2 | PC ae C30:0.K1 | 2 | 0,0610 |
| 3 | Pro.K2 | 3 | 0,0511 |
| 4 | PC aa C40:1.K1 | 4 | 0,0456 |
| 5 | SM C16:1.K1 | 5 | 0,0418 |
| 6 | PC aa C42:1.K1 | 6 | 0,0388 |
| 7 | SM C18:0.K1 | 7 | 0,0365 |
| 8 | C14.K1 | 8 | 0,0343 |
| 9 | Xle.K2 | 9 | 0,0326 |
| 10 | PC ae C30:1.K1 | 10 | 0,0312 |
| 11 | PC aa C30:2.K1 | 11 | 0,0300 |
| 12 | Trp-PTC.K1 | 12 | 0,0290 |
| 13 | PC ae C40:1.K1 | 13 | 0,0280 |
| 14 | Pro-PTC.K1 | 14 | 0,0271 |
| 15 | Val.K2 | 15 | 0,0263 |
| 16 | PC aa C36:2.K1 | 16 | 0,0256 |
| 17 | C18:1.K1 | 17 | 0,0250 |
| 18 | PC aa C42:2.K1 | 18 | 0,0245 |
| 19 | C10:2.K1 | 19 | 0,0240 |
| 20 | Cit.K2 | 20 | 0,0235 |
| 21 | PC ae C40:0.K1 | 21 | 0,0230 |
| 22 | PC aa C40:4.K1 | 22 | 0,0226 |
| 23 | C16:1.K1 | 23 | 0,0221 |
| 24 | PC aa C32:2.K1 | 24 | 0,0218 |
| 25 | PC ae C44:6.K1 | 25 | 0,0214 |
| 26 | SM C18:1.K1 | 26 | 0,0210 |
| 27 | PC aa C42:0.K1 | 27 | 0,0207 |
| 28 | PC aa C32:3.K1 | 28 | 0,0204 |
| 29 | ADMA.K2 | 29 | 0,0202 |
| 30 | Val-PTC.K1 | 30 | 0,0199 |
| 31 | C14:1.K1 | 31 | 0,0197 |
| 32 | PC aa C34:4.K1 | 32 | 0,0194 |
| 33 | Gln.K2 | 33 | 0,0192 |
| 34 | PC ae C42:3.K1 | 34 | 0,0189 |
| 35 | C16:1-OH.K1 | 35 | 0,0187 |
| 36 | PC aa C36:0.K1 | 36 | 0,0185 |
| 37 | PC ae C36:2.K1 | 37 | 0,0183 |
| 38 | C5-M-DC.K1 | 38 | 0,0181 |
| 39 | PC aa C38:6.K1 | 39 | 0,0180 |
| 40 | Arg-PTC.K1 | 40 | 0,0177 |
| 41 | total DMA.K2 | 41 | 0,0176 |
| 42 | Leu.K2 | 42 | 0,0174 |
| 43 | PC aa C36:4.K1 | 43 | 0,0173 |
| 44 | C6 (C4:1-DC).K1 | 44 | 0,0171 |
| 45 | C12.K1 | 45 | 0,0169 |
| 46 | PC ae C38:6.K1 | 46 | 0,0168 |
| 47 | C5-DC (C6-OH).K1 | 47 | 0,0167 |
| 48 | Trp.K2 | 48 | 0,0165 |
| 49 | PC ae C34:3.K1 | 49 | 0,0164 |
| 50 | Phe-PTC.K1 | 50 | 0,0163 |

Table 11 shows a summary of all analytes which were chosen by at least one of the four different methods where the selection is indicated by TRUE. There are several analytes which were chosen by two or even three methods simultaneously.

**Table 11: Summary of the selected analytes. TRUE indicates that an analyte was chosen by the corresponding algorithm.**

| **Nr** | **Analyte** | **CV of raw data** | **SD of log data** | **geNorm** | **NormFinder** |
|---|---|---|---|---|---|
| 1 | ADMA.K2 | | | | TRUE |
| 2 | Arg-PTC.K1 | | TRUE | | TRUE |
| 3 | Asp. EM | | TRUE | | |
| 4 | C10:2.K1 | | | | TRUE |
| 5 | C12-DC.K1 | TRUE | TRUE | | |
| 6 | C12.K1 | TRUE | TRUE | | TRUE |
| 7 | C14:1.K1 | | | | TRUE |
| 8 | C14.K1 | TRUE | | | TRUE |
| 9 | C16:1.K1 | TRUE | TRUE | | TRUE |
| 10 | C16:1-OH.K1 | | | | TRUE |
| 11 | C16:2-OH.K1 | TRUE | | | |
| 12 | C18:1.K1 | | | | TRUE |
| 13 | C18:1-OH.K1 | TRUE | | | |
| 14 | C5-DC (C6-OH).K1 | | | | TRUE |
| 15 | C5-M-DC.K1 | TRUE | TRUE | | TRUE |
| 16 | C6 (C4:1-DC).K1 | TRUE | TRUE | | TRUE |
| 17 | C8.K1 | TRUE | TRUE | | |
| 18 | Cit.K2 | | | | TRUE |
| 19 | Gln.K2 | | TRUE | | TRUE |
| 20 | Leu.K2 | | | | TRUE |
| 21 | lysoPC a C26:0.K1 | TRUE | TRUE | | |
| 22 | lysoPC a C26:1.K1 | TRUE | TRUE | | |
| 23 | lysoPC a C28:0.K1 | TRUE | | | |
| 24 | lysoPC a C28:1.K1 | TRUE | | | |
| 25 | PC aa C26:0.K1 | TRUE | TRUE | | |
| 26 | PC aa C28:1.K1 | | | TRUE | |
| 27 | PC aa C30:0.K1 | | TRUE | TRUE | |
| 28 | PC aa C30:2.K1 | | TRUE | | TRUE |
| 29 | PC aa C32:2.K1 | | TRUE | TRUE | TRUE |
| 30 | PC aa C32:3.K1 | | | | TRUE |
| 31 | PC aa C34:4.K1 | | | | TRUE |
| 32 | PC aa C36:0.K1 | | TRUE | TRUE | TRUE |
| 33 | PC aa C36:2.K1 | | | TRUE | TRUE |
| 34 | PC aa C36:4.K1 | | | | TRUE |
| 35 | PC aa C38:0.K1 | | TRUE | TRUE | TRUE |
| 36 | PC aa C38:1.K1 | | TRUE | TRUE | |
| 37 | PC aa C38:6.K1 | | | | TRUE |
| 38 | PC aa C40:1.K1 | TRUE | TRUE | | TRUE |
| 39 | PC aa C40:4.K1 | | | | TRUE |
| 40 | PC aa C42:0.K1 | TRUE | | | TRUE |
| 41 | PC aa C42:1.K1 | | | | TRUE |
| 42 | PC aa C42:2.K1 | | | | TRUE |
| 43 | PC ae C30:0.K1 | | | | TRUE |
| 44 | PC ae C30:1.K1 | | | TRUE | TRUE |
| 45 | PC ae C34:0.K1 | | | TRUE | |
| 46 | PC ae C34:3.K1 | | | | TRUE |
| 47 | PC ae C36:1.K1 | | | TRUE | |
| 48 | PC ae C36:2.K1 | | | | TRUE |
| 49 | PC ae C38:1.K1 | | TRUE | TRUE | |
| 50 | PC ae C38:2.K1 | | | TRUE | |
| 51 | PC ae C38:6.K1 | | | TRUE | TRUE |
| 52 | PC ae C40:0.K1 | TRUE | TRUE | | TRUE |
| 53 | PC ae C40:1.K1 | | | TRUE | TRUE |
| 54 | PC ae C40:2.K1 | | | TRUE | |
| 55 | PC ae C42:3.K1 | | | | TRUE |
| 56 | PC ae C44:6.K1 | TRUE | TRUE | | TRUE |
| 57 | Phe-PTC.K1 | | | | TRUE |
| 58 | Pro.K2 | | TRUE | | TRUE |
| 59 | Pro-PTC.K1 | | | | TRUE |
| 60 | SM C16:0.K1 | | | TRUE | |
| 61 | SM C16:1.K1 | | TRUE | | TRUE |
| 62 | SM C18:0.K1 | | | TRUE | TRUE |
| 63 | SM C18:1.K1 | | TRUE | | TRUE |
| 64 | SM (OH) C14:1.K1 | | | TRUE | |
| 65 | SM (OH) C16:1.K1 | | | TRUE | |
| 66 | SM (OH) C22:2.K1 | | | TRUE | |
| 67 | Suc.EM | TRUE | TRUE | | |
| 68 | total DMA.K2 | | | | TRUE |
| 69 | Trp.K2 | | | | TRUE |
| 70 | Trp-PTC.K1 | | TRUE | | TRUE |
| 71 | Val. K2 | TRUE | TRUE | | TRUE |
| 72 | Val-PTC.K1 | | TRUE | | TRUE |
| 73 | Xle.K2 | | TRUE | | TRUE |

### Statistical Analysis

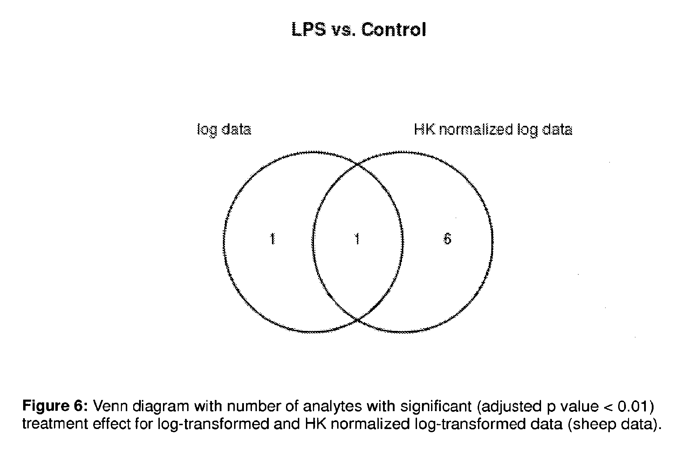

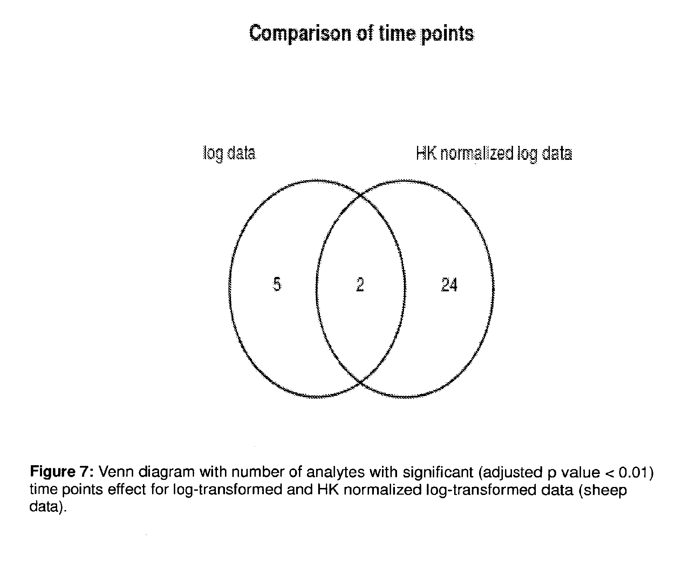

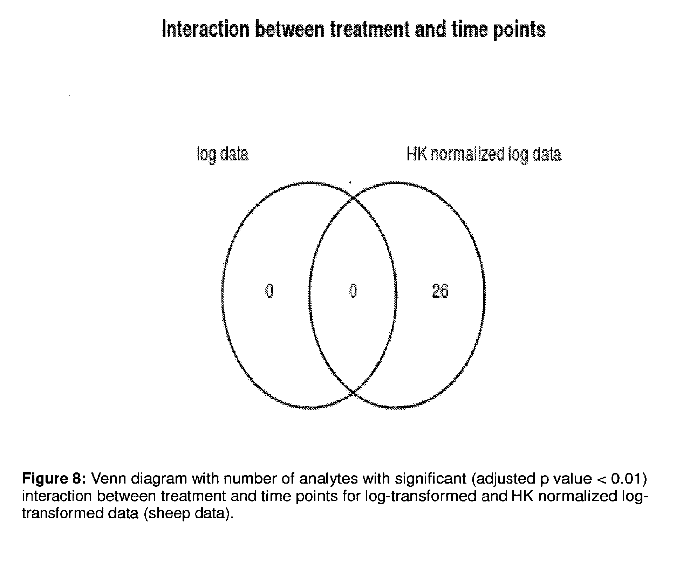

We use a linear model with an autoregressive and heterogeneous variance structure to analyze the data. The obtained p values are corrected by the method of Benjamini and Hochberg (1995) [Benjamini Y., and Hochberg Y. (1995). Controlling the false discovery rate: a practical and powerful approach to multiple testing. Journal of the Royal Statistical Society Series B 57:289-300]. As housekeeping analytes we use SM C16:0.K1, PC aa C38:1.K1 and SM (OH) C22:2.K1 which were top ranked by the geNorm algorithm. We compare the results for log-transformed and housekeeper (HK) normalized log-transformed data. The normalization by means of housekeeping analytes is performed by subtracting the mean of log-transformed concentrations of the three selected housekeepers from the log-transformed concentrations of the other analytes; i.e., in terms of the raw concentration this means that we use the logarithm of the ratio between the raw concentration of all analytes (except the housekeepers) and the geometric mean of the housekeepers for the statistical analysis. The Venn diagrams in Figures 6-8 are based on all metabolites having adjusted p values smaller than 0.01. In all cases more significant differences are detected in case of the HK normalized log-transformed data indicating that the use of the HK normalized log-transformed data leads to an increased power of the statistical analysis.

The assumption of an increased power is confirmed by area under curve (AUC) computations. For the comparison LPS vs. Control we compute the AUC, which is a measure for the predictive power, for each of the analytes. From the 193 analytes included in this analysis 127 have a larger AUC in case of the HK normalized log-transformed data. The corresponding proportion 65.8% (p < 0.001) is significantly different (i.e., larger) from 50%. In addition, the maximum AUC is increased from 0.798 (log data) to 0.892 (HK). Hence, this again speaks for an increased power of the statistical analysis by using the HK normalized log-transformed data instead of the log-transformed data.

### 3. Rat - brain data

Animal model HI brain injury: A model of HI brain injury based on Rice-Vanucci's procedure was performed at postnatal day (P7). Sprague-Dawley rat pups (*from Charles River, Wilmington, MA, U. S.A.* of either sex were anesthetized with inhaled isoflurane (3% for induction of ansthesia, 1,5% for maintenance), the right carotid artery was accessed through a midline incision and surgical ligation was performed with a double suture and a permanent incision. The procedure was performed at room temperature (23-25°C). After closure of the neck wound, pups were returned to their dams for 2 h. The entire surgical procedure lasted no longer than 10 min. The pups were then exposed to hypoxia at 8% oxygen for 100 minutes. Sham-operated animals underwent the same anaesthesia protocol and neck incision and vessel manipulation without ligation or hypoxia. Control animals were kept without any damage. Animals were euthanized i) immediately after hypoxia (P7), ii) after 24hrs (P8), iii) after 5 days (P12), the right hemisphere of the brains were collected and stored at -80°C until further preparation. All animals were randomized to intervention and time point.

### Selection of Housekeeping Analytes

We use data of 150 rats where data are obtained for groups Op, sham and control at time points P7, P8 and P12 after treatment and it is distinguished between male and females as well as between left and right hemisphere.

In a first step, we use the raw data and sort the analytes by the coefficient of variation (CV) which is defined as the ratio of the standard deviation (SD) to the mean; i.e., CV = SD/mean. Table 12 shows the top 20 analytes with smallest CV. In addition we give SD and mean of the raw concentrations.

**Table 12: Top 20 analytes with smallest CV (based on raw data).**

| **Nr** | **Analyte** | **CV** | **SD** | **mean** |
|---|---|---|---|---|
| 1 | PC aa C26:0.K1 | 0,086 | 0,179 | 2,071 |
| 2 | lysoPC a C26:1.K1 | 0,097 | 0,816 | 8,395 |
| 3 | lysoPC a C14:0.K1 | 0,124 | 1,434 | 11,567 |
| 4 | C10.K1 | 0,155 | 0,038 | 0,244 |
| 5 | C8:1.K1 | 0,163 | 0,040 | 0,243 |
| 6 | C0.K1 | 0,167 | 11,601 | 69,387 |
| 7 | C8.K1 | 0,194 | 0,045 | 0,230 |
| 8 | C7-DC.K1 | 0,196 | 0,015 | 0,078 |
| 9 | C14-OH.K1 | 0,199 | 0,012 | 0,058 |
| 10 | C16-OH.K1 | 0,202 | 0,014 | 0,071 |
| 11 | C16:1.K1 | 0,214 | 0,016 | 0,076 |
| 12 | C5-DC(C6-OH).K1 | 0,215 | 0,026 | 0,121 |
| 13 | Gln-PTC.K1 | 0,241 | 828,949 | 3446,608 |
| 14 | C4-OH (C3-DC).K1 | 0,249 | 0,137 | 0,553 |
| 15 | Gln.K2 | 0,250 | 780,395 | 3117,797 |
| 16 | PC ae C42:5.K1 | 0,258 | 0,291 | 1,128 |
| 17 | C18:1-OH.K1 | 0,259 | 0,012 | 0,045 |
| 18 | C16:2-OH.K1 | 0,263 | 0,016 | 0,061 |
| 19 | C16:1-OH.K1 | 0,266 | 0,012 | 0,044 |
| 20 | His-PTC.K1 | 0,275 | 48,090 | 175,152 |

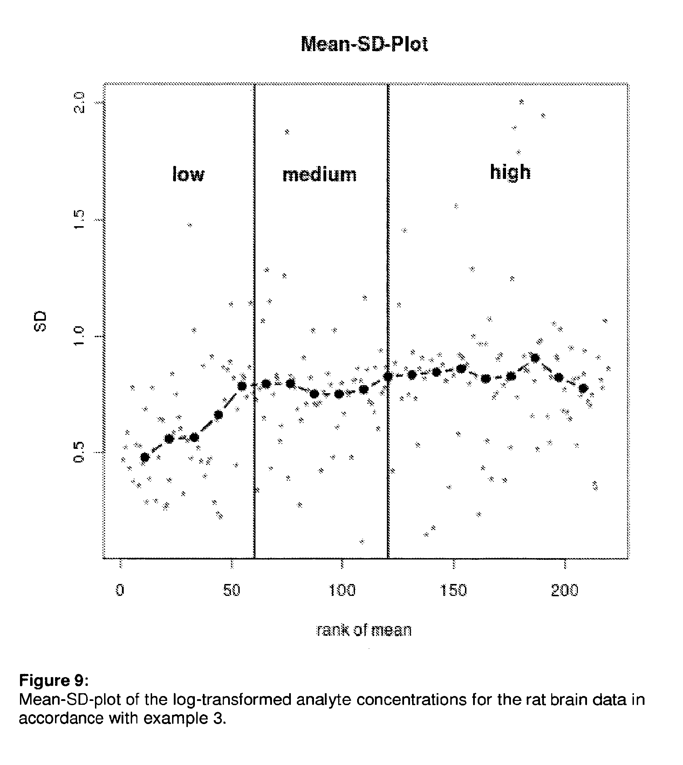

As one often uses the log-transformed analyte concentrations for further analysis, where the log-transformation is used to stabilize variance and to obtain at least approximately normal distributed data, we in a second step compute mean and SD for the log-transformed data. Of course, one could also use other transformations like for instance Box-Cox power transformations [Box, G. E. P. and Cox, D. R. (1964) An analysis of transformations (with discussion). Journal of the Royal Statistical Society B, 26, 211-252.], the generalized log-transformation via vsn [Huber W., von Heydebreck A., Sueltmann H., Poustka A., Vingron M. (2002). Variance stabilization applied to microarray data calibration and to the quantification of differential expression. Bioinformatics 18 Suppl.1 S96-S104.] or vst [Lin S.M., Du P., Huber W., Kibbe, W.A. (2008). Model-based variance-stabilizing transformation for Illumina microarray data. Nucleic Acids Research, Vol. 36, No. 2 e11.] or some other normalization procedure which is well-known from microarray normalization in this step. A comparative survey of normalization procedures in case of Affymetrix microarray data is given in Cope et al. (2004) [L.M. Cope et al. (2004). A Benchmark for Affymetrix GeneChip Expression Measures, Bioinformatics 20(3):323-331] or Irizarry et al. (2006) [R.A. Irizarry et al. (2006). Comparison of Affymetrix GeneChip Expression Measures, Bioinformatics 22(7):789-794]. Figure 9 shows that the variance stabilization works well but not perfectly. Consequently, lower mean log-concentrations tend to have smaller SDs. Since we are interested in the analytes with the smallest SDs, we split the log-concentration in three parts (low, medium, high) to avoid getting only analytes with low concentrations. Analytes are classified as low concentrated if their mean concentration is smaller than 0.5 (i.e., mean log2-concentration < -1), as medium concentrated if their mean concentration is between 0.5 and 4 (i.e., mean log2-concentration between -1 and 2), and as high concentrated if their mean concentration is larger than 4 (i.e., mean log2-concentration > 2). Of course, the choice of the groups is rather arbitrary and one could use other cut-off values and more groups, respectively. Choosing the 10 top ranked analytes, i.e., with lowest SD values, for low, medium and high concentrated analytes, respectively, we obtain the analytes depicted in Table 13.

**Table 13: 10 top ranked analytes for each case; i.e., with smallest SD for low, medium and high concentrated analytes, respectively (based on log-transformed data).**

| **Nr** | **Analyte** | **group** | **SD** | **mean** |
|---|---|---|---|---|
| 1 | C10.K1 | low | 0,222 | -2,053 |
| 2 | C8:1.K1 | low | 0,243 | -2,063 |
| 3 | C16:1.K1 | low | 0,262 | -3,752 |
| 4 | C7-DC.K1 | low | 0,276 | -3,714 |
| 5 | C14-OH.K1 | low | 0,285 | -4,134 |
| 6 | C8.K1 | low | 0,287 | -2,149 |
| 7 | C16-OH.K1 | low | 0,293 | -3,849 |
| 8 | C5-DC(C6-OH).K1 | low | 0,322 | -3,086 |
| 9 | C18:1-OH.K1 | low | 0,360 | -4,522 |
| 10 | C16:1-OH.K1 | low | 0,377 | -4,550 |
| 11 | PC aa C26:0.K1 | medium | 0,119 | 1,045 |
| 12 | PC ae C42:5.K1 | medium | 0,275 | 0,142 |
| 13 | C4-OH (C3-DC).K1 | medium | 0,339 | -0,895 |
| 14 | PC aa C40:1.K1 | medium | 0,390 | -0,100 |
| 15 | lysoPC a C20:3.K1 | medium | 0,424 | 0,434 |
| 16 | lysoPC a C24:0.K1 | medium | 0,434 | -0,511 |
| 17 | PC aa C42:6.K1 | medium | 0,480 | 0,588 |
| 18 | PC ae C42:0.K1 | medium | 0,484 | 0,823 |
| 19 | C5.K1 | medium | 0,550 | -0,268 |
| 20 | Kyn.344.0...146.1.K2 | medium | 0,604 | 1,565 |
| 21 | lysoPC a C26:1.K1 | high | 0,144 | 3,063 |
| 22 | lysoPC a C14:0.K1 | high | 0,175 | 3,521 |
| 23 | C0.K1 | high | 0,237 | 6,097 |
| 24 | Gln-PTC.K1 | high | 0,348 | 11,709 |
| 25 | PC ae C40:0.K1 | high | 0,352 | 4,838 |
| 26 | Gln.K2 | high | 0,369 | 11,560 |
| 27 | His-PTC.K1 | high | 0,382 | 7,401 |
| 28 | Creatinine.K1 | high | 0,389 | 6,645 |
| 29 | Putrescine.K2 | high | 0,434 | 6,123 |
| 30 | Arg-PTC.K1 | high | 0,514 | 8,884 |

Beside the above straight forward approaches we use two algorithms which are known to work well for identifying housekeeping genes (reference genes) in case of real-time quantitative RT-PCR data. First, we apply the method of Vandesompele et al. [Vandesompele et al. (2002). Accurate normalization of real-time quantitative RT-PCR data by geometric averaging of multiple internal control genes. Genome Biology, 3(7):research0034.1-0034.11.] which is called geNorm. That is, we rank the analytes by the stability measure M introduced by Vandesompele et al. and in each step remove the analyte with the largest M value, i.e. the lowest stability. As the geNorm procedure is based on analyte ratios, the two most stable analytes cannot be ranked. geNorm ranks the analytes according to the similarity of the concentration profiles. Hence, the results are quite distinct from the results of the other selection methods (cf. Table 16) and indicate that there is one group of analytes where the analytes have very similar concentration profiles and hence dominate the selection process; confer
Table 14 where the 20 top ranked analytes are depicted.

**Table 14: 20 top ranked analytes using geNorm.**

| **Nr** | **Analyte** | **Rank** | **mean M** |
|---|---|---|---|
| 1 | PC aa C36:4.K1 | 1 | 0,095 |
| 2 | PC aa C38:5.K1 | 1 | 0,095 |
| 3 | PC aa C38:3.K1 | 3 | 0,108 |
| 4 | PC aa C36:3.K1 | 4 | 0,126 |
| 5 | PC aa C40:5.K1 | 5 | 0,129 |
| 6 | PC aa C38:6.K1 | 6 | 0,133 |
| 7 | PC aa C40:4.K1 | 7 | 0,137 |
| 8 | PC aa C40:6.K1 | 8 | 0,141 |
| 9 | PC aa C38:4.K1 | 9 | 0,145 |
| 10 | PC ae C38:5.K1 | 10 | 0,153 |
| 11 | PC aa C36:2.K1 | 11 | 0,159 |
| 12 | PC ae C38:6.K1 | 12 | 0,164 |
| 13 | PC ae C36:4.K1 | 13 | 0,169 |
| 14 | PC aa C36:1.K1 | 14 | 0,174 |
| 15 | PC aa C36:0.K1 | 15 | 0,179 |
| 16 | PC aa C42:4.K1 | 16 | 0,184 |
| 17 | PC aa C42:5.K1 | 17 | 0,188 |
| 18 | PC aa C34:1.K1 | 18 | 0,192 |
| 19 | PC ae C42:1.K1 | 19 | 0,196 |
| 20 | PC ae C38:4.K1 | 20 | 0,200 |

Finally, we use the method introduced by Andersen et al. (2004) [Andersen et al. (2004). Normalization of Real-Time Quantitative Reverse Transcription-PCR Data: A Model-Based Variance Estimation Approach to Identify Genes Suited for Normalization, Applied to Bladder and Colon Cancer Data Sets. Cancer Research 64:5245-5250.] which is called NormFinder. That is, we rank the analytes by the stability measure rho introduced by Andersen et al. where in each step the analyte with the smallest rho value, i.e. the highest stability, given the previously selected analytes is added. The Norm Finder procedure takes the inter and intra group variability of the analyte concentrations into account where we consider the groups Op, sham and control at time points P7, P8 and P12 after treatment and distinguish between males and females as well as between left and right hemisphere. Overall, this leads to 31 different groups. Table 15 shows the 50 top ranked analytes.

**Table 15: 50 top ranked analytes using NormFinder.**

| **Nr** | **Analyte** | **Rank** | **rho** |
|---|---|---|---|
| 1 | PC ae C40:0.K1 | 1 | 0,2269 |
| 2 | Gln-PTC.K1 | 2 | 0,1883 |
| 3 | SM C26:0.K1 | 3 | 0,1274 |
| 4 | PC aa C30:0.K1 | 4 | 0,1120 |
| 5 | C7-DC.K1 | 5 | 0,1010 |
| 6 | PC ae C36:0.K1 | 6 | 0,0954 |
| 7 | lysoPC a C14:0.K1 | 7 | 0,0921 |
| 8 | PC aa C36:6.K1 | 8 | 0,0861 |
| 9 | SM (OH) C22:1.K1 | 9 | 0,0873 |
| 10 | Creatinine.K2 | 10 | 0,0819 |
| 11 | C5-DC(C6-OH).K1 | 11 | 0,0711 |
| 12 | PC aa C42:6.K1 | 12 | 0,0690 |
| 13 | His-PTC.K1 | 13 | 0,0691 |
| 14 | SM (OH) C24:1.K1 | 14 | 0,0635 |
| 15 | PC ae C42:4.K1 | 15 | 0,0620 |
| 16 | C16:2.K1 | 16 | 0,0622 |
| 17 | Gln.K2 | 17 | 0,0598 |
| 18 | PC aa C32:0.K1 | 18 | 0,0569 |
| 19 | C8.K1 | 19 | 0,0565 |
| 20 | lysoPC a C16:0.K1 | 20 | 0,0542 |
| 21 | PG D2. PG | 21 | 0,0529 |
| 22 | PC aa C28:1.K1 | 22 | 0,0511 |
| 23 | PC aa C32:1.K1 | 23 | 0,0517 |
| 24 | Glu.K2 | 24 | 0,0510 |
| 25 | PC ae C40:4.K1 | 25 | 0,0498 |
| 26 | C16:1-OH.K1 | 26 | 0,0492 |
| 27 | PC ae C42:5.K1 | 27 | 0,0496 |
| 28 | SM C16:0.K1 | 28 | 0,0492 |
| 29 | 6-keto-PGF1a.PG | 29 | 0,0470 |
| 30 | SM C20:2.K1 | 30 | 0,0474 |
| 31 | PC ae C40:2.K1 | 31 | 0,0460 |
| 32 | C0.K1 | 32 | 0,0456 |
| 33 | PC ae C38:0.K1 | 33 | 0,0460 |
| 34 | C3:1.K1 | 34 | 0,0461 |
| 35 | lysoPC a C18:0.K1 | 35 | 0,0436 |
| 36 | C4-OH (C3-DC).K1 | 36 | 0,0429 |
| 37 | C5-OH (C3-DC-M).K1 | 37 | 0,0434 |
| 38 | C5:1-DC.K1 | 38 | 0,0421 |
| 39 | PC ae C40:5.K1 | 39 | 0,0407 |
| 40 | PC ae C44:5.K1 | 40 | 0,0404 |
| 41 | PC aa C42:0.K1 | 41 | 0,0407 |
| 42 | C14:1.K1 | 42 | 0,0409 |
| 43 | Arg-PTC.K1 | 43 | 0,0396 |
| 44 | PC aa C34:2.K1 | 44 | 0,0389 |
| 45 | C16-OH.K1 | 45 | 0,0397 |
| 46 | PC ae C34:1.K1 | 46 | 0,0397 |
| 47 | 15S-HETE.PG | 47 | 0,0394 |
| 48 | C12:1.K1 | 48 | 0,0385 |
| 49 | SM (OH) C22:2.K1 | 49 | 0,0385 |
| 50 | PC aa C34:3.K1 | 50 | 0,0384 |

Table 16 shows a summary of all analytes which were chosen by at least one of the four different methods where the selection is indicated by TRUE. There are several analytes which were chosen by two or even three methods simultaneously.

**Table 16: Summary of the selected analytes. TRUE indicates that an analyte was chosen by the corresponding algorithm.**

| **Nr** | **Analyte** | **CV of raw data** | **SD of log data** | **geNorm** | **NormFinder** |
|---|---|---|---|---|---|
| 1 | 15S-HETE.PG | | | | TRUE |
| 2 | 6-keto-PGF1a.PG | | | | TRUE |
| 3 | Arg-PTC.K1 | | TRUE | | TRUE |
| 4 | C0.K1 | TRUE | TRUE | | TRUE |
| 5 | C10.K1 | TRUE | TRUE | | |
| 6 | C12:1.K1 | | | | TRUE |
| 7 | C14:1.K1 | | | | TRUE |
| 8 | C14-OH.K1 | TRUE | TRUE | | |
| 9 | C16:1.K1 | TRUE | TRUE | | |
| 10 | C16:1-OH.K1 | TRUE | TRUE | | TRUE |
| 11 | C16:2. K1 | | | | TRUE |
| 12 | C16:2-OH.K1 | TRUE | | | |
| 13 | C16-OH.K1 | TRUE | TRUE | | TRUE |
| 14 | C18:1-OH.K1 | TRUE | TRUE | | |
| 15 | C3:1.K1 | | | | TRUE |
| 16 | C4-OH (C3-DC).K1 | TRUE | TRUE | | TRUE |
| 17 | C5:1-DC.K1 | | | | TRUE |
| 18 | C5-DC(C6-OH).K1 | TRUE | TRUE | | TRUE |
| 19 | C5.K1 | | TRUE | | |
| 20 | C5-OH (C3-DC-M).K1 | | | | TRUE |
| 21 | C7-DC.K1 | TRUE | TRUE | | TRUE |
| 22 | C8:1.K1 | TRUE | TRUE | | |
| 23 | C8.K1 | TRUE | TRUE | | TRUE |
| 24 | Creatinine.K1 | | TRUE | | |
| 25 | Creatinine.K2 | | | | TRUE |
| 26 | Gln.K2 | TRUE | TRUE | | TRUE |
| 27 | Gln-PTC.K1 | TRUE | TRUE | | TRUE |
| 28 | Glu.K2 | | | | TRUE |
| 29 | His-PTC.K1 | TRUE | TRUE | | TRUE |
| 30 | Kyn.K2 | | TRUE | | |
| 31 | lysoPC a C14:0.K1 | TRUE | TRUE | | TRUE |
| 32 | lysoPC a C16:0.K1 | | | | TRUE |
| 33 | lysoPC a C18:0.K1 | | | | TRUE |
| 34 | lysoPC a C20:3.K1 | | TRUE | | |
| 35 | lysoPC a C24:0.K1 | | TRUE | | |
| 36 | lysoPC a C26:1.K1 | TRUE | TRUE | | |
| 37 | PC aa C26:0.K1 | TRUE | TRUE | | |
| 38 | PC aa C28:1.K1 | | | | TRUE |
| 39 | PC aa C30:0.K1 | | | | TRUE |
| 40 | PC aa C32:0.K1 | | | | TRUE |
| 41 | PC aa C32:1.K1 | | | | TRUE |
| 42 | PC aa C34:1.K1 | | | TRUE | |
| 43 | PC aa C34:2.K1 | | | | TRUE |
| 44 | PC aa C34:3.K1 | | | | TRUE |
| 45 | PC aa C36:0.K1 | | | TRUE | |
| 46 | PC aa C36:1.K1 | | | TRUE | |
| 47 | PC aa C36:2.K1 | | | TRUE | |
| 48 | PC aa C36:3.K1 | | | TRUE | |
| 49 | PC aa C36:4.K1 | | | TRUE | |
| 50 | PC aa C36:6.K1 | | | | TRUE |
| 51 | PC aa C38:3.K1 | | | TRUE | |
| 52 | PC aa C38:4.K1 | | | TRUE | |
| 53 | PC aa C38:5.K1 | | | TRUE | |
| 54 | PC aa C38:6.K1 | | | TRUE | |
| 55 | PC aa C40:1.K1 | | TRUE | | |
| 56 | PC aa C40:4.K1 | | | TRUE | |
| 57 | PC aa C40:5.K1 | | | TRUE | |
| 58 | PC aa C40:6.K1 | | | TRUE | |
| 59 | PC aa C42:0.K1 | | | | TRUE |
| 60 | PC aa C42:4.K1 | | | TRUE | |
| 61 | PC aa C42:5.K1 | | | TRUE | |
| 62 | PC aa C42:6.K1 | | TRUE | | TRUE |
| 63 | PC ae C34:1.K1 | | | | TRUE |
| 64 | PC ae C36:0.K1 | | | | TRUE |
| 65 | PC ae C36:4.K1 | | | TRUE | |
| 66 | PC ae C38:0.K1 | | | | TRUE |
| 67 | PC ae C38:4.K1 | | | TRUE | |
| 68 | PC ae C38:5.K1 | | | TRUE | |
| 69 | PC ae C38:6.K1 | | | TRUE | |
| 70 | PC ae C40:0.K1 | | TRUE | | TRUE |
| 71 | PC ae C40:2.K1 | | | | TRUE |
| 72 | PC ae C40:4.K1 | | | | TRUE |
| 73 | PC ae C40:5.K1 | | | | TRUE |
| 74 | PC ae C42:0.K1 | | TRUE | | |
| 75 | PC ae C42:1.K1 | | | TRUE | |
| 76 | PC ae C42:4.K1 | | | | TRUE |
| 77 | PC ae C42:5.K1 | TRUE | TRUE | | TRUE |
| 78 | PC ae C44:5.K1 | | | | TRUE |
| 79 | PGD2.PG | | | | TRUE |
| 80 | Putrescine.K2 | | TRUE | | |
| 81 | SM C16:0.K1 | | | | TRUE |
| 82 | SM C20:2.K1 | | | | TRUE |
| 83 | SM C26:0.K1 | | | | TRUE |
| 84 | SM (OH) C22:1.K1 | | | | TRUE |
| 85 | SM (OH) C22:2.K1 | | | | TRUE |
| 86 | SM (OH) C24:1.K1 | | | | TRUE |

### Statistical Analysis

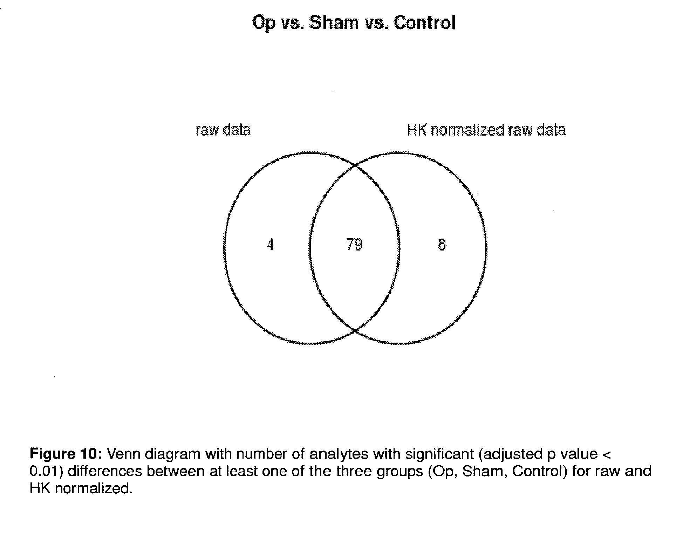

We analyze only a subset of the data. We choose time point P7 and the right hemisphere and investigate the influence of treatment and gender. A previous analysis showed that there is no significant gender effect. Hence, we omit gender and perform a Kruskal-Wallis test (non-parametric one-way ANOVA) using raw concentrations and HK normalized raw concentrations. The obtained p values are corrected by the method of Benjamini and Hochberg (1995) [Benjamini Y., and Hochberg Y. (1995). Controlling the false discovery rate: a practical and powerful approach to multiple testing. Journal of the Royal Statistical Society Series B 57:289-300]. As housekeeping analytes we use PC aa C26:0.K1, lysoPC a C26:1.K1, lysoPC a C14:0.K1, C10.K1 and C8:1.K1 which are the top five analytes with respect to the CV of the raw concentrations. We compare the results of raw and housekeeper (HK) normalized raw data. The normalization by means of housekeeping analytes is performed by dividing the raw concentrations of the analytes (except for the five housekeepers) by the geometric mean of five selected housekeepers. The Venn diagram in Figure 10 is based on all metabolites having adjusted p values smaller than 0.01. The results for the two approaches are in very good agreement with a slight advantage in terms of more significant differences in case of the HK normalized raw data. This indicates that the use of the HK normalized raw data leads to an increased power of the statistical analysis.
The assumption of an increased power is confirmed by area under curve (AUC) computations. For the comparison Op vs. Sham we compute the AUC, which is a measure for the predictive power, for each of the analytes. From the 206 analytes included in this analysis 153 have a larger AUC in case of the HK normalized log-transformed data. The corresponding proportion 74.3% (p < 0.001) is significantly different (i.e., larger) from 50%. The maximum AUC in both cases is maximal, i.e., identical to 1.000, where the maximum is attained for 15 analytes in case of the log-transformed data and for 16 analytes in case of the HK normalized log-transformed data. Hence, these results again speak for an increased power of the statistical analysis by using the HK normalized log-transformed data instead of the log-transformed data.

### 4. Human - plasma data

### Selection of Housekeeping Analytes

We use data of 80 subjects where data are obtained by 14 patients with pneumonia, 45 patients with mixed sepsis and 21 controls.

100 µl serum samples were analyzed for target metabolites as described in examples 1 to 3.

In a first step, we use the raw data and sort the analytes by the coefficient of variation (CV) which is defined as the ratio of the standard deviation (SD) to the mean; i.e., CV = SD/mean. Table 17 shows the top 20 analytes with smallest CV. In addition we give SD and mean of the raw concentrations.

**Table 17: Top 20 analytes with smallest CV (based on raw data).**

| **Nr** | **Analyte** | **CV** | **SD** | **mean** |
|---|---|---|---|---|
| 1 | lysoPC a C26:1.K1 | 0,113 | 0,248 | 2,200 |
| 2 | PC ae C40:0.K1 | 0,123 | 0,440 | 3,585 |
| 3 | C12-DC.K1 | 0,134 | 0,008 | 0,062 |
| 4 | PC aa C42:6.K1 | 0,208 | 0,111 | 0,535 |
| 5 | PC aa C26:0.K1 | 0,223 | 0,175 | 0,786 |
| 6 | C3:1.K1 | 0,294 | 0,002 | 0,008 |
| 7 | SM C16:0.K1 | 0,322 | 20,649 | 64,175 |
| 8 | PC ae C42:5.K1 | 0,323 | 0,346 | 1,072 |
| 9 | PC aa C42:5.K1 | 0,325 | 0,066 | 0,203 |
| 10 | PC ae C40:6.K1 | 0,329 | 1,147 | 3,490 |
| 11 | SM (OH) C24:1.K1 | 0,336 | 0,381 | 1,132 |
| 12 | SM C20:2.K1 | 0,337 | 0,181 | 0,537 |
| 13 | Val. K2 | 0,340 | 66,198 | 194,421 |
| 14 | SM C16:1.K1 | 0,357 | 3,397 | 9,529 |
| 15 | PC ae C38:4.K1 | 0,358 | 2,751 | 7,682 |
| 16 | PC ae C40:5.K1 | 0,359 | 1,697 | 4,726 |
| 17 | PC ae C42:0.K1 | 0,359 | 0,066 | 0,183 |
| 18 | Val-PTC.K1 | 0,360 | 69,777 | 193,889 |
| 19 | PC ae C36:0.K1 | 0,360 | 0,489 | 1,359 |
| 20 | SM C24:0.K1 | 0,365 | 3,030 | 8,299 |

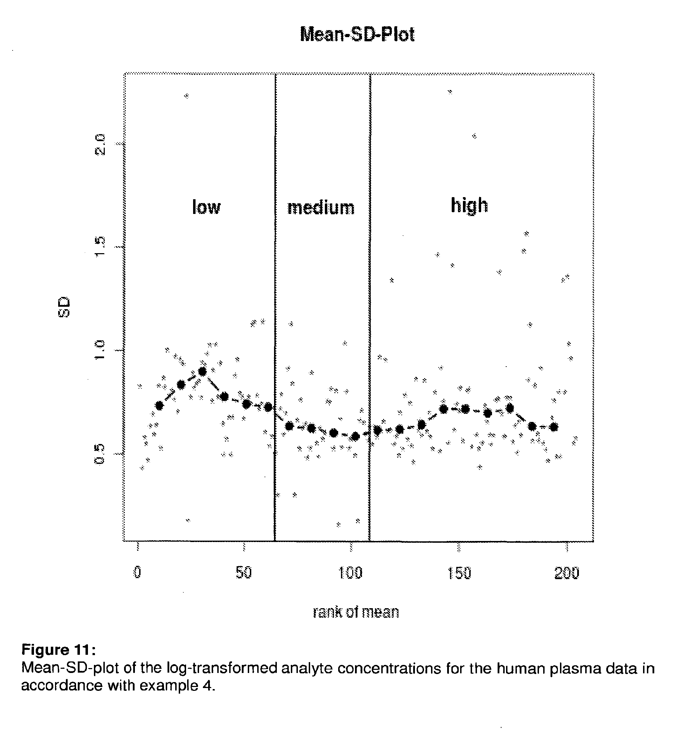

As one often uses the log-transformed analyte concentrations for further analysis, where the log-transformation is used to stabilize variance and to obtain at least approximately normal distributed data, we in a second step compute mean and SD for the log-transformed data. Of course, one could also use other transformations like for instance Box-Cox power transformations [Box, G. E. P. and Cox, D. R. (1964) An analysis of transformations (with discussion). Journal of the Royal Statistical Society B, 26, 211-252.], the generalized log-transformation via vsn [Huber W., von Heydebreck A., Sueltmann H., Poustka A., Vingron M. (2002). Variance stabilization applied to microarray data calibration and to the quantification of differential expression. Bioinformatics 18 Suppl.1 S96-S104.] or vst [Lin S.M., Du P., Huber W., Kibbe, W.A. (2008). Model-based variance-stabilizing transformation for Illumina microarray data. Nucleic Acids Research, Vol. 36, No. 2 e11.] or some other normalization procedure which is well-known from microarray normalization in this step. A comparative survey of normalization procedures in case of Affymetrix microarray data is given in Cope et al. (2004) [L.M. Cope et al. (2004). A Benchmark for Affymetrix GeneChip Expression Measures, Bioinformatics 20(3):323-331] or Irizarry et al. (2006) [R.A. Irizarry et al. (2006). Comparison of Affymetrix GeneChip Expression Measures, Bioinformatics 22(7):789-794]. Figure 11 shows that the variance stabilization works well but not perfectly. Hence, we split the log-concentration in three parts (low, medium, high) as in the previous examples. Analytes are classified as low concentrated if their mean concentration is smaller than 0.5 (i.e., mean log2-concentration < -1), as medium concentrated if their mean concentration is between 0.5 and 4 (i.e., mean log2-concentration between -1 and 2), and as high concentrated if their mean concentration is larger than 4 (i.e., mean log2-concentration > 2). Of course, the choice of the groups is rather arbitrary and one could use other cut-off values and more groups, respectively. Choosing the 10 top ranked analytes, i.e., with lowest SD values, for low, medium and high concentrated analytes, respectively, we obtain the analytes depicted in Table 18.

**Table 18: 10 top ranked analytes for each case; i.e., with smallest SD for low, medium and high concentrated analytes, respectively (based on log-transformed data).**

| **Nr** | **Analyte** | **group** | **SD** | **mean** |
|---|---|---|---|---|
| 1 | C12-DC.K1 | low | 0,173 | -4,033 |
| 2 | C3:1.K1 | low | 0,430 | -7,067 |
| 3 | C16:2-OH.K1 | low | 0,469 | -6,728 |
| 4 | PC ae C42:0.K1 | low | 0,494 | -2,538 |
| 5 | PC aa C42:5.K1 | low | 0,497 | -2,381 |
| 6 | C3-OH.K1 | low | 0,527 | -5,795 |
| 7 | PC aa C40:1.K1 | low | 0,540 | -1,266 |
| 8 | C16-OH.K1 | low | 0,546 | -7,050 |
| 9 | C12:1.K1 | low | 0,573 | -2,402 |
| 10 | C18:1-OH.K1 | low | 0,583 | -7,058 |
| 11 | lysoPC a C26:1.K1 | medium | 0,158 | 1,129 |
| 12 | PC ae C40:0.K1 | medium | 0,173 | 1,832 |
| 13 | PC aa C42:6.K1 | medium | 0,299 | -0,934 |
| 14 | PC aa C26:0.K1 | medium | 0,300 | -0,379 |
| 15 | PC ae C42:5.K1 | medium | 0,482 | 0,023 |
| 16 | PC ae C36:0.K1 | medium | 0,489 | 0,361 |
| 17 | PC ae C40:6.K1 | medium | 0,493 | 1,724 |
| 18 | SM C20:2.K1 | medium | 0,499 | -0,979 |
| 19 | SM (OH) C16:1.K1 | medium | 0,525 | 1,078 |
| 20 | SM (OH) C24:1.K1 | medium | 0,526 | 0,090 |
| 21 | SM C16:0.K1 | high | 0,436 | 5,937 |
| 22 | SM C16:1.K1 | high | 0,465 | 3,175 |
| 23 | Val.K2 | high | 0,472 | 7,526 |
| 24 | PC aa C34:2.K1 | high | 0,486 | 8,221 |
| 25 | PC aa C34:1.K1 | high | 0,488 | 8,384 |
| 26 | PC ae C38:4.K1 | high | 0,498 | 2,857 |
| 27 | PC aa C36:4.K1 | high | 0,505 | 6,706 |
| 28 | SM C24:1.K1 | high | 0,512 | 4,414 |
| 29 | Val-PTC.K1 | high | 0,520 | 7,509 |
| 30 | SM C18:0.K1 | high | 0,525 | 4,016 |

Beside the above straight forward approaches we use two algorithms which are known to work well for identifying housekeeping genes (reference genes) in case of real-time quantitative RT-PCR data. First, we apply the method of Vandesompele et al. [Vandesompele et al. (2002). Accurate normalization of real-time quantitative RT-PCR data by geometric averaging of multiple internal control genes. Genome Biology, 3(7):research0034.1-0034.11.] which is called geNorm. That is, we rank the analytes by the stability measure M introduced by Vandesompele et al. and in each step remove the analyte with the largest M value, i.e. the lowest stability. As the geNorm procedure is based on analyte ratios, the two most stable analytes cannot be ranked. geNorm ranks the analytes according to the similarity of the concentration profiles. Hence, the results are quite distinct from the results of the other selection methods (cf. Table 21) and indicate that there is one group of analytes where the analytes have very similar concentration profiles and hence dominate the selection process; confer
Table 19 where the 20 top ranked analytes are depicted.

**Table 19: 20 top ranked analytes using geNorm.**

| **Nr** | **Analyte** | **Rank** | **mean M** |
|---|---|---|---|
| 1 | PC ae C38:1.K1 | 1 | 0,170 |
| 2 | PC ae C38:2.K1 | 1 | 0,170 |
| 3 | SM (OH) C22:2.K1 | 3 | 0,215 |
| 4 | SM (OH) C22:1.K1 | 4 | 0,219 |
| 5 | PC ae C40:1.K1 | 5 | 0,245 |
| 6 | PC ae C40:2.K1 | 6 | 0,256 |
| 7 | SM (OH) C24:1.K1 | 7 | 0,268 |
| 8 | PC aa C40:1.K1 | 8 | 0,287 |
| 9 | PC ae C42:0.K1 | 9 | 0,297 |
| 10 | SM C26:1.K1 | 10 | 0,304 |
| 11 | PC aa C40:3.K1 | 11 | 0,309 |
| 12 | PC aa C38:1.K1 | 12 | 0,315 |
| 13 | PC ae C38:0.K1 | 13 | 0,323 |
| 14 | PC ae C40:4.K1 | 14 | 0,330 |
| 15 | PC aa C42:5.K1 | 15 | 0,335 |
| 16 | PC aa C42:4.K1 | 16 | 0,341 |
| 17 | PC ae C40:5.K1 | 17 | 0,346 |
| 18 | SM C24:0.K1 | 18 | 0,352 |
| 19 | PC aa C38:0.K1 | 19 | 0,357 |
| 20 | PC aa C42:6.K1 | 20 | 0,361 |

Finally, we use the method introduced by Andersen et al. (2004) [Andersen et al. (2004). Normalization of Real-Time Quantitative Reverse Transcription-PCR Data: A Model-Based Variance Estimation Approach to Identify Genes Suited for Normalization, Applied to Bladder and Colon Cancer Data Sets. Cancer Research 64:5245-5250.] which is called NormFinder. That is, we rank the analytes by the stability measure rho introduced by Andersen et al. where in each step the analyte with the smallest rho value, i.e. the highest stability, given the previously selected analytes is added. The NormFinder procedure takes the inter and intra group variability of the analyte concentrations into account where we consider the groups pneumonia, mixed sepsis and control. Table 20 shows the 50 top ranked analytes.

**Table 20: 50 top ranked analytes using NormFinder.**

| **Nr** | **Analyte** | **Rank** | **rho** |
|---|---|---|---|
| 1 | PC ae C40:0.K1 | 1 | 0,1035 |
| 2 | PC ae C42:5.K1 | 2 | 0,0687 |
| 3 | lysoPC a C14:0.K1 | 3 | 0,0607 |
| 4 | C12:1.K1 | 4 | 0,0600 |
| 5 | SM C16:1.K1 | 5 | 0,0564 |
| 6 | PC aa C36:4.K1 | 6 | 0,0477 |
| 7 | PC aa C34:4.K1 | 7 | 0,0445 |
| 8 | PC aa C38:4.K1 | 8 | 0,0454 |
| 9 | C12-DC.K1 | 9 | 0,0419 |
| 10 | PC aa C32:2.K1 | 10 | 0,0358 |
| 11 | PC aa C34:2.K1 | 11 | 0,0381 |
| 12 | SM C20:2.K1 | 12 | 0,0321 |
| 13 | PC aa C34:3.K1 | 13 | 0,0336 |
| 14 | PC aa C38:5.K1 | 14 | 0,0307 |
| 15 | Ser-PTC.K1 | 15 | 0,0331 |
| 16 | SM C24:1.K1 | 16 | 0,0305 |
| 17 | Ser.K2 | 17 | 0,0310 |
| 18 | C16:2-OH.K1 | 18 | 0,0298 |
| 19 | C12.K1 | 19 | 0,0285 |
| 20 | PC ae C36:2.K1 | 20 | 0,0306 |
| 21 | Val-PTC.K1 | 21 | 0,0286 |
| 22 | C0.K1 | 22 | 0,0275 |
| 23 | PC ae C38:4.K1 | 23 | 0,0295 |
| 24 | PC aa C36:5.K1 | 24 | 0,0286 |
| 25 | His-PTC.K1 | 25 | 0,0281 |
| 26 | C3:1.K1 | 26 | 0,0274 |
| 27 | PC aa C30:2.K1 | 27 | 0,0261 |
| 28 | SM C22:3.K1 | 28 | 0,0272 |
| 29 | PC aa C38:3.K1 | 29 | 0,0255 |
| 30 | PC aa C42:6.K1 | 30 | 0,0264 |
| 31 | C16-OH.K1 | 31 | 0,0245 |
| 32 | C16:2.K1 | 32 | 0,0251 |
| 33 | Val.K2 | 33 | 0,0252 |
| 34 | PC aa C40:6.K1 | 34 | 0,0234 |
| 35 | lysoPC a C20:3.K1 | 35 | 0,0253 |
| 36 | lysoPC a C20:4.K1 | 36 | 0,0229 |
| 37 | SM C16:0.K1 | 37 | 0,0249 |
| 38 | lysoPC a C26:1.K1 | 38 | 0,0225 |
| 39 | C5.K1 | 39 | 0,0233 |
| 40 | C18:1-OH.K1 | 40 | 0,0247 |
| 41 | PC aa C26:0.K1 | 41 | 0,0227 |
| 42 | His.K2 | 42 | 0,0232 |
| 43 | Trp-PTC.K1 | 43 | 0,0224 |
| 44 | Tyr.K2 | 44 | 0,0230 |
| 45 | SM C24:0.K1 | 45 | 0,0213 |
| 46 | lysoPC a C18:2.K1 | 46 | 0,0230 |
| 47 | Glu.EM | 47 | 0,0220 |
| 48 | PC ae C42:4.K1 | 48 | 0,0218 |
| 49 | Suc.EM | 49 | 0,0226 |
| 50 | PC aa C40:4.K1 | 50 | 0,0210 |

Table 21 shows a summary of all analytes which were chosen by at least one of the four different methods where the selection is indicated by TRUE. There are several analytes which were chosen by two or even three methods simultaneously.

**Table 21: Summary of the selected analytes. TRUE indicates that an analyte was chosen by the corresponding algorithm.**

| **Nr** | **Analyte** | **CV of raw data** | **SD of log data** | **geNorm** | **NormFinder** |
|---|---|---|---|---|---|
| 1 | C0.K1 | | | | TRUE |
| 2 | C12:1.K1 | | TRUE | | TRUE |
| 3 | C12-DC.K1 | TRUE | TRUE | | TRUE |
| 4 | C12.K1 | | | | TRUE |
| 5 | C16:2.K1 | | | | TRUE |
| 6 | C16:2-OH.K1 | | TRUE | | TRUE |
| 7 | C16-OH.K1 | | TRUE | | TRUE |
| 8 | C18:1-OH.K1 | | TRUE | | TRUE |
| 9 | C3:1.K1 | TRUE | TRUE | | TRUE |
| 10 | C3-OH.K1 | | TRUE | | |
| 11 | C5.K1 | | | | TRUE |
| 12 | Glu.EM | | | | TRUE |
| 13 | His.K2 | | | | TRUE |
| 14 | His-PTC.K1 | | | | TRUE |
| 15 | lysoPC a C14:0.K1 | | | | TRUE |
| 16 | lysoPC a C18:2.K1 | | | | TRUE |
| 17 | lysoPC a C20:3.K1 | | | | TRUE |
| 18 | lysoPC a C20:4.K1 | | | | TRUE |
| 19 | lysoPC a C26:1.K1 | TRUE | TRUE | | TRUE |
| 20 | PC aa C26:0.K1 | TRUE | TRUE | | TRUE |
| 21 | PC aa C30:2.K1 | | | | TRUE |
| 22 | PC aa C32:2.K1 | | | | TRUE |
| 23 | PC aa C34:1.K1 | | TRUE | | |
| 24 | PC aa C34:2.K1 | | TRUE | | TRUE |
| 25 | PC aa C34:3.K1 | | | | TRUE |
| 26 | PC aa C34:4.K1 | | | | TRUE |
| 27 | PC aa C36:4.K1 | | TRUE | | TRUE |
| 28 | PC aa C36:5.K1 | | | | TRUE |
| 29 | PC aa C38:0.K1 | | | TRUE | |
| 30 | PC aa C38:1.K1 | | | TRUE | |
| 31 | PC aa C38:3.K1 | | | | TRUE |
| 32 | PC aa C38:4.K1 | | | | TRUE |
| 33 | PC aa C38:5.K1 | | | | TRUE |
| 34 | PC aa C40:1.K1 | | TRUE | TRUE | |
| 35 | PC aa C40:3.K1 | | | TRUE | |
| 36 | PC aa C40:4.K1 | | | | TRUE |
| 37 | PC aa C40:6.K1 | | | | TRUE |
| 38 | PC aa C42:4.K1 | | | TRUE | |
| 39 | PC aa C42:5.K1 | TRUE | TRUE | TRUE | |
| 40 | PC aa C42:6.K1 | TRUE | TRUE | TRUE | TRUE |
| 41 | PC ae C36:0.K1 | TRUE | TRUE | | |
| 42 | PC ae C36:2.K1 | | | | TRUE |
| 43 | PC ae C38:0.K1 | | | TRUE | |
| 44 | PC ae C38:1.K1 | | | TRUE | |
| 45 | PC ae C38:2. K1 | | | TRUE | |
| 46 | PC ae C38:4.K1 | TRUE | TRUE | | TRUE |
| 47 | PC ae C40:0.K1 | TRUE | TRUE | | TRUE |
| 48 | PC ae C40:1.K1 | | | TRUE | |
| 49 | PC ae C40:2.K1 | | | TRUE | |
| 50 | PC ae C40:4.K1 | | | TRUE | |
| 51 | PC ae C40:5.K1 | TRUE | | TRUE | |
| 52 | PC ae C40:6.K1 | TRUE | TRUE | | |
| 53 | PC ae C42:0.K1 | TRUE | TRUE | TRUE | |
| 54 | PC ae C42:4.K1 | | | | TRUE |
| 55 | PC ae C42:5.K1 | TRUE | TRUE | | TRUE |
| 56 | Ser.K2 | | | | TRUE |
| 57 | Ser-PTC.K1 | | | | TRUE |
| 58 | SM C16:0.K1 | TRUE | TRUE | | TRUE |
| 59 | SM C16:1.K1 | TRUE | TRUE | | TRUE |
| 60 | SM C18:0.K1 | | TRUE | | |
| 61 | SM C20:2.K1 | TRUE | TRUE | | TRUE |
| 62 | SM C22:3.K1 | | | | TRUE |
| 63 | SM C24:0.K1 | TRUE | | TRUE | TRUE |
| 64 | SM C24:1.K1 | | TRUE | | TRUE |
| 65 | SM C26:1.K1 | | | TRUE | |
| 66 | SM (OH) C16:1.K1 | | TRUE | | |
| 67 | SM (OH) C22:1.K1 | | | TRUE | |
| 68 | SM (OH) C22:2.K1 | | | TRUE | |
| 69 | SM (OH) C24:1.K1 | TRUE | TRUE | TRUE | |
| 70 | Suc.EM | | | | TRUE |
| 71 | Trp-PTC.K1 | | | | TRUE |
| 72 | Tyr. K2 | | | | TRUE |
| 73 | Val. K2 | TRUE | TRUE | | TRUE |
| 74 | Val-PTC.K1 | TRUE | TRUE | | TRUE |

### Statistical Analysis

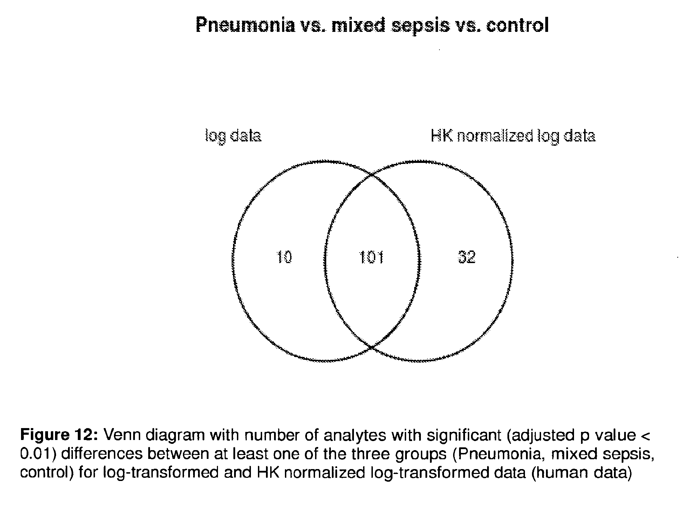

We perform a Welch-modification of the one-way ANOVA to analyze the data using log-transformed and HK normalized log-transformed concentrations. The obtained p values are corrected by the method of Benjamini and Hochberg (1995) [Benjamini Y., and Hochberg Y. (1995). Controlling the false discovery rate: a practical and powerful approach to multiple testing. Journal of the Royal Statistical Society Series B 57:289-300]. As housekeeping analytes we use lysoPC a C26:1.K1, PC ae C40:0.K1, PC aa C42:6.K1, PC aa C26:0.K1 which are the top four analytes with the lowest SD at medium concentration. We compare the results of log-transformed and housekeeper (HK) normalized log-transformed data. The normalization by means of housekeeping analytes is performed by subtracting the mean of the four selected housekeepers from the log-concentrations of the remaining analytes. The Venn diagram in Figure 12 is based on all metabolites having adjusted p values smaller than 0.01. The results for the two approaches are in very good agreement with an advantage in terms of more significant differences in case of the HK normalized log-transformed data. This indicates that the use of the HK normalized log-transformed data leads to an increased power of the statistical analysis.
The assumption of an increased power is confirmed by area under curve (AUC) computations. For the comparisons Control vs. Pneumonia and Control vs. Mixed Sepsis we compute the AUC, which is a measure for the predictive power, for each of the analytes. From the 195 analytes included in this analysis 106 (Control vs. Pneumonia) and 128 (Control vs. Mixed Sepsis), respectively, have a larger AUC in case of the HK normalized log-transformed data. The proportion 65.6% (p < 0.001) for Control vs. Mixed Sepsis is significantly different (i.e., larger) from 50%, the proportion for Control vs. Pneumonia is also larger than 50% (54.3%) but not significantly different from 50%. At the same time the maximum AUC stays about the same 0.997 (log data) vs. 1.000 (HK) for Control vs. Pneumonia respectively, is slightly increased 0.945 (log data) vs. 0.971 (HK) for Control vs. Mixed Sepsis. These results again speak for an increased power of the statistical analysis by using the HK normalized log-transformed data instead of the log-transformed data.

The identification and use of diagnostic metabolites is based on the finding and the essential feature that the control metabolites and their levels do not vary along with the disease type or the state of a given disease and can not be distinguished from control metabolite levels in healthy individuals. This allows the quantitative determination of other metabolites and their changes along with the state of a disease and thus diagnosis, prognosis and therapy control.

Normalization of quantitative data by means of control-, reference or housekeeping metabolites offers several advantages. It enables the identification of metabolites with among various states of health or due to a disease or a distinct grade/ score of a disease differentially regulated concentrations / levels of metabolites as well as the development of diagnostic tools based on that.
This invention provides the identities of analytes and metabolites that can be used as normalization, endogenous reference, or "housekeeping" analyte or metabolite in samples. The concentration level(s) of these analytes or metabolites may be advantageously used in methods based on determination of metabolic information to diagnose or classify a disease and applied to these ends to samples of subjects from different species, diseased states and tissues.

In some embodiments, subsets of the endogenous reference metabolites of the present invention can be advantageously used for normalization of distinct variable metabolites to characterize the physiology of a subject or to diagnose a certain disease in a subject.

The applications of endogenous reference metabolites thus also include the identification of responders and non-responders, therapy control and the use of specific responsive metabolites to these ends.
The method of our invention is characterized by the ability of determining the presence or absence, the amount, the level or the concentration of one or more metabolites or metabolic biomarker in the sample of a subject relative to the amount, level or concentration of one or more endogenous reference metabolites and of using this information for diagnosis, prognosis and therapy control.
The endogenous reference metabolites can be used as single endogenous reference metabolites and values or in a combination of

In one embodiment said control- or target metabolites can be used in trials for determination of toxicity, safety and efficacy of compounds or mixtures of compounds and drugs and to model signaling pathways.

The present invention further provides a method for comparing data obtained with different assay platforms, wherein the above steps for normalizing are reiterated and the normalization factor is used to correct a signal provided by measurement of the test sample with one given method such as mass spectrometry which correction makes said signal directly comparable to a metabolite signal provided by assaying the same metabolite of the reference with one or several of alternative method for metabolite and endogenous reference metabolite determination such as IR-spectroscopy, NMR-spectroscopy, Raman spectroscopy, immunoassays, ELISAs, Western blotting, binding assays using aptamers, nucleotides or chemically modified aptamers or nucleotides for metabolite binding and indication of levels by any quantifiable signal such as eg. fl uorescence.
Data and metabolite levels thus can be determined by any assay known to a person skilled in the arts such as, but not limited to infrared spectroscopy, raman spectroscopy, mass spectroscopy, ELISAs or other immunological assays using antibodies and or enzymes and or nucleotides and or aptamers or by other methods for specific binding or immobilization of metabolites or their recognizing counterparts, to solid supports and applying various methods for detection and visualization such as, but not limited to fluorescence imaging, or MRI imaging.

The method of the present invention is suitable for use in high-throughput screening experiments.

The endogenous reference metabolites may be chemically modified or may be labelled by any suitable means known to a person skilled in the arts, such as by labeling with dyes containingg reactive chemical groups such as but not limited to reaction with isothiocyanates, cyanates, anhydrides, thiols, amines, an azo (N3) group or fluorine, activated esters such as N-hydroxysuccinimide esters, or labelling with cyanines, biotin, digoxygenin, fluorescein, a dideoxynucleotide, or any other form of label.

Another embodiment is characterized by use of a radioactive marker or label, in particular ³²P, ¹⁴C, ¹²⁵I, ³³P or ³H for detection of control- or target metabolites.

Another embodiment is characterized by use of a non-radioactive detectable marker, in particular a dye or a fluorescent marker or quantum dots, an enzyme marker, an immune marker or a marker enabling detection via electrical signals in particular change of current, resistance, or capacity on endogenous reference or target metabolites.

The expression of analytes thus may also be detected by use of immuno-histochemistry techniques or assays applying enzymes or other antibody or aptamer or related technologies-mediated detection as non-limiting examples. Additional means for analysis of analyte determination are available, including enzymatic, or (catalytic chemical transformation of metabolites or detection by Raman spectroscopy, IR-spectroscopy, NMR-spectroscopy, UV-spectroscopy and other spectroscopic technologies familiar to persons skilled in the arts. These methods may also include preceding or intermediate chemical modification or labeling, e.g. with isotopes or functional groups containing respective isotopes such as carbon 13, deuterium atoms or derivatization with fluorescent or paramagnetic labels to enable specialized methods of detection and quantification.

In some embodiments this can include specific labeling of all known or a fraction of the reference analytes or a differential treatment or labeling of endogenous reference metabolites and variable metabolites.
Other objects, advantages and features of the present invention will become more apparent upon reading of the following non restrictive description of preferred embodiments thereof, given by way of example only with reference to the accompanying drawings.

In another embodiment, levels of endogenous reference metabolites can be compared with the levels of metabolites with the values of the endogenous reference metabolites or target metabolites determined in separate experiments or used in the form of calibration curves or calibration tables.

In some embodiments, the invention provides a method of determining the metabolite level of one or more metabolites in a cell, tissue or body liquid from a subject. The method comprises determining the metabolite level of one or more metabolites. The method comprises determining the metabolite concentrations of said one or more metabolites or analytes, and comparing said concentration level(s) to the concentration level of a reference analyte or metabolite as described above.
The concentration level(s) of one or more reference analytes of the invention provides a means to "normalize" the metabolomics data from analytes and metabolites of interest for comparison of data from a sample. Stated differently, the concentration or relative abundance of a metabolite of interest is calculated in a manner "relative to" the levels(s) of one or more reference analytes of the invention. The normalization may also be used for comparisons between samples, especially when they are conducted in separate experiments. The methods of the invention may be advantageously used in a kit format, a multi-well array, an array based format, and thus a plurality of metabolites may be evaluated for their concentration at the same time. One or more of the reference analytes of the invention may also be evaluated as part of the same experiment.

In some non-limiting embodiments, the endogenous reference metabolites of the invention are used in a method of classifying a subject as diseased, to diagnose to disease, to classify a cell or a tissue as diseased, to recognize a cell containing sample as including a tumor cell of (or from) a type of tissue or a tissue origin. The classification or diagnosis is based upon a comparison of the levels or concentrations or relative abundance of a plurality of analytes in the sample to their levels in confirmed diseased samples and/or known non-diseased samples.

As used herein, "a plurality" refers to the state of two or more. Such use in classification will be used in additional description of the invention below as a non-limiting example. The reference analytes of the invention may also be used in the classification of body liquids taken from- or a sample as containing cells from a tissue or organ site, without limitation to tumor cells.

In additional non-limiting embodiments, the invention is described with respect to human subjects. However, samples from other subjects may also be used. All that is necessary is the ability to assess the levels or concentrations of metabolites in a plurality of known samples such that the expression levels in an unknown or test sample may be compared. Thus the invention may be applied to samples from any organism for which a plurality of metabolites, and a plurality of known samples, is available.

In one preferred embodiment control metabolites and comparison to target metabolites are used to diagnose asphyxia, severity of asphyxia and to control therapy and treatment of asphyxia.

In one preferred embodiment control metabolites and comparison to target metabolites are used to diagnose hypoxia, ischemia, ischemic encephalopathy and stroke, perinatal asphyxia, choking, drowning, electric shock, injury, or the inhalation of toxic gases, and/or determine the severities of said disorders and/or to control therapy and treatment of these conditions.
Furthermore, with the method of the present invention, for the first time, a method for normalizing in vitro monitoring of normoxic, hypoxic and hyperoxic conditions and/or normobaric and hyperbaric oxygen therapy by endogenous metabolites is provided. Such method is characterized by use of at least one endogenous reference metabolite and at least one biological sample of at least one tissue of a mammalian subject.
For example, in some embodiments, the present invention provides a method of diagnosing asphyxia, hypoxia or ischemia and/or duration/severity comprising the use of one or more (e.g., 2 or more, 3 or more, 5 or more, 10 or more, etc.) endogenous reference metabolites according to the present invention, measured together with one or 2 or more, 3 or more, 5 or more, 10 or more, etc. target metabolites in a multiplex or panel format: detecting the presence or absence of or quantitate one or more (e.g., 2 or more, 3 or more, 5 or more, 10 or more, etc.) asphyxia specific target metabolites (measured together in a multiplex or panel format) in a sample (e.g., a tissue (e.g., biopsy) sample, a blood sample, a serum sample, or a urine sample) from a subject; and diagnosing asphyxia based on the normalized values of asphyxia specific target metabolites.
The present invention further provides a method of screening compounds by superior quantitation of responsive metabolites comprising: contacting an animal, a tissue, a cell containing an asphyxia-specific metabolite with a test compound; and determine the level of the asphyxia specific metabolite quantitatively by use of the endogenous reference metabolites. In some embodiments, the method further comprises the step of comparing the level of the asphyxia specific metabolite in the presence of the test compound or therapeutic intervention to the level of the asphyxia specific metabolite in the absence of the asphyxia specific metabolite applying said endogenous reference metabolites. In some embodiments, the cell is in vitro, in a non-human mammal, or ex vivo. In some embodiments, the test compound is a small molecule or a nucleic acid (e.g., antisense nucleic acid, a siRNA, or a miRNA) or oxygen/xenon or any neuroprotective drug that inhibits the expression of an enzyme involved in the synthesis or breakdown of an asphyxia specific metabolite. In some embodiments, the method is a high throughput method.
"Asphyxia" in this context relates to any diseased state linked to lack of oxygen, oxygen saturation, hypoxia. Asphyxia can be induced either pre-/perinatally due to a lack of oxygen supply by the umbilical cord or can be caused by any condition associated with an inability to breathe and/or inadequate lung ventilation like choking, drowning, electric shock, injury, or the inhalation of toxic gases.
In one preferred embodiment control metabolites and comparison to target metabolites are used to diagnose response of a subject to bacterial fragments or fragments of bacterial cell walls or immune response to bacterial fragments or fragments of bacterial cell walls, severity of said conditions and to control therapy and treatment of conditions due to systemic inflammation.

In a preferred embodiment of the present invention, the endogenous reference metabolites and target metabolite levels normalized and quantitated by use of these endogenous reference metabolites are applied to characterize, determine or confirm pathophysiological conditions corresponding to the label "diseased", physiological conditions corresponding to the label "healthy" or pathophysiological conditions corresponding to different labels of "grades of a disease", "subtypes of a disease", different values of a "score for a defined disease"; said prognostic conditions corresponding to a label "good", "medium", "poor", or "therapeutically responding" or "therapeutically non-responding" or "therapeutically poor responding".
A particular useful application of the method in accordance with the present invention is that said disorder is hypoxic ischemic encephalopathy, neonatal asphyxia, or systemic inflammation or sepsis or immune response, said mammalian subject is a human being, said biological sample is blood, wherein missing data is imputed;
wherein raw data of metabolite concentrations are preprocessed using the log transformation;
wherein linear mixed effect models are used to identify metabolites which are differentially present;
wherein random forest is selected as suitable classifying algorithm, the training of the classifying algorithm including preprocessed metabolite concentrations, is carried out with stratified bootstrap replications
applying said trained random forests classifier to said preprocessed metabolite concentration data set to a subject under suspicion of having hypoxic ischemic encephalopathy, and using the trained classifier to diagnose hypoxic ischemic encephalopathy.

Preferably, the tissue from which the biological samples can be obtained is selected from the group consisting of blood and other body fluids, cerebrospinal fluids, urine; brain tissue, nerve tissue, and/or said sample is a biopsy sample and/or said mammalian subject includes humans.
In some embodiments endogenous reference metabolite based normalization may be used in species comparison, comparison of different tissue types, comparison of the same type of tissue among different mammalian species, comparison of different types of tissue among different mammalian species. Thus the invented method and provided endogenous reference metabolites can be used in animal model comparison and data transfer, including data comparison and transfer from animal models to man and vice versa and in applications including but not limited to drug development in mammals and man, in diagnosis and animal diagnostics.

Thus, the invention is contemplated for use with other samples, including those of mammals, primates, and animals used in clinical testing (such as rats, mice, rabbits, dogs, cats, and chimpanzees) as non-limiting examples. The invention provides for the normalization of the metabolite levels of the assay with one or more of the reference analytes disclosed herein. Thus, the classifying may alternatively be based upon a comparison of the levels of the assay analytes to the levels of reference analytes in the same samples, relative to, or based on, the same comparison in known diseased samples and/or known non-diseased samples. As a non-limiting example, the normalized analyte levels of the assay may be determined in a set of known diseased samples to provide a database against which the normalized analyte levels detected or determined in a sample, tissue, body liquid or cell containing sample from a subject is compared. The analyte level(s) in a sample also may be compared to the level(s) of said analytes in normal or non-diseased sample, tissue, body liquid or cell, preferably from the same sample or subject. In other embodiments of the invention the analyte levels may be compared to levels of reference analytes in the same sample or a ratio of concentration levels may be used.

Beyond target metabolite values, the method may further comprise inclusion of standard lab parameters commonly used in clinical chemistry and critical care units, in particular, blood gases, preferably arterial blood oxygen, blood pH, base status, and lactate, serum and/or plasma levels of routinely used low molecular weight biochemical compounds, enzymes, enzymatic activities, cell surface receptors and/or cell counts, in particular red and/or white cell counts, platelet counts.
One non-limiting example of this is seen in the case of a multi-well or array-based platform to determine metabolite concentrations, where the level of other metabolites is also measured.
Data from control metabolites or derived from control metabolite level data can be used and further processed for software and or used in expert systems, patient management systems or distance diagnosis systems and used in diagnosing a disease, differentiate healthy from diseased subjects, identify or characterize states of a disease, determine the severity of a disease and determine scores, to determine the origin or cause of a disease.
It is clear to a person skilled in the art that the features of the present invention as described in said embodiments can be used in any possible combinations.

## Claims

1. Method for normalization of intensity data corresponding to amounts and/or concentrations of selected target metabolites in a biological sample of a mammalian subject, wherein said intensity data are obtained by a metabolomics analysis method comprising the generation of intensity data for the quantitation of endogenous metabolites by mass spectrometry (MS), in particular MS-technologies such as Matrix Assisted Laser Desorption/Ionisation (MALDI), Electro Spray Ionization (ESI), Atmospheric Pressure Chemical Ionization (APCI), ¹H-, ¹³C- and/or ³¹P-Nuclear Magnetic Resonance spectroscopy (NMR), optionally coupled to MS, determination of metabolite concentrations by use of MS-technologies and/or methods coupled to separation, in particular Liquid Chromatography (LC-MS), Gas Chromatography (GC-MS), or Capillary Electrophoresis (CE-MS), with a plurality of endogenous reference metabolites,
comprising
carrying out at least one in vitro metabolomics analysis method of said selected target metabolites in said biological sample;
simultaneously carrying out in the same sample a quantitative analysis of a plurality of endogenous reference metabolites or derivatives thereof, wherein said endogenous reference metabolites are such compounds in the biological sample which are present in the subject at an essentially constant level; and wherein said endogenous reference metabolites or derivatives thereof have a molecular mass less than 1500 Da and are selected from the group consisting of:
Amino acids, in particular, arginine, aspartic acid, citrulline, glutamic acid (glutamate), glutamine, leucine, isoleucine, histidine, ornithine, proline;
phenylalanine, serine, tryptophane, tyrosine, valine, kynurenine;
phenylthio carbamyl amino acids (PTC-amino acids), in particular, PCT-arginine, PTC-glutamine, PTC-histidine, PTC-methionine, PTC-ornithine) PTC-phenylalanine, PTC-proline, PTC-serine, PTC-tryptophane, PTC-tyrosine, PTC-valine;
dimethylarginine, in particular N, N-dimethyl-L-arginine;
carboxylic acids, namely 15(S)-hydroxy-5Z, 8Z, 11Z, 13E-eicosatetraenoic acid [(5Z, 8Z, 11Z, 13E, 15S)-15-Hydroxyicosa-5, 8, 11, 13-tetraenoic acid], succinic acid (succinate);
carnitine; acylcarnitines having from 1 to 20 carbon atoms in the acyl residue; acylcarnitines having from 3 to 20 carbon atoms in the acyl residue and having 1 to 4 double bonds in the acyl residue; acylcarnitines having from 1 to 20 carbon atoms in the acyl residue and having from 1 to 3 OH-groups in the acyl residue; acylcarnitines having from 3 to 20 carbon atoms in the acyl residue with 1 to 4 double bonds and 1 to 3 OH-groups in the acyl residue;
phospholipides, in particular lysophosphatidylcholines (monoacylphosphatidylcholines) having from 1 to 30 carbon atoms in the acyl residue; lysophosphatidylcholines having from 3 to 30 carbon atoms in the acyl residue and having 1 to 6 double bonds in the acyl residue;
phosphatidylcholines (diacylphosphatidylcholines) having a total of from 1 to 50 carbon atoms in the acyl residues; phatidylcholines having a total from 3 to 50 carbon atoms in the acyl residues and having a total of 1 to 8 double bonds in the acyl residues;
sphingolipids, in particular sphingomyelines having a total number of carbon atoms in the acyl chains from 10 to 30; sphingomyelines having a total number of carbon atoms in the acyl chains from 10 to 30 and 1 to 5 double bonds; hydroxysphinogomyelines having a total number of carbon atoms in the acyl residues from 10 to 30; hydroxysphingoyelines having a total number of carbon atoms in the acyl residues from 10 to 30 and 1 to 5 double bonds;
prostaglandines, namely 6-keto-prostaglandin F1 alpha, prostagiandin D2;
putrescine;
and wherein
said detected intensities of said selected target metabolites each are related to said intensities of said endogenous reference metabolites.

2. Method according to claim 1, wherein the plurality of intensities of the target and endogenous reference metabolites are subjected to a mathematical preprocessing, in particular transformations such as applying logarithms, generalized logarithms, power transformations.

3. Method according to claim 1 or 2, wherein the plurality of intensities of the endogenous reference metabolites are aggregated to one reference value.

4. Method according 10 claim 3, wherein the plurality of intensities of the endogenous reference metabolites are aggregated to one reference value by calculation of geometric mean value, arithmetic mean value, median value, weighted arithmetic mean value.

5. Method according to anyone of claims 1 to 4, wherein a ratio is formed by each of the intensities of the target metabolites and the determined reference value in case of linear intensities, or the determined reference value is subtracted from each target metabolite intensity in case of logarithmic intensities.

6. Method according to anyone of claims 1 to 5, wherein said endogenous reference metabolites are selected from the group consisting of:
15((S)-hydroxy-5Z, 8Z, 11Z, 13E-eicosatetraenoic acid
6-keto-Prostaglandin F1 alpha
asymmetrical Dimethylarginin
Arginine
PTC-Arginine
Aspartic acid
Carnitine (free)
Decanoylcarnitine [Caprylcarnitine] (Fumarylcarnitine)
Decenoylcarnitine
Decadienoylcarnitine
Dodecanoylcarnitine [Laurylcarnitine]
Dodecenoylcarnitine
Dodecanedioylcarnitine
Tetradecanoylcarnitine
Tetradecenoylcarnitine [Myristoleylcarnitine]
3-Hydroxytetradecenoylcarnitine [3-Hydroxymyristoleylcarnitine]
3-Hydroxytetradecadienoylcarnitine
3-Hydroxytetradecanoylcarnitine [Hydroxymyristylcarnitine]
Hexadecenoylcarnitine [Palmitoleylcarnitine]
3-Hydroxyhexadecenoylcarnitine [3-Hydroxypalmitoleylcarnitine]
Hexadecadienoylcarnitine
3-Hydroxyhexadecadienoylcarnitine
3-Hydroxyhexadecanolycarnitine [3-Hydroxypalmitoylcarnitine]
Octadecanoylcarnitine [Stearylcarnitine]
Octadecenoylcarnitine [Oleylcarnitine]
3-Hydroxyoctadecenoylcarnitine [3-Hydroxyoleylcarnitine]
Acetylcarnitine
Propenoylcarnitine
Hydroxypropionylcarnitine
Butenoylcarnitine
3-Hydroxybutyrylcarnitine / Malonylcarnitine
Isovalerylcarnitine / 2-Methylbutyrylcarnitine / Valerylcarnitine
Tiglylcarnitine / 3-Methyl-crotonylcarnitine
Glutarylcarnitine / Hydroxycaproylcarnitine
Glutarylcarnitine / Hydroxycaproylcarnitine
Methylglutarylcarnitine
3-Hydroxyisovalerylcarnitine / 3-Hydroxy-2-methylbutyryl
Hexanoylcarnitine [Caproylcarnitine]
Hexenoylcarnitine
Pimelylcarnitine
Octanoylcarnitine [Caprylylcarnitine]
Octenoylcarnitine
Nonanoylcarnitine [Pelargonylcarnitine]
Citrulline
Creatinine
Glutamine
PTC-Glutamine
Glutamate
Histidine
PTC-Histidine
Kynurenine
Leucine
Lysophosphatidylcholine with acyl residue C14:0
Lysophosphatidylcholine with acyl residue C16:0
Lysophosphatidylcholine with acyl residue C16:1
Lysophosphatidylcholine with acyl residue C18:0
Lysophosphatidylcholine with acyl residue C18:1
Lysophosphatidylcholine with acyl residue C18:2
Lysophosphatidylcholine with acyl residue C20:3
Lysophosphatidylcholine with acyl residue C20:4
Lysophosphatidylcholine with acyl residue C24:0
Lysophosphatidylcholine with acyl residue C26:0
Lysophosphatidylcholine with acyl residue C26:1
Lysophosphatidylcholine with acyl residue C28:0
Lysophosphatidylcholine with acyl residue C28:1
Lysophosphatidylcholine with acyl residue C6:0
PTC-Methionine
Ornithine
PTC-Ornithine
Phosphatidylcholine with diacyl residue sum C24:0
Phosphatidylcholine with diacyl residue sum C26:0
Phosphatidylcholine with diacyl residue sum C28:1
Phosphatidylcholine with diacyl residue sum C30:0
Phosphatidylcholine with diacyl residue sum C30:2
Phosphatidylcholine with diacyl residue sum C32:0
Phosphatidylcholine with diacyl residue sum C32:1
Phosphatidylcholine with diacyl residue sum C32:2
Phosphatidylcholine with diacyl residue sum C32:3
Phosphatidylcholine with diacyl residue sum C34:1
Phosphatidylcholine with diacyl residue sum C34:2
Phosphatidylcholine with diacyl residue sum C34:3
Phosphatidylcholine with diacyl residue sum C34:4
Phosphatidylcholine with diacyl residue sum C36:0
Phosphatidylcholine with diacyl residue sum C36:1
Phosphatidylcholine with diacyl residue sum C36:2
Phosphatidylcholine with diacyl residue sum C36:3
Phosphatidylcholine with diacyl residue sum C36:4
Phosphatidylcholine with diacyl residue sum C36:5
Phosphatidylcholine with diacyl residue sum C36:6
Phosphatidylcholine with diacyl residue sum C38:0
Phosphatidylcholine with diacyl residue sum C38:1
Phosphatidylcholine with diacyl residue sum C38:3
Phosphatidylcholine with diacyl residue sum C38:4
Phosphatidylcholine with diacyl residue sum C38:5
Phosphatidylcholine with diacyl residue sum C38:6
Phosphatidylcholine with diacyl residue sum C42:1
Phosphatidylcholine with diacyl residue sum C40:3
Phosphatidylcholine with diacyl residue sum C40:4
Phosphatidylcholine with diacyl residue sum C40:5
Phosphatidylcholine with diacyl residue sum C40:6
Phosphatidylcholine with diacyl residue sum C42:0
Phosphatidylcholine with diacyl residue sum C42:1
Phosphatidylcholine with diacyl residue sum C42:2
Phosphatidylcholine with diacyl residue sum C42:4
Phosphatidylcholine with diacyl residue sum C42:5
Phosphatidylcholine with diacyl residue sum C42:6
Phosphatidylcholine with acyl-alkyl residue sum C30:0
Phosphatidylcholine with acyl-alkyl residue sum C30:1
Phosphatidylcholine with acyl-alkyl residue sum C34:0
Phosphatidylcholine with acyl-alkyl residue sum C34:1
Phosphatidylcholine with acyl-alkyl residue sum C34:2
Phosphatidylcholine with acyl-alkyl residue sum C34:3
Phosphatidylcholine with acyl-alkyl residue sum C36:0
Phosphatidylcholine with acyl-alkyl residue sum C36:1
Phosphatidylcholine with acyl-alkyl residue sum C36:2
Phosphatidylcholine with acyl-alkyl residue sum C36:3
Phosphatidylcholine with acyl-alkyl residue sum C36:4
Phosphatidylcholine with acyl-alkyl residue sum C38:0
Phosphatidylcholine with acyl-alkyl residue sum C38:1
Phosphatidylcholine with acyl-alkyl residue sum C38:2
Phosphatidylcholine with acyl-alkyl residue sum C38:3
Phosphatidylcholine with acyl-alkyl residue sum C38:4
Phosphatidylcholine with acyl-alkyl residue sum C38:5
Phosphatidylcholine with acyl-alkyl residue sum C38:6
Phosphatidylcholine with acyl-alkyl residue sum C40:0
Phosphatidylcholine with acyl-alkyl residue sum C40:1
Phosphatidylcholine with acyl-alkyl residue sum C40:2
Phosphatidylcholine with acyl-alkyl residue sum C40:3
Phosphatidylcholine with acyl-alkyl residue sum C40:4
Phosphatidylcholine with acyl-alkyl residue sum C40:5
Phosphatidylcholine with acyl-alkyl residue sum C40:6
Phosphatidylcholine with acyl-alkyl residue sum C42:0
Phosphatidylcholine with acyl-alkyl residue sum C42:1
Phosphatidylcholine with acyl-alkyl residue sum C42:3
Phosphatidylcholine with acyl-alkyl residue sum C42:4
Phosphatidylcholine with acyl-alkyl residue sum C42:5
Phosphatidylcholine with acyl-alkyl residue sum C44:5
Phosphatidylcholine with acyl-alkyl residue sum C44:6
Prostaglandin D2
Phenylalanine
PTC-Phenylalanine
Proline
PTC-Proline
Putrescine
Serine
PTC-Serine
Hydroxysphingomyeline with acyl residue sum C14:1
Hydroxysphingomyelin with acyl residue sum C16:1
Hydroxysphingomyeline with acyl residue sum C22:1
Hydroxysphingomyeline with acyl residue sum C22:2
Hydroxysphingomyelin with acyl residue sum C24:1
Sphingomyeline with acyl residue sum C16:0
Sphingomyeline with acyl residue sum C16:1
Sphingomyeline with acyl residue sum C18:0
Sphingomyeline with acyl residue sum C18:1
Sphingomyeline with acyl residue sum C20:2
Sphingomyeline with acyl residue sum C22:3
Sphingomyeline with acyl residue sum C24:0
Sphingomyeline with acyl residue sum C24:1
Sphingomyeline with acyl residue sum C26:0
Sphingomyeline with acyl residue sum C26:1
Succinic acid (succinate)
Total dimethylarginine: sum ADMA + SDMA
Tryptophan
PTC-Tryptophan
Tyrosine
Valine
PTC-Valine
Leucine + Isoleucine
wherein the designation "residue Cn:m" or "residue sum Cn:m" represents the chain length of the acyl/alkyl residue(s), n represents the number of total carbon atoms in the acyl/alkyl residue(s), and m represents the number of total double bonds in the residue(s).

7. Method according to anyone of claims 1 to 6, wherein such metabolites are used as the endogenous reference metabolites which show stability in accordance with statistical stability measures being selected from the group consisting of coefficient of variation (CV) of raw intensity data, standard deviation (SD) of logarithmic intensity data, stability measure (M) of geNorm-algorithm or stability measure value (rho) of NormFinder-algorithm.

8. Method according to anyone of claims 1 to 7, wherein said endogenous reference metabolites show stability in accordance with at least 2 statistical stability measures being selected from the group consisting of coefficient of variation (CV) of raw intensity data, standard deviation (SD) of logarithmic intensity data, stability measure (M) of geNorm-algorithm or stability measure value (rho) of NormFinder-algorithm, and/or such endogenous reference metabolites being in particular selected from the group consisting of:
PTC-Arginine
Carnitine (free)
Decenoylcarnitine
Decanoylcarnitine [Caprylcarnitine] (Fumarylcarnitine)
Dodecenoylcarnitine
Dodecanedioylcarnitine
Dodecanoylcarnitine [Laurylcarnitine]
Tetradecanoylcarnitine
3-Hydroxytetradecanoylcarnitine [Hydroxymyristylcarnitine]
Hexadecenoylcarnitine [Palmitoleylcarnitine]
3-Hydroxyhexadecenoylcarnitine [3-Hydroxypalmitoleylcarnitine]
3-Hydroxyhexadecadienoylcarnitine
3-Hydroxyhexadecanolycarnitine [3-Hydroxypalmitoylcarnitine]
3-Hydroxyoctadecenoylcarnitine [3-Hydroxyoleylcarnitine]
Propenoylcarnitine
Hydroxypropionylcarnitine
3-Hydroxybutyrylcarnitine *I* Malonylcarnitine
Methylglutarylcarnitine
3-Hydroxyisovalerylcarnitine / 3-Hydroxy-2-methylbutyryl
Hexenoylcarnitine
Hexanoylcarnitine [Caproylcarnitine]
Pimelylcarnitine
Octenoylcarnitine
Octanoylcarnitine [Caprylylcarnitine]
Glutamine
PTC-Glutamine
PTC-Histidine
Lysophosphatidylcholine with acyl residue C14:0
Lysophosphatidylcholine with acyl residue C26:0
Lysophosphatidylcholine with acyl residue C26:1
Lysophosphatidylcholine with acyl residue C28:0
Lysophosphatidylcholine with acyl residue C28:1
Phosphatidylcholine with diacyl residue sum C24:0
Phosphatidylcholine with diacyl residue sum C26:0
Phosphatidylcholine with diacyl residue sum C30:0
Phosphatidylcholine with diacyl residue sum C30:2
Phosphatidylcholine with diacyl residue sum C32:2
Phosphatidylcholine with diacyl residue sum C34:2
Phosphatidylcholine with diacyl residue sum C36:0
Phosphatidylcholine with diacyl residue sum C36:2
Phosphatidylcholine with diacyl residue sum C36:4
Phosphatidylcholine with diacyl residue sum C38:0
Phosphatidylcholine with diacyl residue sum C38:1
Phosphatidylcholine with diacyl residue sum C42:1
Phosphatidylcholine with diacyl residue sum C42:0
Phosphatidylcholine with diacyl residue sum C42:5
Phosphatidylcholine with diacyl residue sum C42:6
Phosphatidylcholine with acyl-alkyl residue sum C30:1
Phosphatidylcholine with acyl-alkyl residue sum C34:1
Phosphatidylcholine with acyl-alkyl residue sum C36:0
Phosphatidylcholine with acyl-alkyl residue sum C38:1
Phosphatidylcholine with acyl-alkyl residue sum C38:4
Phosphatidylcholine with acyl-alkyl residue sum C38:6
Phosphatidylcholine with acyl-alkyl residue sum C40:0
Phosphatidylcholine with acyl-alkyl residue sum C40:1
Phosphatidylcholine with acyl-alkyl residue sum C40:5
Phosphatidylcholine with acyl-alkyl residue sum C40:6
Phosphatidylcholine with acyl-alkyl residue sum C42:0
Phosphatidylcholine with acyl-alkyl residue sum C42:5
Phosphatidylcholine with acyl-alkyl residue sum C44:6
Phenylalanine
PTC-Phenylalanine
Proline
Sphingomyeline with acyl residue sum C16:0
Sphingomyeline with acyl residue sum C16:1
Sphingomyeline with acyl residue sum C18:0
Sphingomyeline with acyl residue sum C18:1
Sphingomyeline with acyl residue sum C20:2
Sphingomyeline with acyl residue sum C24:0
Sphingomyeline with acyl residue sum C24:1
Hydroxysphingomyeline with acyl residue sum C14:1
Hydroxysphingomyeline with acyl residue sum C16:1
Hydroxysphingomyeline with acyl residue sum C22:2
Hydroxysphingomyelin with acyl residue sum C24:1
Succinic acid (succinate)
PTC-Tryptophan
Valine
PTC-Valine
Leucine + Isoleucine
wherein the designation "residue Cn:m" or "residue sum Cn:m" represents the chain length of the acyl/alkyl residue(s), n represents the number of total carbon atoms in the acyl/alkyl residue(s), and m represents the number of total double bonds in the residue(s).

9. Method according to anyone of claims 1 to 8, wherein said endogenous reference metabolites show stability in accordance with at least 3 statistical stability measures being selected from the group consisting of coefficient of variation (CV) of raw intensity data, standard deviation (SD) of logarithmic intensity data, stability measure (M) of geNorm -algorithm or stability measure value (rho) of NormFinder-algorithm, and/or such endogenous reference metabolites being in particular selected from the group consisting of:
Carnitine (free)
Decanoylcarnitine [Caprylcarnitine] (Fumarylcarnitine)
Dodecanedioylcarnitine
Dodecanoylcarnitine [Laurylcarnitine]
Hexadecenoylcarnitine [Palmitoleylcarnitine]
3-Hydroxyhexadecenoylcarnitine [3-Hydroxypalmitoleylcarnitine]
3-Hydroxyhexadecanolycarnitine [3-Hydroxypalmitoylcarnitine]
Propenoylcarnitine
Hydroxypropionylcarnitine
3-Hydroxybutyrylcarnitine / Malonylcarnitine
Methylglutarylcarnitine
Hexanoylcarnitine [Caproylcarnitine]
Pimelylcarnitine
Octenoylcarnitine
Octanoylcarnitine [Caprylylcarnitine]
Glutamine
PTC-Glutamine
PTC-Histidine
Lysophosphatidylcholine with acyl residue C14:0
Lysophosphatidylcholine with acyl residue C26:0
Lysophosphatidylcholine with acyl residue C26:1
Lysophosphatidylcholine with acyl residue C28:1
Phosphatidylcholine with diacyl residue sum C26:0
Phosphatidylcholine with diacyl residue sum C32:2
Phosphatidylcholine with diacyl residue sum C36:0
Phosphatidylcholine with diacyl residue sum C38:0
Phosphatidylcholine with diacyl residue sum C42:1
Phosphatidylcholine with diacyl residue sum C42:5
Phosphatidylcholine with diacyl residue sum C42:6
Phosphatidylcholine with acyl-alkyl residue sum C38:4
Phosphatidylcholine with acyl-alkyl residue sum C40:0
Phosphatidylcholine with acyl-alkyl residue sum C42:0
Phosphatidylcholine with acyl-alkyl residue sum C42:5
Phosphatidylcholine with acyl-alkyl residue sum C44:6
Sphingomyeline with acyl residue sum C16:0
Sphingomyeline with acyl residue sum C16:1
Sphingomyeline with acyl residue sum C20:2
Sphingomyeline with acyl residue sum C24:0
Hydroxysphingomyelin with acyl residue sum C24:1
Valine
PTC-Valine
wherein the designation "residue Cn:m" or "residue sum Cn:m" represents the chain length of the acyl/alkyl residue(s), n represents the number of total carbon atoms in the acyl/alkyl residue(s), and m represents the number of total double bonds in the residue(s).

10. Method according to anyone of claims 1 to 8, wherein said endogenous reference metabolites show stability in accordance with all statistical stability measures being selected from the group consisting of coefficient of variation (CV) of raw intensity data, standard deviation (SD) of logarithmic intensity data, stability measure (M) of geNorm-algorithm or stability measure value (rho) of NormFinder-algorithm, and/or such endogenous reference metabolites being in particular Phosphatidylcholine with diacyl residue sum C42:6, wherein the designation "C42:6" represents the chain length of the acyl residues, 42 represents the number of total carbon atoms in the acyl residues, and 6 represents the number of total double bonds in the residues.

11. Method according to anyone of claims 1 to 10, wherein said plurality of endogenous metabolites comprises 2 to 80, in particular 2 to 60, preferably 2 to 50, preferred 2 to 30, more preferred 2 to 10, particularly preferred 2 to 10, preferably 3 to 5 endogenous metabolites.

12. Use of a plurality of compounds or derivatives thereof wherein said compounds have a molecular mass less than 1500 Da as endogenous reference metabolites in metabolomics analysis methods, wherein said metabolomics analysis methods comprise the generation of intensity data for the quantitation of endogenous metabolites by mass spectrometry (MS), in particular MS-technologies such as Matrix Assisted Laser Desorption/Ionisation (MALDI), Electro Spray Ionization (ESI), Atmospheric Pressure Chemical Ionization (APCI), ¹H-, ¹³C-and/or ³¹P-Nuclear Magnetic Resonance spectroscopy (NMR), optionally coupled to MS, determination of metabolite concentrations by use of MS-technologies and/or methods coupled to separation, in particular Liquid Chromatography (LC-MS), Gas Chromatography (GC-MS), or Capillary Electrophoresis (CE-MS), wherein said endogenous reference metabolites are selected from the group consisting of:
Amino acids, in particular, arginine, aspartic Acid, citrulline, glutamic acid (glutamate), glutamine, leucine, isoleucine, histidine, ornithine, proline, phenylalanine, serine, tryptophane, tyrosine, valine, kynurenine;
phenylthio carbamyl amino acids (PTC-amino acids), in particular, PCT-arginine, PTC-glutamine, PTC-histidine, PTC-methionine, PTC-ornithine, PTC-phenylalanine, PTC-proline, PTC-serine, PTC-tryptophane, PTC-tyrosine, PTC-valine; dimethylarginine, in particular N,N-dimethyl-L-arginine;
carboxylic acids, namely 15(S)-hydroxy-5Z, 8Z, 11Z, 13E-eicosatetraenoic acid [(5Z,8Z, 11Z, 13E, 15S)-15-Hydroxyicosa-5, 8, 11, 13-tetraenoic acid], succinic acid (succinate);
carnitine; acylcarnitines having from 1 to 20 carbon atoms in the acyl residue; acylcarnitines having from 3 to 20 carbon atoms in the acyl residue and having 1 to 4 double bonds in the acyl residue; acylcarnitines having from 1 to 20 carbon atoms in the acyl residue and having from 1 to 3 OH-groups in the acyl residue; acylcarnitines having from 3 to 20 carbon atoms in the acyl residue with 1 to 4 double bonds and 1 to 3 OH-groups in the acyl residue;
phospholipides, in particular lysophosphatidylcholines (monoacylphosphatidylcholines) having from 1 to 30 carbon atoms in the acyl residue; lysophosphatidylcholines having from 3 to 30 carbon atoms in the acyl residue and having 1 to 6 double bonds in the acyl residue;
phosphatidylcholines (diacylphosphatidylcholines) having a total of from 1 to 50 carbon atoms in the acyl residues; phatidylcholines having a total from 3 to 50 carbon atoms in the acyl residues and having a total of 1 to 8 double bonds in the acyl residues;
sphingolipids, in particular sphingomyelines having a total number of carbon atoms in the acyl chains from 10 to 30; sphingomyelines having a total number of carbon atoms in the acyl chains from 10 to 30 and 1 to 5 double bonds; hydroxysphinogomyelines having a total number of carbon atoms in the acyl residues from 10 to 30;
hydroxysphingoyelines having a total number of carbon atoms in the acyl residues from 10 to 30 and 1 to 5 double bonds;
prostaglandines, namely 6-keto-prostaglandin Flalpha, prostaglandin D2;
putrescine.

13. Use according to claim 12, wherein said endogenous reference metabolites are selected from the group consisting of:
15((S)-hydroxy-5Z, 8Z, 11Z, 13E-eicosatetraenoic acid
6-keto-Prostaglandin Flalpha
asymmetrical Dimethylarginin
Arginine
PTC-Arginine
Aspartic acid
Carnitine (free)
Decanoylcarnitine [Caprylcarnitine] (Fumarylcarnitine)
Decenoylcarnitine
Decadienoylcarnitine
Dodecanoylcarnitine [Laurylcarnitine]
Dodecenoylcarnitine
Dodecanedioylcarnitine
Tetradecanoylcarnitine
Tetradecenoylcarnitine [Myristoleylcarnitine]
3-Hydroxytetradecenoylcarnitine [3-Hydroxymyristoleylcarnitine]
3-Hydroxytetradecadienoylcarnitine
3-Hydroxytetradecanoylcarnitine [Hydroxymyristylcarnitine]
Hexadecenoylcarnitine [Palmitoleylcarnitine]
3-Hydroxyhexadecenoylcarnitine [3-Hydroxypalmitoleylcarnitine]
Hexadecadienoylcarnitine
3-Hydroxyhexadecadienoylcarnitine
3-Hydroxyhexadecanolycarnitine [3-Hydroxypalmitoylcarnitine]
Octadecanoylcarnitine [Stearylcarnitine]
Octadecenoylcarnitine [Oleylcarnitine]
3-Hydroxyoctadecenoylcarnitine [3-Hydroxyoleylcarnitine]
Acetylcarnitine
Propenoylcarnitine
Hydroxypropionylcarnitine
Butenoylcarnitine
3-Hydroxybutyrylcarnitine / Malonylcarnitine
Isovalerylcarnitine / 2-Methylbutyrylcarnitine / Valerylcarnitine
Tiglylcarnitine / 3-Methyl-crotonylcarnitine
Glutarylcarnitine / Hydroxycaproylcarnitine
Glutarylcarnitine / Hydroxycaproylcarnitine
Methylglutarylcarnitine
3-Hydroxyisovalerylcarnitine / 3-Hydroxy-2-methylbutyryl
Hexanoylcarnitine [Caproylcarnitine]
Hexenoylcarnitine
Pimelylcarnitine
Octanoylcarnitine [Caprylylcarnitine]
Octenoylcarnitine
Nonanoylcarnitine [Pelargonylcarnitine]
Citrulline
Creatinine
Glutamine
PTC-Glutamine
Glutamate
Histidine
PTC-Histidine
Kynurenine
Leucine
Lysophosphatidylcholine with acyl residue C14:0
Lysophosphatidylcholine with acyl residue C16:0
Lysophosphatidylcholine with acyl residue C16:1
Lysophosphatidylcholine with acyl residue C18:0
Lysophosphatidylcholine with acyl residue C 18:1
Lysophosphatidylcholine with acyl residue C18:2
Lysophosphatidylcholine with acyl residue C20:3
Lysophosphatidylcholine with acyl residue C20:4
Lysophosphatidylcholine with acyl residue C24:0
Lysophosphatidylcholine with acyl residue C26:0
Lysophosphatidylcholine with acyl residue C26:1
Lysophosphatidylcholine with acyl residue C28:0
Lysophosphatidylcholine with acyl residue C28:1
Lysophosphatidylcholine with acyl residue C6:0
PTC-Methionine
Ornithine
PTC-Ornithine
Phosphatidylcholine with diacyl residue sum C24:0
Phosphatidylcholine with diacyl residue sum C26:0
Phosphatidylcholine with diacyl residue sum C28:1
Phosphatidylcholine with diacyl residue sum C30:0
Phosphatidylcholine with diacyl residue sum C30:2
Phosphatidylcholine with diacyl residue sum C32:0
Phosphatidylcholine with diacyl residue sum C32:1
Phosphatidylcholine with diacyl residue sum C32:2
Phosphatidylcholine with diacyl residue sum C32:3
Phosphatidylcholine with diacyl residue sum C34:1
Phosphatidylcholine with diacyl residue sum C34:2
Phosphatidylcholine with diacyl residue sum C34:3
Phosphatidylcholine with diacyl residue sum C34:4
Phosphatidylcholine with diacyl residue sum C36:0
Phosphatidylcholine with diacyl residue sum C36:1
Phosphatidylcholine with diacyl residue sum C36:2
Phosphatidylcholine with diacyl residue sum C36:3
Phosphatidylcholine with diacyl residue sum C36:4
Phosphatidylcholine with diacyl residue sum C36:5
Phosphatidylcholine with diacyl residue sum C36:6
Phosphatidylcholine with diacyl residue sum C38:0
Phosphatidylcholine with diacyl residue sum C38:1
Phosphatidylcholine with diacyl residue sum C38:3
Phosphatidylcholine with diacyl residue sum C38:4
Phosphatidylcholine with diacyl residue sum C38:5
Phosphatidylcholine with diacyl residue sum C38:6
Phosphatidylcholine with diacyl residue sum C42:1
Phosphatidylcholine with diacyl residue sum C40:3
Phosphatidylcholine with diacyl residue sum C40:4
Phosphatidylcholine with diacyl residue sum C40:5
Phosphatidylcholine with diacyl residue sum C40:6
Phosphatidylcholine with diacyl residue sum C42:0
Phosphatidylcholine with diacyl residue sum C42:1
Phosphatidylcholine with diacyl residue sum C42:2
Phosphatidylcholine with diacyl residue sum C42:4
Phosphatidylcholine with diacyl residue sum C42:5
Phosphatidylcholine with diacyl residue sum C42:6
Phosphatidylcholine with acyl-alkyl residue sum C30:0
Phosphatidylcholine with acyl-alkyl residue sum C30:1
Phosphatidylcholine with acyl-alkyl residue sum C34:0
Phosphatidylcholine with acyl-alkyl residue sum C34:1
Phosphatidylcholine with acyl-alkyl residue sum C34:2
Phosphatidylcholine with acyl-alkyl residue sum C34:3
Phosphatidylcholine with acyl-alkyl residue sum C36:0
Phosphatidylcholine with acyl-alkyl residue sum C36:1
Phosphatidylcholine with acyl-alkyl residue sum C36:2
Phosphatidylcholine with acyl-alkyl residue sum C36:3
Phosphatidylcholine with acyl-alkyl residue sum C36:4
Phosphatidylcholine with acyl-alkyl residue sum C38:0
Phosphatidylcholine with acyl-alkyl residue sum C38:1
Phosphatidylcholine with acyl-alkyl residue sum C38:2
Phosphatidylcholine with acyl-alkyl residue sum C38:3
Phosphatidylcholine with acyl-alkyl residue sum C38:4
Phosphatidylcholine with acyl-alkyl residue sum C38:5
Phosphatidylcholine with acyl-alkyl residue sum C38:6
Phosphatidylcholine with acyl-alkyl residue sum C40:0
Phosphatidylcholine with acyl-alkyl residue sum C40:1
Phosphatidylcholine with acyl-alkyl residue sum C40:2
Phosphatidylcholine with acyl-alkyl residue sum C40:3
Phosphatidylcholine with acyl-alkyl residue sum C40:4
Phosphatidylcholine with acyl-alkyl residue sum C40:5
Phosphatidylcholine with acyl-alkyl residue sum C40:6
Phosphatidylcholine with acyl-alkyl residue sum C42:0
Phosphatidylcholine with acyl-alkyl residue sum C42:1
Phosphatidylcholine with acyl-alkyl residue sum C42:3
Phosphatidylcholine with acyl-alkyl residue sum C42:4
Phosphatidylcholine with acyl-alkyl residue sum C42:5
Phosphatidylcholine with acyl-alkyl residue sum C44:5
Phosphatidylcholine with acyl-alkyl residue sum C44:6
Prostaglandin D2
Phenylalanine
PTC-Phenylalanine
Proline
PTC-Proline
Putrescine
Serine
PTC-Serine
Hydroxysphingomyeline with acyl residue sum C14:1
Hydroxysphingomyeline with acyl residue sum C16:1
Hydroxysphingomyeline with acyl residue sum C22:1
Hydroxysphingomyeline with acyl residue sum C22:2
Hydroxysphingomyelin with acyl residue sum C24:1
Sphingomyeline with acyl residue sum C16:0
Sphingomyeline with acyl residue sum C16:1
Sphingomyeline with acyl residue sum C18:0
Sphingomyeline with acyl residue sum C 18:1
Sphingomyeline with acyl residue sum C20:2
Sphingomyeline with acyl residue sum C22:3
Sphingomyeline with acyl residue sum C24:0
Sphingomyeline with acyl residue sum C24:1
Sphingomyeline with acyl residue sum C26:0
Sphingomyeline with acyl residue sum C26: 1
Succinic acid (succinate)
Total dimethylarginine: sum ADMA + SDMA
Tryptophan
PTC-Tryptophan
Tyrosine
Valine
PTC-Valine
Leucine + Isoleucine
wherein the designation "residue Cn:m" or "residue sum Cn:m" represents the chain length of the acyl/alkyl residue(s), n represents the number of total carbon atoms in the acyl/alkyl residue(s), and m represents the number of total double bonds in the residue(s).

14. Use according to claim 13, wherein said endogenous reference metabolites show stability in accordance with statistical stability measures being selected from the group consisting of coefficient of variation (CV) of raw intensity data, standard deviation (SD) of logarithmic intensity data, stability measure (M) of geNorm-algorithm or stability measure value (rho) of NormFinder-algorithm.

15. Use according to anyone of claims 12 to 14, wherein said endogenous reference metabolites show stability in accordance with at least 2 statistical stability measures being selected from the group consisting of coefficient of variation (CV) of raw intensity data, standard deviation (SD) of logarithmic intensity data, stability measure (M) of geNorm-algorithm or stability measure value (rho) of NormFinder-algorithm, and/or such endogenous reference metabolites being in particular selected from the group consisting of:
PTC-Arginine
Carnitine (free)
Decenoylcarnitine
Decanoylcarnitine [Caprylcarnitine] (Fumarylcarnitine)
Dodecenoylcarnitine
Dodecanedioylcarnitine
Dodecanoylcarnitine [Laurylcarnitine]
Tetradecanoylcarnitine
3-Hydroxytetradecanoylcarnitine [Hydroxymyristylcarnitine]
Hexadecenoylcarnitine [Palmitoleylcarnitine]
3-Hydroxyhexadecenoylcarnitine [3-Hydroxypalmitoleylcarnitine]
3-Hydroxyhexadecadienoylcarnitine
3-Hydroxyhexadecanolycarnitine [3-Hydroxypalmitoylcarnitine]
3-Hydroxyoctadecenoylcarnitine [3-Hydroxyoleylcarnitine]
Propenoylcarnitine
Hydroxypropionylcarnitine
3-Hydroxybutyrylcarnitine / Malonylcarnitine Methylglutarylcarnitine
3-Hydroxyisovalerylcarnitine / 3-Hydroxy-2-methylbutyryl
Hexenoylcarnitine
Hexanoylcarnitine [Caproylcarnitine]
Pimelylcarnitine
Octenoylcarnitine
Octanoylcarnitine [Caprylylcarnitine]
Glutamine
PTC-Glutamine
PTC-Histidine
Lysophosphatidylcholine with acyl residue C14:0
Lysophosphatidylcholine with acyl residue C26:0
Lysophosphatidylcholine with acyl residue C26:1
Lysophosphatidylcholine with acyl residue C28:0
Lysophosphatidylcholine with acyl residue C28:1
Phosphatidylcholine with diacyl residue sum C24:0
Phosphatidylcholine with diacyl residue sum C26:0
Phosphatidylcholine with diacyl residue sum C30:0
Phosphatidylcholine with diacyl residue sum C30:2
Phosphatidylcholine with diacyl residue sum C32:2
Phosphatidylcholine with diacyl residue sum C34:2
Phosphatidylcholine with diacyl residue sum C36:0
Phosphatidylcholine with diacyl residue sum C36:2
Phosphatidylcholine with diacyl residue sum C36:4
Phosphatidylcholine with diacyl residue sum C38:0
Phosphatidylcholine with diacyl residue sum C38:1
Phosphatidylcholine with diacyl residue sum C42:1
Phosphatidylcholine with diacyl residue sum C42:0
Phosphatidylcholine with diacyl residue sum C42:5
Phosphatidylcholine with diacyl residue sum C42:6
Phosphatidylcholine with acyl-alkyl residue sum C30:1
Phosphatidylcholine with acyl-alkyl residue sum C34:1
Phosphatidylcholine with acyl-alkyl residue sum C36:0
Phosphatidylcholine with acyl-alkyl residue sum C38:1
Phosphatidylcholine with acyl-alkyl residue sum C38:4
Phosphatidylcholine with acyl-alkyl residue sum C38:6
Phosphatidylcholine with acyl-alkyl residue sum C40:0
Phosphatidylcholine with acyl-alkyl residue sum C40:1
Phosphatidylcholine with acyl-alkyl residue sum C40:5
Phosphatidylcholine with acyl-alkyl residue sum C40:6
Phosphatidylcholine with acyl-alkyl residue sum C42:0
Phosphatidylcholine with acyl-alkyl residue sum C42:5
Phosphatidylcholine with acyl-alkyl residue sum C44:6
Phenylalanine
PTC-Phenylalanine
Proline
Sphingomyeline with acyl residue sum C16:0
Sphingomyeline with acyl residue sum C16:1
Sphingomyeline with acyl residue sum C18:0
Sphingomyeline with acyl residue sum C18:1
Sphingomyeline with acyl residue sum C20:2
Sphingomyeline with acyl residue sum C24:0
Sphingomyeline with acyl residue sum C24:1
Hydroxysphingomyeline with acyl residue sum C14:1
Hydroxysphingomyelin with acyl residue sum C16:1
Hydroxysphingomyeline with acyl residue sum C22:2
Hydroxysphingomyeline with acyl residue sum C24:1
Succinic acid (succinate)
PTC-Tryptophan
Valine
PTC-Valine
Leucine + Isoleucine
wherein the designation "residue Cn:m" or "residue sum Cn:m" represents the chain length of the acyl/alkyl residue(s), n represents the number of total carbon atoms in the acyl/alkyl residue(s), and m represents the number of total double bonds in the residue(s).

16. Use according to anyone of claims 12 to 15, wherein said endogenous reference metabolites show stability in accordance with at least 3 statistical stability measures being selected from the group consisting of coefficient of variation (CV) of raw intensity data, standard deviation (SD) of logarithmic intensity data, stability measure (M) of geNorm-algorithm or stability measure value (rho) of NormFinder-algorithm, and/or such endogenous reference metabolites being in particular selected from the group consisting of:
Carnitine (free)
Decanoylcarnitine [Caprylcarnitine] (Fumarylcarnitine)
Dodecanedioylcarnitine
Dodecanoylcarnitine [Laurylcarnitine]
Hexadecenoylcarnitine [Palmitoleylcarnitine]
3-Hydroxyhexadecenoylcarnitine [3-Hydroxypalmitoleylcarnitine]
3-Hydroxyhexadecanolycarnitine [3-Hydroxypalmitoylcarnitine]
Propenoylcarnitine
Hydroxypropionylcarnitine
3-Hydroxybutyrylcarnitine / Malonylcarnitine
Methylglutarylcarnitine
Hexanoylcarnitine [Caproylcarnitine]
Pimelylcarnitine
Octenoylcarnitine
Octanoylcarnitine [Caprylylcarnitine]
Glutamine
PTC-Glutamine
PTC-Histidine
Lysophosphatidylcholine with acyl residue C14:0
Lysophosphatidylcholine with acyl residue C26:0
Lysophosphatidylcholine with acyl residue C26:1
Lysophosphatidylcholine with acyl residue C28:1
Phosphatidylcholine with diacyl residue sum C26:0
Phosphatidylcholine with diacyl residue sum C32:2
Phosphatidylcholine with diacyl residue sum C36:0
Phosphatidylcholine with diacyl residue sum C38:0
Phosphatidylcholine with diacyl residue sum C42:1
Phosphatidylcholine with diacyl residue sum C42:5
Phosphatidylcholine with diacyl residue sum C42:6
Phosphatidylcholine with acyl-alkyl residue sum C38:4
Phosphatidylcholine with acyl-alkyl residue sum C40:0
Phosphatidylcholine with acyl-alkyl residue sum C42:0
Phosphatidylcholine with acyl-alkyl residue sum C42:5
Phosphatidylcholine with acyl-alkyl residue sum C44:6
Sphingomyeline with acyl residue sum C16:0
Sphingomyeline with acyl residue sum C16:1
Sphingomyeline with acyl residue sum C20:2
Sphingomyeline with acyl residue sum C24:0
Hydroxysphingomyelin with acyl residue sum C24:1
Valine
PTC-Valine
wherein the designation "residue Cn:m" or "residue sum Cn:m" represents the chain length of the acyl/alkyl residue(s), n represents the number of total carbon atoms in the acyl/alkyl residue(s), and m represents the number of total double bonds in the residue(s).

17. Use according to anyone of claims 12 to 16, wherein said endogenous reference metabolites show stability in accordance with all statistical stability measures being selected from the group consisting of coefficient of variation (CV) of raw intensity data, standard deviation (SD) of logarithmic intensity data, stability measure (M) of geNorm-algorithm or stability measure value (rho) of NormFinder-algorithm, and/or such endogenous reference metabolites being in particular Phosphatidylcholine with diacyl residue sum C42:6, wherein the designation "C42:6" represents the chain length of the acyl residues, 42 represents the number of total carbon atoms in the acyl residues, and 6 represents the number of total double bonds in the residues.

18. Use according to anyone of claims 12 to 17, wherein said plurality of endogenous metabolites comprises 2 to 80, in particular 2 to 60, preferably 2 to 50, preferred 2 to 30, more preferred 2 to 10, particularly preferred 2 to 10, preferably 3 to 5 endogenous metabolites.

## Patentansprüche

1. Verfahren zur Normalisierung von Intensitätsdaten, die Mengen und/oder Konzentrationen ausgewählter Target-Metaboliten in einer biologischen Probe eines zur Gattung Mammalia gehörenden Probanden entsprechen, wobei die Intensitätsdaten anhand eines Metabolomikanalyseverfahrens erhalten werden, das umfasst: Generieren von Intensitätsdaten für die Quantifizierung endogener Metaboliten durch Massenspektrometrie (MS), insbesondere MS-Technologien wie Matrix-unterstützte Laser-Desorption/Ionisation (MALDI), Elektrospray-Ionisation (ESI), chemische Ionisation bei Atmosphärendruck (APCI), ¹H-, ¹³C-und/oder ³¹P-Kernspinresonanz-Spektroskopie (NMR), optional mit MS gekoppelt, Bestimmen von Metabolitenkonzentrationen unter Verwendung von MS-Technologien und/oder -Verfahren, die mit Trennung gekoppelt sind, insbesondere mit Flüssigchromatographie (GC-MS) oder kapillarer Elektrophorese (CE-MS), mit mehreren endogenen Referenz-Metaboliten, umfassend:
Durchführen mindestens eines in-vitro-Metabolomikanalyseverfahrens an den ausgewählten Target-Metaboliten in der biologischen Probe;
gleichzeitiges Ausführen einer quantitativen Analyse mehrerer endogener Referenz-Metaboliten oder von Derivaten davon in derselben Probe, wobei die endogenen Referenz-Metaboliten solche Verbindungen in der biologischen Probe sind, die in dem Probanden auf einem im Wesentlichen konstanten Spiegel vorhanden sind; und wobei die endogenen Referenz-Metaboliten oder deren Derivate eine Molekülmasse von weniger als 1500 Da aufweisen und ausgewählt werden aus der Gruppe bestehend aus:
Aminosäuren, insbesondere Arginin, Aspartamsäure, Citrullin, Glutaminsäure (Glutamat), Glutamin, Leucin, Isoleucin, Histidin, Ornithin, Prolin;
Phenylalanin, Serin, Tryptophan, Tyrosin, Valin, Kynurenin;
Phenylthiocarbamylaminosäuren (PTC-Aminosäuren), insbesondere PCT-Arginin, PTC-Glutamin, PTC-Histidin, PTC-Methionin, PTC-Ornithin) PTC-Phenylalanin, PTC-Prolin, PTC-Serin, PTC-Tryptophan, PTC-Tyrosin, PTC-Valin;
Dimethylarginin, insbesondere N, N-Dimethyl-L-arginin;
Carbonsäuren, und zwar 15(S)-Hydroxy-5Z,8Z,11Z,13E-eicosatetraensäure [(5Z,8Z,11Z,13E,15S)-15-Hydroxyicosa-5,8,11,13-tetraensäure], Bernsteinsäure (Succinat);
Carnitin; Acylcarnitinen mit 1 bis 20 Kohlenstoffatomen im Acylrest; Acylcarnitinen mit 3 bis 20 Kohlenstoffatomen im Acylrest und mit 1 bis 4 Doppelbindungen im Acylrest; Acylcarnitinen mit 1 bis 20 Kohlenstoffatomen im Acylrest und mit 1 bis 3 OH-Gruppen im Acylrest; Acylcarnitinen mit 3 bis 20 Kohlenstoffatomen im Acylrest mit 1 bis 4 Doppelbindungen und 1 bis 3 OH-Gruppen im Acylrest;
Phospholipiden, insbesondere
Lysophosphatidylcholinen (Monoacylphosphatidylcholinen) mit 1 bis 30 Kohlenstoffatomen im Acylrest; Lysophosphatidylcholinen mit 3 bis 30 Kohlenstoffatomen im Acylrest und mit 1 bis 6 Doppelbindungen im Acylrest;
Phosphatidylcholinen (Diacylphosphatidylcholinen) mit insgesamt 1 bis 50 Kohlenstoffatomen in den Acylresten; Phatidylcholinen mit insgesamt 3 bis 50 Kohlenstoffatomen in den Acylresten und mit insgesamt 1 bis 8 Doppelbindungen in den Acylresten;
Sphingolipiden, insbesondere
Sphingomyelinen mit einer Gesamtzahl von Kohlenstoffatomen in den Acylketten von 10 bis 30; Sphingomyelinen mit einer Gesamtzahl von Kohlenstoffatomen in den Acylketten von 10 bis 30 und 1 bis 5 Doppelbindungen; Hydroxysphinogomyelinen mit einer Gesamtzahl von Kohlenstoffatomen in den Acylresten von 10 bis 30; Hydroxysphingomyelinen mit einer Gesamtzahl von Kohlenstoffatomen in den Acylresten von 10 bis 30 und 1 bis 5 Doppelbindungen;
Prostaglandinen, und zwar 6-keto-Prostaglandin F1 alpha, Prostaglandin D2;
Putrescin;
und wobei
die erfassten Intensitäten der ausgewählten Target-Metaboliten jeweils mit den Intensitäten der endogenen Referenz-Metaboliten in Beziehung stehen.

2. Verfahren nach Anspruch 1, wobei die mehreren Intensitäten der Target- und der endogenen Referenz-Metaboliten einer mathematischen Vorverarbeitung unterzogen werden, insbesondere Transformationen wie der Anwendung von Logarithmen, generalisierten Logarithmen, Leistungstransformationen.

3. Verfahren nach Anspruch 1 oder 2, wobei die mehreren Intensitäten der endogenen Referenz-Metaboliten in einem Referenzwert zusammengefasst werden.

4. Verfahren nach Anspruch 3, wobei die mehreren Intensitäten der endogenen Referenz-Metaboliten durch Berechnen eines geometrischen Mittelwerts, eines arithmetischen Mittelwerts, eines Medianwerts, eines gewichteten arithmetischen Mittelwerts zusammengefasst werden.

5. Verfahren nach irgendeinem der Ansprüche 1 bis 4, wobei im Falle von linearen Intensitäten ein Verhältnis von den einzelnen Intensitäten der Target-Metaboliten und dem bestimmten Referenzwert gebildet wird oder, im Falle von logarithmischen Intensitäten, der bestimmte Referenzwert von den einzelnen Target-Metaboliten subtrahiert wird.

6. Verfahren nach einem der Ansprüche 1 bis 5, wobei die endogenen Referenz-Metaboliten ausgewählt werden aus der Gruppe bestehend aus:
15((S)-Hydroxy-5Z,8Z,11Z,13E-eicosatetraensäure
6-keto-Prostaglandin-F1 alpha
asymmetrischem Dimethylarginin
Arginin
PTC-Arginin
Aspartamsäure
Carnitin (frei)
Decanoylcarnitin [Caprylcarnitin] (Fumarylcarnitin)
Decenoylcarnitin
Decadienoylcarnitin
Dodecanoylcarnitin [Laurylcarnitin]
Dodecenoylcarnitin
Dodecandioylcarnitin
Tetradecanoylcarnitin
Tetradecenoylcarnitin [Myristoleylcarnitin]
3-Hydroxytetradecenoylcarnitin [3-Hydroxymyristoleylcarnitin]
3-Hydroxytetradecadienoylcarnitin
3-Hydroxytetradecanoylcarnitin [Hydroxymyristylcarnitin]
Hexadecenoylcarnitin [Palmitoleylcarnitin]
3-Hydroxyhexadecenoylcarnitin [3-Hydroxypalmitoleylcarnitin]
Hexadecadienoylcarnitin
3-Hydroxyhexadecadienoylcarnitin
3-Hydroxyhexadecanolycarnitin [3-Hydroxypalmitoylcarnitin]
Octadecanoylcarnitin [Stearylcarnitin]
Octadecenoylcarnitin [Oleylcarnitin]
3-Hydroxyoctadecenoylcarnitin [3-Hydroxyoleylcarnitin]
Acetylcarnitin
Propenoylcarnitin
Hydroxypropionylcarnitin
Butenoylcarnitin
3-Hydroxybutyrylcarnitin / Malonylcarnitin
Isovalerylcarnitin / 2-Methylbutyrylcarnitin / Valerylcarnitin
Tiglylcarnitin / 3-Methylcrotonylcarnitin
Glutarylcarnitin / Hydroxycaproylcarnitin
Glutarylcarnitin / Hydroxycaproylcarnitin
Methylglutarylcarnitin
3-Hydroxyisovalerylcarnitin / 3-Hydroxy-2-methylbutyryl
Hexanoylcarnitin [Caproylcarnitin]
Hexenoylcarnitin
Pimelylcarnitin
Octanoylcarnitin [Caprylylcarnitin]
Octenoylcarnitin
Nonanoylcarnitin [Pelargonylcarnitin]
Citrullin
Creatinin
Glutamin
PTC-Glutamin
Glutamat
Histidin
PTC-Histidin
Kynurenin
Leucin
Lysophosphatidylcholin mit Acylrest C14:0
Lysophosphatidylcholin mit Acylrest C16:0
Lysophosphatidylcholin mit Acylrest C16:1
Lysophosphatidylcholin mit Acylrest C18:0
Lysophosphatidylcholin mit Acylrest C18:1
Lysophosphatidylcholin mit Acylrest C18:2
Lysophosphatidylcholin mit Acylrest C20:3
Lysophosphatidylcholin mit Acylrest C20:4
Lysophosphatidylcholin mit Acylrest C24:0
Lysophosphatidylcholin mit Acylrest C26:0
Lysophosphatidylcholin mit Acylrest C26:1
Lysophosphatidylcholin mit Acylrest C28:0
Lysophosphatidylcholin mit Acylrest C28:1
Lysophosphatidylcholin mit Acylrest C6:0
PTC-Methionin
Ornithin
PTC-Ornithin
Phosphatidylcholin mit Diacylrestsumme C24:0
Phosphatidylcholin mit Diacylrestsumme C26:0
Phosphatidylcholin mit Diacylrestsumme C28:1
Phosphatidylcholin mit Diacylrestsumme C30:0
Phosphatidylcholin mit Diacylrestsumme C30:2
Phosphatidylcholin mit Diacylrestsumme C32:0
Phosphatidylcholin mit Diacylrestsumme C32:1
Phosphatidylcholin mit Diacylrestsumme C32:2
Phosphatidylcholin mit Diacylrestsumme C32:3
Phosphatidylcholin mit Diacylrestsumme C34:1
Phosphatidylcholin mit Diacylrestsumme C34:2
Phosphatidylcholin mit Diacylrestsumme C34:3
Phosphatidylcholin mit Diacylrestsumme C34:4
Phosphatidylcholin mit Diacylrestsumme C36:0
Phosphatidylcholin mit Diacylrestsumme C36:1
Phosphatidylcholin mit Diacylrestsumme C36:2
Phosphatidylcholin mit Diacylrestsumme C36:3
Phosphatidylcholin mit Diacylrestsumme C36:4
Phosphatidylcholin mit Diacylrestsumme C36:5
Phosphatidylcholin mit Diacylrestsumme C36:6
Phosphatidylcholin mit Diacylrestsumme C38:0
Phosphatidylcholin mit Diacylrestsumme C38:1
Phosphatidylcholin mit Diacylrestsumme C38:3
Phosphatidylcholin mit Diacylrestsumme C38:4
Phosphatidylcholin mit Diacylrestsumme C38:5
Phosphatidylcholin mit Diacylrestsumme C38:6
Phosphatidylcholin mit Diacylrestsumme C42:1
Phosphatidylcholin mit Diacylrestsumme C40:3
Phosphatidylcholin mit Diacylrestsumme C40:4
Phosphatidylcholin mit Diacylrestsumme C40:5
Phosphatidylcholin mit Diacylrestsumme C40:6
Phosphatidylcholin mit Diacylrestsumme C42:0
Phosphatidylcholin mit Diacylrestsumme C42:1
Phosphatidylcholin mit Diacylrestsumme C42:2
Phosphatidylcholin mit Diacylrestsumme C42:4
Phosphatidylcholin mit Diacylrestsumme C42:5
Phosphatidylcholin mit Diacylrestsumme C42:6
Phosphatidylcholin mit Acyl-Alkylrestsumme C30:0
Phosphatidylcholin mit Acyl-Alkylrestsumme C30:1
Phosphatidylcholin mit Acyl-Alkylrestsumme C34:0
Phosphatidylcholin mit Acyl-Alkylrestsumme C34:1
Phosphatidylcholin mit Acyl-Alkylrestsumme C34:2
Phosphatidylcholin mit Acyl-Alkylrestsumme C34:3
Phosphatidylcholin mit Acyl-Alkylrestsumme C36:0
Phosphatidylcholin mit Acyl-Alkylrestsumme C36:1
Phosphatidylcholin mit Acyl-Alkylrestsumme C36:2
Phosphatidylcholin mit Acyl-Alkylrestsumme C36:3
Phosphatidylcholin mit Acyl-Alkylrestsumme C36:4
Phosphatidylcholin mit Acyl-Alkylrestsumme C38:0
Phosphatidylcholin mit Acyl-Alkylrestsumme C38:1
Phosphatidylcholin mit Acyl-Alkylrestsumme C38:2
Phosphatidylcholin mit Acyl-Alkylrestsumme C38:3
Phosphatidylcholin mit Acyl-Alkylrestsumme C38:4
Phosphatidylcholin mit Acyl-Alkylrestsumme C38:5
Phosphatidylcholin mit Acyl-Alkylrestsumme C38:6
Phosphatidylcholin mit Acyl-Alkylrestsumme C40:0
Phosphatidylcholin mit Acyl-Alkylrestsumme C40:1
Phosphatidylcholin mit Acyl-Alkylrestsumme C40:2
Phosphatidylcholin mit Acyl-Alkylrestsumme C40:3
Phosphatidylcholin mit Acyl-Alkylrestsumme C40:4
Phosphatidylcholin mit Acyl-Alkylrestsumme C40:5
Phosphatidylcholin mit Acyl-Alkylrestsumme C40:6
Phosphatidylcholin mit Acyl-Alkylrestsumme C42:0
Phosphatidylcholin mit Acyl-Alkylrestsumme C42:1
Phosphatidylcholin mit Acyl-Alkylrestsumme C42:3
Phosphatidylcholin mit Acyl-Alkylrestsumme C42:4
Phosphatidylcholin mit Acyl-Alkylrestsumme C42:5
Phosphatidylcholin mit Acyl-Alkylrestsumme C44:5
Phosphatidylcholin mit Acyl-Alkylrestsumme C44:6
Prostaglandin D2
Phenylalanin
PTC-Phenylalanin
Prolin
PTC-Prolin
Putrescin
Serin
PTC-Serin
Hydroxysphingomyelin mit Acylrestsumme C14:1
Hydroxysphingomyelin mit Acylrestsumme C16:1
Hydroxysphingomyelin mit Acylrestsumme C22:1
Hydroxysphingomyelin mit Acylrestsumme C22:2
Hydroxysphingomyelin mit Acylrestsumme C24:1
Sphingomyelin mit Acylrestsumme C16:0
Sphingomyelin mit Acylrestsumme C16:1
Sphingomyelin mit Acylrestsumme C18:0
Sphingomyelin mit Acylrestsumme C18:1
Sphingomyelin mit Acylrestsumme C20:2
Sphingomyelin mit Acylrestsumme C22:3
Sphingomyelin mit Acylrestsumme C24:0
Sphingomyelin mit Acylrestsumme C24:1
Sphingomyelin mit Acylrestsumme C26:0
Sphingomyelin mit Acylrestsumme C26:1
Bernsteinsäure (Succinat)
Gesamt-Dimethylarginin: Summe ADMA + SDMA
Tryptophan
PTC-Tryptophan
Tyrosin
Valin
PTC-Valin
Leucin + Isoleucin
wobei die Bezeichnung "-rest Cn:m" oder "-restsumme Cn:m" die Kettenlänge des einen bzw. der mehreren Acyl-/Alkylreste darstellt, n die Anzahl der Kohlenstoffatome in dem einen bzw. den mehreren Acyl-/Alkylresten darstellt und m die Gesamtzahl der Doppelbindungen in dem einen bzw. den mehreren Resten darstellt.

7. Verfahren nach irgendeinem der Ansprüche 1 bis 6, wobei solche Metaboliten als endogene Referenz-Metaboliten verwendet werden, die sich gemäß statistischen Stabilitätsmessungen, die ausgewählt werden aus der Gruppe bestehend aus Variationskoeffizient (CV) von Intensitätsrohdaten, Standardabweichung (SD) von logarithmischen Intensitätsdaten, Stabilitätsmessung (M) von GeNorm-Algorithmus oder Stabilitätsmesswert (rho) von NormFinder-Algorithmus, als stabil erweisen.

8. Verfahren nach einem der Ansprüche 1 bis 7, wobei sich die endogenen Referenz-Metaboliten gemäß mindestens 2 statistischen Stabilitätsmessungen, die ausgewählt werden aus der Gruppe bestehend aus Variationskoeffizient (CV) von Intensitätsrohdaten, Standardabweichung (SD) von logarithmischen Intensitätsdaten, Stabilitätsmessung (M) von GeNorm-Algorithmus oder Stabilitätsmesswert (rho) von NormFinder-Algorithmus als stabil erweisen und/oder diese endogenen Referenz-Metaboliten insbesondere ausgewählt werden aus der Gruppe bestehend aus:
PTC-Arginin
Carnitin (frei)
Decenoylcarnitin
Decanoylcarnitin [Caprylcarnitin] (Fumarylcarnitin)
Dodecenoylcarnitin
Dodecandioylcarnitin
Dodecanoylcarnitin [Laurylcarnitin]
Tetradecanoylcarnitin
3-Hydroxytetradecanoylcarnitin [Hydroxymyristylcarnitin]
Hexadecenoylcarnitin [Palmitoleylcarnitin]
3-Hydroxyhexadecenoylcarnitin [3-Hydroxypalmitoleylcarnitin]
3-Hydroxyhexadecadienoylcarnitin
3-Hydroxyhexadecanolycarnitin [3-Hydroxypalmitoylcarnitin]
3-Hydroxyoctadecenoylcarnitin [3-Hydroxyoleylcarnitin]
Propenoylcarnitin
Hydroxypropionylcarnitin
3-Hydroxybutyrylcarnitin *I* Malonylcarnitin
Methylglutarylcarnitin
3-Hydroxyisovalerylcarnitin / 3-Hydroxy-2-methylbutyryl
Hexenoylcarnitin
Hexanoylcarnitin [Caproylcarnitin]
Pimelylcarnitin
Octenoylcarnitin
Octanoylcarnitin [Caprylylcarnitin]
Glutamin
PTC-Glutamin
PTC-Histidin
Lysophosphatidylcholin mit Acylrest C14:0
Lysophosphatidylcholin mit Acylrest C26:0
Lysophosphatidylcholin mit Acylrest C26:1
Lysophosphatidylcholin mit Acylrest C28:0
Lysophosphatidylcholin mit Acylrest C28:1
Phosphatidylcholin mit Diacylrestsumme C24:0
Phosphatidylcholin mit Diacylrestsumme C26:0
Phosphatidylcholin mit Diacylrestsumme C30:0
Phosphatidylcholin mit Diacylrestsumme C30:2
Phosphatidylcholin mit Diacylrestsumme C32:2
Phosphatidylcholin mit Diacylrestsumme C34:2
Phosphatidylcholin mit Diacylrestsumme C36:0
Phosphatidylcholin mit Diacylrestsumme C36:2
Phosphatidylcholin mit Diacylrestsumme C36:4
Phosphatidylcholin mit Diacylrestsumme C38:0
Phosphatidylcholin mit Diacylrestsumme C38:1
Phosphatidylcholin mit Diacylrestsumme C42:1
Phosphatidylcholin mit Diacylrestsumme C42:0
Phosphatidylcholin mit Diacylrestsumme C42:5
Phosphatidylcholin mit Diacylrestsumme C42:6
Phosphatidylcholin mit Acyl-Alkylrestsumme C30:1
Phosphatidylcholin mit Acyl-Alkylrestsumme C34:1
Phosphatidylcholin mit Acyl-Alkylrestsumme C36:0
Phosphatidylcholin mit Acyl-Alkylrestsumme C38:1
Phosphatidylcholin mit Acyl-Alkylrestsumme C38:4
Phosphatidylcholin mit Acyl-Alkylrestsumme C38:6
Phosphatidylcholin mit Acyl-Alkylrestsumme C40:0
Phosphatidylcholin mit Acyl-Alkylrestsumme C40:1
Phosphatidylcholin mit Acyl-Alkylrestsumme C40:5
Phosphatidylcholin mit Acyl-Alkylrestsumme C40:6
Phosphatidylcholin mit Acyl-Alkylrestsumme C42:0
Phosphatidylcholin mit Acyl-Alkylrestsumme C42:5
Phosphatidylcholin mit Acyl-Alkylrestsumme C44:6
Phenylalanin
PTC-Phenylalanin
Prolin
Sphingomyelin mit Acylrestsumme C16:0
Sphingomyelin mit Acylrestsumme C16:1
Sphingomyelin mit Acylrestsumme C18:0
Sphingomyelin mit Acylrestsumme C18:1
Sphingomyelin mit Acylrestsumme C20:2
Sphingomyelin mit Acylrestsumme C24:0
Sphingomyelin mit Acylrestsumme C24:1
Hydroxysphingomyelin mit Acylrestsumme C14:1
Hydroxysphingomyelin mit Acylrestsumme C16:1
Hydroxysphingomyelin mit Acylrestsumme C22:2
Hydroxysphingomyelin mit Acylrestsumme C24:1
Bernsteinsäure (Succinat)
PTC-Tryptophan
Valin
PTC-Valin
Leucin + Isoleucin
wobei die Bezeichnung "-rest Cn:m" oder "-restsumme Cn:m" die Kettenlänge des einen bzw. der mehreren Acyl-/Alkylreste darstellt, n die Anzahl der Kohlenstoffatome in dem einen bzw. den mehreren Acyl-/Alkylresten darstellt und m die Gesamtzahl der Doppelbindungen in dem einen bzw. den mehreren Resten darstellt.

9. Verfahren nach einem der Ansprüche 1 bis 8, wobei sich die endogenen Referenz-Metaboliten gemäß mindestens 3 statistischen Stabilitätsmessungen, die ausgewählt werden aus der Gruppe bestehend aus Variationskoeffizient (CV) von Intensitätsrohdaten, Standardabweichung (SD) von logarithmischen Intensitätsdaten, Stabilitätsmessung (M) von GeNorm-Algorithmus oder Stabilitätsmesswert (rho) von NormFinder-Algorithmus als stabil erweisen und/oder diese endogenen Referenz-Metaboliten insbesondere ausgewählt werden aus der Gruppe bestehend aus:
Carnitin (frei)
Decanoylcarnitin [Caprylcarnitin] (Fumarylcarnitin)
Dodecandioylcarnitin
Dodecanoylcarnitin [Laurylcarnitin]
Hexadecenoylcarnitin [Palmitoleylcarnitin]
3-Hydroxyhexadecenoylcarnitin [3-Hydroxypalmitoleylcarnitin]
3-Hydroxyhexadecanolycarnitin [3-Hydroxypalmitoylcarnitin]
Propenoylcarnitin
Hydroxypropionylcarnitin
3-Hydroxybutyrylcarnitin / Malonylcarnitin
Methylglutarylcarnitin
Hexanoylcarnitin [Caproylcarnitin]
Pimelylcarnitin
Octenoylcarnitin
Octanoylcarnitin [Caprylylcarnitin]
Glutamin
PTC-Glutamin
PTC-Histidin
Lysophosphatidylcholin mit Acylrest C14:0
Lysophosphatidylcholin mit Acylrest C26:0
Lysophosphatidylcholin mit Acylrest C26:1
Lysophosphatidylcholin mit Acylrest C28:1
Phosphatidylcholin mit Diacylrestsumme C26:0
Phosphatidylcholin mit Diacylrestsumme C32:2
Phosphatidylcholin mit Diacylrestsumme C36:0
Phosphatidylcholin mit Diacylrestsumme C38:0
Phosphatidylcholin mit Diacylrestsumme C42:1
Phosphatidylcholin mit Diacylrestsumme C42:5
Phosphatidylcholin mit Diacylrestsumme C42:6
Phosphatidylcholin mit Acyl-Alkylrestsumme C38:4
Phosphatidylcholin mit Acyl-Alkylrestsumme C40:0
Phosphatidylcholin mit Acyl-Alkylrestsumme C42:0
Phosphatidylcholin mit Acyl-Alkylrestsumme C42:5
Phosphatidylcholin mit Acyl-Alkylrestsumme C44:6
Sphingomyelin mit Acylrestsumme C16:0
Sphingomyelin mit Acylrestsumme C16:1
Sphingomyelin mit Acylrestsumme C20:2
Sphingomyelin mit Acylrestsumme C24:0
Hydroxysphingomyelin mit Acylrestsumme C24:1
Valin
PTC-Valin
wobei die Bezeichnung "-rest Cn:m" oder "-restsumme Cn:m" die Kettenlänge des einen bzw. der mehreren Acyl-/Alkylreste darstellt, n die Anzahl der Kohlenstoffatome in dem einen bzw. den mehreren Acyl-/Alkylresten darstellt und m die Gesamtzahl der Doppelbindungen in dem einen bzw. den mehreren Resten darstellt.

10. Verfahren nach irgendeinem der Ansprüche 1 bis 8, wobei sich die endogenen Referenz-Metaboliten gemäß allen statistischen Stabilitätsmessungen, die ausgewählt werden aus der Gruppe bestehend aus Variationskoeffizient (CV) von Intensitätsrohdaten, Standardabweichung (SD) von logarithmischen Intensitätsdaten, Stabilitätsmessung (M) von GeNorm-Algorithmus oder Stabilitätsmesswert (rho) von NormFinder-Algorithmus als stabil erweisen und/oder wobei es sich bei diesen endogenen Referenz-Metaboliten insbesondere um Phosphatidylcholin mit einer Diacylrestsumme C42:6 handelt, wobei die Bezeichnung "C42:6" die Kettenlänge der Acylreste darstellt, 42 die Gesamtzahl der Kohlenstoffatome in den Acylresten darstellt und 6 die Gesamtzahl der Doppelbindungen in den Resten darstellt.

11. Verfahren nach einem der Ansprüche 1 bis 10, wobei die mehreren endogenen Metaboliten 2 bis 80, insbesondere 2 bis 60, vorzugsweise 2 bis 50, bevorzugt 2 bis 30, stärker bevorzugt 2 bis 10, besonders bevorzugt 2 bis 10, vorzugsweise 3 bis 5 endogene Metaboliten umfassen.

12. Verwendung mehrerer Verbindungen oder Derivate davon, wobei die Verbindungen eine Molekülmasse von weniger als 1500 Da aufweisen, als endogene Referenz-Metaboliten in Metabolomikanalyseverfahren, wobei die Metabolomikanalyseverfahren umfassen: Generierung von Intensitätsdaten für die Quantifizierung endogener Metaboliten durch Massenspektrometrie (MS), insbesondere MS-Technologien wie Matrix-unterstützte Laser-Desorption/Ionisation (MALDI), Elektrospray-Ionisation (ESI), chemische Ionisation bei Atmosphärendruck (APCI), ¹H-, ¹³C- und/oder ³¹P-Kernspinresonanz-Spektroskopie (NMR), optional mit MS gekoppelt, Bestimmen von Metabolitenkonzentrationen unter Verwendung von MS-Technologien und/oder -Verfahren, die mit Trennung gekoppelt sind, insbesondere mit Flüssigchromatographie (GC-MS) oder kapillarer Elektrophorese (CE-MS), wobei die endogenen Referenz-Metaboliten ausgewählt werden aus der Gruppe bestehend aus:
Aminosäuren, insbesondere Arginin, Aspartamsäure, Citrullin, Glutaminsäure (Glutamat), Glutamin, Leucin, Isoleucin, Histidin, Ornithin, Prolin, Phenylalanin, Serin, Tryptophan, Tyrosin, Valin, Kynurenin;
Phenylthiocarbamylaminosäuren (PTC-Aminosäuren), insbesondere PCT-Arginin, PTC-Glutamin, PTC-Histidin, PTC-Methionin, PTC-Ornithin) PTC-Phenylalanin, PTC-Prolin, PTC-Serin, PTC-Tryptophan, PTC-Tyrosin, PTC-Valin; Dimethylarginin, insbesondere N,N-Dimethyl-L-arginin;
Carbonsäuren, und zwar 15(S)-Hydroxy-5Z,8Z,11Z,13E-eicosatetraensäure [(5Z,8Z,11Z,13E,15S)-15-Hydroxyicosa-5,8,11,13-tetraensäure], Bernsteinsäure (Succinat);
Carnitin; Acylcarnitinen mit 1 bis 20 Kohlenstoffatomen im Acylrest; Acylcarnitinen mit 3 bis 20 Kohlenstoffatomen im Acylrest und mit 1 bis 4 Doppelbindungen im Acylrest; Acylcarnitinen mit 1 bis 20 Kohlenstoffatomen im Acylrest und mit 1 bis 3 OH-Gruppen im Acylrest; Acylcarnitinen mit 3 bis 20 Kohlenstoffatomen im Acylrest mit 1 bis 4 Doppelbindungen und 1 bis 3 OH-Gruppen im Acylrest.
Phospholipiden, insbesondere Lysophosphatidylcholinen (Monoacylphosphatidylcholinen) mit 1 bis 30 Kohlenstoffatomen im Acylrest; Lysophosphatidylcholinen mit 3 bis 30 Kohlenstoffatomen im Acylrest und mit 1 bis 6 Doppelbindungen im Acylrest;
Phosphatidylcholinen (Diacylphosphatidylcholinen) mit insgesamt 1 bis 50 Kohlenstoffatomen in den Acylresten; Phatidylcholinen mit insgesamt 3 bis 50 Kohlenstoffatomen in den Acylresten und mit insgesamt 1 bis 8 Doppelbindungen in den Acylresten;
Sphingolipiden, insbesondere Sphingomyelinen mit einer Gesamtzahl von Kohlenstoffatomen in den Acylketten von 10 bis 30; Sphingomyelinen mit einer Gesamtzahl von Kohlenstoffatomen in den Acylketten von 10 bis 30 und 1 bis 5 Doppelbindungen; Hydroxysphinogomyelinen mit einer Gesamtzahl von Kohlenstoffatomen in den Acylresten von 10 bis 30;
Hydroxysphingomyelinen mit einer Gesamtzahl von Kohlenstoffatomen in den Acylresten von 10 bis 30 und 1 bis 5 Doppelbindungen;
Prostaglandinen, und zwar 6-keto-Prostaglandin F1 alpha, Prostaglandin D2;
Putrescin;

13. Verwendung nach Anspruch 12, wobei die endogenen Referenz-Metaboliten ausgewählt werden aus der Gruppe bestehend aus:
15((S)-Hydroxy-5Z,8Z,11Z,13E-eicosatetraensäure
6-keto-Prostaglandin F1 alpha
asymmetrischem Dimethylarginin
Arginin
PTC-Arginin
Aspartamsäure
Carnitin (frei)
Decanoylcarnitin [Caprylcarnitin] (Fumarylcarnitin)
Decenoylcarnitin
Decadienoylcarnitin
Dodecanoylcarnitin [Laurylcarnitin]
Dodecenoylcarnitin
Dodecandioylcarnitin
Tetradecanoylcarnitin
Tetradecenoylcarnitin [Myristoleylcarnitin]
3-Hydroxytetradecenoylcarnitin [3-Hydroxymyristoleylcarnitin]
3-Hydroxytetradecadienoylcarnitin
3-Hydroxytetradecanoylcarnitin [Hydroxymyristylcarnitin]
Hexadecenoylcarnitin [Palmitoleylcarnitin]
3-Hydroxyhexadecenoylcarnitin [3-Hydroxypalmitoleylcarnitin]
Hexadecadienoylcarnitin
3-Hydroxyhexadecadienoylcarnitin
3-Hydroxyhexadecanolycarnitin [3-Hydroxypalmitoylcarnitin]
Octadecanoylcarnitin [Stearylcarnitin]
Octadecenoylcarnitin [Oleylcarnitin]
3-Hydroxyoctadecenoylcarnitin [3-Hydroxyoleylcarnitin]
Acetylcarnitin
Propenoylcarnitin
Hydroxypropionylcarnitin
Butenoylcarnitin
3-Hydroxybutyrylcarnitin / Malonylcarnitin
Isovalerylcarnitin / 2-Methylbutyrylcarnitin / Valerylcarnitin
Tiglylcarnitin / 3-Methylcrotonylcarnitin
Glutarylcarnitin / Hydroxycaproylcarnitin
Glutarylcarnitin / Hydroxycaproylcarnitin
Methylglutarylcarnitin
3-Hydroxyisovalerylcarnitin / 3-Hydroxy-2-methylbutyryl
Hexanoylcarnitin [Caproylcarnitin]
Hexenoylcarnitin
Pimelylcarnitin
Octanoylcarnitin [Caprylylcarnitin]
Octenoylcarnitin
Nonanoylcarnitin [Pelargonylcarnitin]
Citrullin
Creatinin
Glutamin
PTC-Glutamin
Glutamat
Histidin
PTC-Histidin
Kynurenin
Leucin
Lysophosphatidylcholin mit Acylrest C14:0
Lysophosphatidylcholin mit Acylrest C16:0
Lysophosphatidylcholin mit Acylrest C16:1
Lysophosphatidylcholin mit Acylrest C18:0
Lysophosphatidylcholin mit Acylrest C18:1
Lysophosphatidylcholin mit Acylrest C18:2
Lysophosphatidylcholin mit Acylrest C20:3
Lysophosphatidylcholin mit Acylrest C20:4
Lysophosphatidylcholin mit Acylrest C24:0
Lysophosphatidylcholin mit Acylrest C26:0
Lysophosphatidylcholin mit Acylrest C26:1
Lysophosphatidylcholin mit Acylrest C28:0
Lysophosphatidylcholin mit Acylrest C28:1
Lysophosphatidylcholin mit Acylrest C6:0
PTC-Methionin
Ornithin
PTC-Ornithin
Phosphatidylcholin mit Diacylrestsumme C24:0
Phosphatidylcholin mit Diacylrestsumme C26:0
Phosphatidylcholin mit Diacylrestsumme C28:1
Phosphatidylcholin mit Diacylrestsumme C30:0
Phosphatidylcholin mit Diacylrestsumme C30:2
Phosphatidylcholin mit Diacylrestsumme C32:0
Phosphatidylcholin mit Diacylrestsumme C32:1
Phosphatidylcholin mit Diacylrestsumme C32:2
Phosphatidylcholin mit Diacylrestsumme C32:3
Phosphatidylcholin mit Diacylrestsumme C34:1
Phosphatidylcholin mit Diacylrestsumme C34:2
Phosphatidylcholin mit Diacylrestsumme C34:3
Phosphatidylcholin mit Diacylrestsumme C34:4
Phosphatidylcholin mit Diacylrestsumme C36:0
Phosphatidylcholin mit Diacylrestsumme C36:1
Phosphatidylcholin mit Diacylrestsumme C36:2
Phosphatidylcholin mit Diacylrestsumme C36:3
Phosphatidylcholin mit Diacylrestsumme C36:4
Phosphatidylcholin mit Diacylrestsumme C36:5
Phosphatidylcholin mit Diacylrestsumme C36:6
Phosphatidylcholin mit Diacylrestsumme C38:0
Phosphatidylcholin mit Diacylrestsumme C38:1
Phosphatidylcholin mit Diacylrestsumme C38:3
Phosphatidylcholin mit Diacylrestsumme C38:4
Phosphatidylcholin mit Diacylrestsumme C38:5
Phosphatidylcholin mit Diacylrestsumme C38:6
Phosphatidylcholin mit Diacylrestsumme C42:1
Phosphatidylcholin mit Diacylrestsumme C40:3
Phosphatidylcholin mit Diacylrestsumme C40:4
Phosphatidylcholin mit Diacylrestsumme C40:5
Phosphatidylcholin mit Diacylrestsumme C40:6
Phosphatidylcholin mit Diacylrestsumme C42:0
Phosphatidylcholin mit Diacylrestsumme C42:1
Phosphatidylcholin mit Diacylrestsumme C42:2
Phosphatidylcholin mit Diacylrestsumme C42:4
Phosphatidylcholin mit Diacylrestsumme C42:5
Phosphatidylcholin mit Diacylrestsumme C42:6
Phosphatidylcholin mit Acyl-Alkylrestsumme C30:0
Phosphatidylcholin mit Acyl-Alkylrestsumme C30:1
Phosphatidylcholin mit Acyl-Alkylrestsumme C34:0
Phosphatidylcholin mit Acyl-Alkylrestsumme C34:1
Phosphatidylcholin mit Acyl-Alkylrestsumme C34:2
Phosphatidylcholin mit Acyl-Alkylrestsumme C34:3
Phosphatidylcholin mit Acyl-Alkylrestsumme C36:0
Phosphatidylcholin mit Acyl-Alkylrestsumme C36:1
Phosphatidylcholin mit Acyl-Alkylrestsumme C36:2
Phosphatidylcholin mit Acyl-Alkylrestsumme C36:3
Phosphatidylcholin mit Acyl-Alkylrestsumme C36:4
Phosphatidylcholin mit Acyl-Alkylrestsumme C38:0
Phosphatidylcholin mit Acyl-Alkylrestsumme C38:1
Phosphatidylcholin mit Acyl-Alkylrestsumme C38:2
Phosphatidylcholin mit Acyl-Alkylrestsumme C38:3
Phosphatidylcholin mit Acyl-Alkylrestsumme C38:4
Phosphatidylcholin mit Acyl-Alkylrestsumme C38:5
Phosphatidylcholin mit Acyl-Alkylrestsumme C38:6
Phosphatidylcholin mit Acyl-Alkylrestsumme C40:0
Phosphatidylcholin mit Acyl-Alkylrestsumme C40:1
Phosphatidylcholin mit Acyl-Alkylrestsumme C40:2
Phosphatidylcholin mit Acyl-Alkylrestsumme C40:3
Phosphatidylcholin mit Acyl-Alkylrestsumme C40:4
Phosphatidylcholin mit Acyl-Alkylrestsumme C40:5
Phosphatidylcholin mit Acyl-Alkylrestsumme C40:6
Phosphatidylcholin mit Acyl-Alkylrestsumme C42:0
Phosphatidylcholin mit Acyl-Alkylrestsumme C42:1
Phosphatidylcholin mit Acyl-Alkylrestsumme C42:3
Phosphatidylcholin mit Acyl-Alkylrestsumme C42:4
Phosphatidylcholin mit Acyl-Alkylrestsumme C42:5
Phosphatidylcholin mit Acyl-Alkylrestsumme C44:5
Phosphatidylcholin mit Acyl-Alkylrestsumme C44:6
Prostaglandin D2
Phenylalanin
PTC-Phenylalanin
Prolin
PTC-Prolin
Putrescin
Serin
PTC-Serin
Hydroxysphingomyelin mit Acylrestsumme C14:1
Hydroxysphingomyelin mit Acylrestsumme C16:1
Hydroxysphingomyelin mit Acylrestsumme C22:1
Hydroxysphingomyelin mit Acylrestsumme C22:2
Hydroxysphingomyelin mit Acylrestsumme C24:1
Sphingomyelin mit Acylrestsumme C16:0
Sphingomyelin mit Acylrestsumme C16:1
Sphingomyelin mit Acylrestsumme C18:0
Sphingomyelin mit Acylrestsumme C 18:1
Sphingomyelin mit Acylrestsumme C20:2
Sphingomyelin mit Acylrestsumme C22:3
Sphingomyelin mit Acylrestsumme C24:0
Sphingomyelin mit Acylrestsumme C24:1
Sphingomyelin mit Acylrestsumme C26:0
Sphingomyelin mit Acylrestsumme C26: 1
Bernsteinsäure (Succinat)
Gesamt-Dimethylarginin: Summe ADMA + SDMA
Tryptophan
PTC-Tryptophan
Tyrosin
Valin
PTC-Valin
Leucin + Isoleucin
wobei die Bezeichnung "-rest Cn:m" oder "-restsumme Cn:m" die Kettenlänge des einen bzw. der mehreren Acyl-/Alkylreste darstellt, n die Anzahl der Kohlenstoffatome in dem einen bzw. den mehreren Acyl-/Alkylresten darstellt und m die Gesamtzahl der Doppelbindungen in dem einen bzw. den mehreren Resten darstellt.

14. Verwendung nach Anspruch 13, wobei sich die endogenen Referenz-Metaboliten gemäß statistischen Stabilitätsmessungen, die ausgewählt werden aus der Gruppe bestehend aus Variationskoeffizient (CV) von Intensitätsrohdaten, Standardabweichung (SD) von logarithmischen Intensitätsdaten, Stabilitätsmessung (M) von GeNorm-Algorithmus oder Stabilitätsmesswert (rho) von NormFinder-Algorithmus, als stabil erweisen.

15. Verwendung nach einem der Ansprüche 12 bis 14, wobei sich die endogenen Referenz-Metaboliten gemäß mindestens 2 statistischen Stabilitätsmessungen, die ausgewählt werden aus der Gruppe bestehend aus Variationskoeffizient (CV) von Intensitätsrohdaten, Standardabweichung (SD) von logarithmischen Intensitätsdaten, Stabilitätsmessung (M) von GeNorm-Algorithmus oder Stabilitätsmesswert (rho) von NormFinder-Algorithmus als stabil erweisen und/oder diese endogenen Referenz-Metaboliten insbesondere ausgewählt werden aus der Gruppe bestehend aus:
PTC-Arginin
Carnitin (frei)
Decenoylcarnitin
Decanoylcarnitin [Caprylcarnitin] (Fumarylcarnitin)
Dodecenoylcarnitin
Dodecandioylcarnitin
Dodecanoylcarnitin [Laurylcarnitin]
Tetradecanoylcarnitin
3-Hydroxytetradecanoylcarnitin [Hydroxymyristylcarnitin]
Hexadecenoylcarnitin [Palmitoleylcarnitin]
3-Hydroxyhexadecenoylcarnitin [3-Hydroxypalmitoleylcarnitin]
3-Hydroxyhexadecadienoylcarnitin
3-Hydroxyhexadecanolycarnitin [3-Hydroxypalmitoylcarnitin]
3-Hydroxyoctadecenoylcarnitin [3-Hydroxyoleylcarnitine]
Propenoylcarnitin
Hydroxypropionylcarnitin
3-Hydroxybutyrylcarnitin / Malonylcarnitin
Methylglutarylcarnitin
3-Hydroxyisovalerylcarnitin / 3-Hydroxy-2-methylbutyryl
Hexenoylcarnitin
Hexanoylcarnitin [Caproylcarnitin]
Pimelylcarnitin
Octenoylcarnitin
Octanoylcarnitin [Caprylylcarnitin]
Glutamin
PTC-Glutamin
PTC-Histidin
Lysophosphatidylcholin mit Acylrest C14:0
Lysophosphatidylcholin mit Acylrest C26:0
Lysophosphatidylcholin mit Acylrest C26:1
Lysophosphatidylcholin mit Acylrest C28:0
Lysophosphatidylcholin mit Acylrest C28:1
Phosphatidylcholin mit Diacylrestsumme C24:0
Phosphatidylcholin mit Diacylrestsumme C26:0
Phosphatidylcholin mit Diacylrestsumme C30:0
Phosphatidylcholin mit Diacylrestsumme C30:2
Phosphatidylcholin mit Diacylrestsumme C32:2
Phosphatidylcholin mit Diacylrestsumme C34:2
Phosphatidylcholin mit Diacylrestsumme C36:0
Phosphatidylcholin mit Diacylrestsumme C36:2
Phosphatidylcholin mit Diacylrestsumme C36:4
Phosphatidylcholin mit Diacylrestsumme C38:0
Phosphatidylcholin mit Diacylrestsumme C38:1
Phosphatidylcholin mit Diacylrestsumme C42:1
Phosphatidylcholin mit Diacylrestsumme C42:0
Phosphatidylcholin mit Diacylrestsumme C42:5
Phosphatidylcholin mit Diacylrestsumme C42:6
Phosphatidylcholin mit Acyl-Alkylrestsumme C30:1
Phosphatidylcholin mit Acyl-Alkylrestsumme C34:1
Phosphatidylcholin mit Acyl-Alkylrestsumme C36:0
Phosphatidylcholin mit Acyl-Alkylrestsumme C38:1
Phosphatidylcholin mit Acyl-Alkylrestsumme C38:4
Phosphatidylcholin mit Acyl-Alkylrestsumme C38:6
Phosphatidylcholin mit Acyl-Alkylrestsumme C40:0
Phosphatidylcholin mit Acyl-Alkylrestsumme C40:1
Phosphatidylcholin mit Acyl-Alkylrestsumme C40:5
Phosphatidylcholin mit Acyl-Alkylrestsumme C40:6
Phosphatidylcholin mit Acyl-Alkylrestsumme C42:0
Phosphatidylcholin mit Acyl-Alkylrestsumme C42:5
Phosphatidylcholin mit Acyl-Alkylrestsumme C44:6
Phenylalanin
PTC-Phenylalanin
Prolin
Sphingomyelin mit Acylrestsumme C16:0
Sphingomyelin mit Acylrestsumme C16:1
Sphingomyelin mit Acylrestsumme C18:0
Sphingomyelin mit Acylrestsumme C18:1
Sphingomyelin mit Acylrestsumme C20:2
Sphingomyelin mit Acylrestsumme C24:0
Sphingomyelin mit Acylrestsumme C24:1
Hydroxysphingomyelin mit Acylrestsumme C14:1
Hydroxysphingomyelin mit Acylrestsumme C16:1
Hydroxysphingomyelin mit Acylrestsumme C22:2
Hydroxysphingomyelin mit Acylrestsumme C24:1
Bernsteinsäure (Succinat)
PTC-Tryptophan
Valin
PTC-Valin
Leucin + Isoleucin
wobei die Bezeichnung "-rest Cn:m" oder "-restsumme Cn:m" die Kettenlänge des einen bzw. der mehreren Acyl-/Alkylreste darstellt, n die Anzahl der Kohlenstoffatome in dem einen bzw. den mehreren Acyl-/Alkylresten darstellt und m die Gesamtzahl der Doppelbindungen in dem einen bzw. den mehreren Resten darstellt.

16. Verwendung nach einem der Ansprüche 12 bis 15, wobei sich die endogenen Referenz-Metaboliten gemäß mindestens 3 statistischen Stabilitätsmessungen, die ausgewählt werden aus der Gruppe bestehend aus Variationskoeffizient (CV) von Intensitätsrohdaten, Standardabweichung (SD) von logarithmischen Intensitätsdaten, Stabilitätsmessung (M) von GeNorm-Algorithmus oder Stabilitätsmesswert (rho) von NormFinder-Algorithmus als stabil erweisen und/oder diese endogenen Referenz-Metaboliten insbesondere ausgewählt werden aus der Gruppe bestehend aus:
Carnitin (frei)
Decanoylcarnitin [Caprylcarnitin] (Fumarylcarnitin)
Dodecandioylcarnitin
Dodecanoylcarnitin [Laurylcarnitin]
Hexadecenoylcarnitin [Palmitoleylcarnitin]
3-Hydroxyhexadecenoylcarnitin [3-Hydroxypalmitoleylcarnitin]
3-Hydroxyhexadecanolycarnitin [3-Hydroxypalmitoylcarnitin]
Propenoylcarnitin
Hydroxypropionylcarnitin
3-Hydroxybutyrylcarnitin / Malonylcarnitin
Methylglutarylcarnitin
Hexanoylcarnitin [Caproylcarnitin]
Pimelylcarnitin
Octenoylcarnitin
Octanoylcarnitin [Caprylylcarnitin]
Glutamin
PTC-Glutamin
PTC-Histidin
Lysophosphatidylcholin mit Acylrest C14:0
Lysophosphatidylcholin mit Acylrest C26:0
Lysophosphatidylcholin mit Acylrest C26:1
Lysophosphatidylcholin mit Acylrest C28:1
Phosphatidylcholin mit Diacylrestsumme C26:0
Phosphatidylcholin mit Diacylrestsumme C32:2
Phosphatidylcholin mit Diacylrestsumme C36:0
Phosphatidylcholin mit Diacylrestsumme C38:0
Phosphatidylcholin mit Diacylrestsumme C42:1
Phosphatidylcholin mit Diacylrestsumme C42:5
Phosphatidylcholin mit Diacylrestsumme C42:6
Phosphatidylcholin mit Acyl-Alkylrestsumme C38:4
Phosphatidylcholin mit Acyl-Alkylrestsumme C40:0
Phosphatidylcholin mit Acyl-Alkylrestsumme C42:0
Phosphatidylcholin mit Acyl-Alkylrestsumme C42:5
Phosphatidylcholin mit Acyl-Alkylrestsumme C44:6 Sphingomyelin mit Acylrestsumme C16:0
Sphingomyelin mit Acylrestsumme C16:1
Sphingomyelin mit Acylrestsumme C20:2
Sphingomyelin mit Acylrestsumme C24:0
Hydroxysphingomyelin mit Acylrestsumme C24:1
Valin
PTC-Valin
wobei die Bezeichnung "-rest Cn:m" oder "-restsumme Cn:m" die Kettenlänge des einen bzw. der mehreren Acyl-/Alkylreste darstellt, n die Anzahl der Kohlenstoffatome in dem einen bzw. den mehreren Acyl-/Alkylresten darstellt und m die Gesamtzahl der Doppelbindungen in dem einen bzw. den mehreren Resten darstellt.

17. Verwendung nach irgendeinem der Ansprüche 12 bis 16, wobei sich die endogenen Referenz-Metaboliten gemäß allen statistischen Stabilitätsmessungen, die ausgewählt werden aus der Gruppe bestehend aus Variationskoeffizient (CV) von Intensitätsrohdaten, Standardabweichung (SD) von logarithmischen Intensitätsdaten, Stabilitätsmessung (M) von GeNorm-Algorithmus oder Stabilitätsmesswert (rho) von NormFinder-Algorithmus als stabil erweisen und/oder wobei es sich bei diesen endogenen Referenz-Metaboliten insbesondere um Phosphatidylcholin mit einer Diacylrestsumme C42:6 handelt, wobei die Bezeichnung "C42:6" die Kettenlänge der Acylreste darstellt, 42 die Gesamtzahl der Kohlenstoffatome in den Acylresten darstellt und 6 die Gesamtzahl der Doppelbindungen in den Resten darstellt.

18. Verwendung nach einem der Ansprüche 12 bis 17, wobei die mehreren endogenen Metaboliten 2 bis 80, insbesondere 2 bis 60, vorzugsweise 2 bis 50, bevorzugt 2 bis 30, stärker bevorzugt 2 bis 10, besonders bevorzugt 2 bis 10, vorzugsweise 3 bis 5 endogene Metaboliten umfassen.

## Revendications

1. Méthode de normalisation des données d'intensité correspondant à des quantités et/ou des concentrations de métabolites cibles choisis au sein d'un échantillon biologique d'un sujet mammifère, où lesdites données d'intensité sont obtenues par le biais d'une méthode d'analyse métabolomique comprenant la génération de données d'intensité pour la quantification de métabolites endogènes par spectrométrie de masse (MS), en particulier les technologies MS telles que MALDI (désorption-ionisation laser assistée par matrice), ESI (ionisation par électrospray), APCI (ionisation chimique à pression atmosphérique), la spectroscopie de résonance magnétique nucléaire (RMN) ¹H, ¹³C et/ou ³¹P, éventuellement couplée à la MS, la détermination de concentrations de métabolites par utilisation de méthodes et/ou de technologies MS couplées à une séparation, en particulier la chromatographie liquide (LC-MS), la chromatographie en phase gazeuse (GC-MS) ou l'électrophorèse capillaire (CE-MS), avec une multitude de métabolites endogènes de référence,
**comprenant**
la mise en oeuvre d'au moins une méthode d'analyse métabolomique *in vitro* desdits métabolites cibles choisis au sein dudit échantillon biologique ;
la mise en oeuvre simultanée dans le même échantillon d'une analyse quantitative d'une multitude de métabolites de références endogènes ou de leurs dérivés, où lesdits métabolites endogènes de références sont des composés de l'échantillon biologique qui sont présents chez le sujet à un niveau essentiellement constant ; et où lesdits métabolites endogènes de références ou leurs dérivés présentent une masse moléculaire inférieure à 1500 Da et sont choisis dans le groupe constitué par les suivants :
acides aminés, en particulier arginine, acide aspartique, citrulline, acide glutamique (glutamate), glutamine, leucine, isoleucine, histidine, ornithine, proline ;
phénylalanine, sérine, tryptophane, tyrosine, valine, kynurénine ;
acides phénylthiocarbamylaminés (acides PTC-aminés), en particulier PCT-arginine, PTC-glutamine, PTC-histidine, PTC-méthionine, PTC-ornithine, PTC-phénylalanine, PTC-proline, PTC-sérine, PTC-tryptophane, PTC-tyrosine, PTC-valine ;
diméthylarginine, en particulier N, N-diméthyl-L-arginine ;
acides carboxyliques, nommément acide 15(S)-hydroxy-5Z, 8Z, 11Z, 13E-éicosatétraénoïque [acide (5Z, 8Z, 11Z, 13E, 15S)-15hydroxyicosa-5, 8, 11, 13-tétraénoïque], acide succinique (succinate) ;
carnitine ; acylcarnitines comportant entre 1 et 20 atomes de carbone au niveau du résidu acyle ; acylcarnitines comportant entre 3 et 20 atomes de carbone au niveau du résidu acyle et entre 1 et 4 doubles liaisons au niveau du résidu acyle ; acylcarnitines comportant entre 1 et 20 atomes de carbone au niveau du résidu acyle et comportant entre 1 et 3 groupements OH au niveau du résidu acyle ; acylcarnitines comportant entre 3 et 20 atomes de carbone au niveau du résidu acyle avec 1 à 4 doubles liaisons et 1 à 3 groupements OH au niveau du résidu acyle ;
phospholipides, en particulier
lysophosphatidylcholines (monoacylphosphatidylcholines) comportant entre 1 et 30 atomes de carbone au niveau du résidu acyle; lysophosphatidylcholines comportant entre 3 et 30 atomes de carbone au niveau du résidu acyle et entre 1 et 6 doubles liaisons au niveau du résidu acyle ;
phosphatidylcholines (diacylphosphatidylcholines) comportant au total entre 1 et 50 atomes de carbone au niveau des résidus acyle ; phosphatidylcholines comportant au total entre 3 et 50 atomes de carbone au niveau des résidus acyle et au total entre 1 et 8 doubles liaisons au niveau des résidus acyle ;
sphingolipides, en particulier sphingomyélines comportant un nombre total d'atomes de carbone au niveau des chaînes acyle compris entre 10 et 30 ; sphingomyélines comportant un nombre total d'atomes de carbone au niveau des chaînes acyle compris entre 10 et 30 et entre 1 et 5 doubles liaisons ; hydroxysphingomyélines comportant un nombre total d'atomes de carbone au niveau des résidus acyle compris entre 10 et 30 ; hydroxysphingomyélines comportant un nombre total d'atomes de carbone au niveau des résidus acyle compris entre 10 et 30 et entre 1 et 5 doubles liaisons ;
prostaglandines, nommément 6-céto-prostaglandine F1 alpha, prostaglandine D2 ;
putrescine ;
et où
chacune desdites intensités détectées desdits métabolites cibles choisis est liée auxdites intensités desdits métabolites endogènes de référence.

2. Méthode selon la revendication 1, où la multitude d'intensités des métabolites cibles et endogènes de référence est soumise à un prétraitement mathématique, en particulier des transformations comme l'application de logarithmes, de logarithmes généralisés, de transformations par puissances.

3. Méthode selon la revendication 1 ou 2, où la multitude d'intensités des métabolites endogènes de référence est agrégée à une valeur de référence unique.

4. Méthode selon la revendication 3, où la multitude d'intensités des métabolites endogènes de référence est agrégée à une valeur de référence unique par calcul de la valeur moyenne géométrique, de la valeur moyenne arithmétique, de la valeur médiane, de la valeur moyenne arithmétique pondérée.

5. Méthode selon l'une quelconque des revendications 1 à 4, où un rapport est formé par chacune des intensités des métabolites cibles et la valeur de référence déterminée en cas d'intensités linéaires, ou la valeur de référence déterminée est soustraite de chacune des intensités des métabolites cibles en cas d'intensités logarithmiques.

6. Méthode selon l'une quelconque des revendications 1 à 5, où lesdits métabolites endogènes de référence sont choisis dans le groupe constitué par les suivants :
acide 15(S)-hydroxy-5Z, 8Z, 11Z, 13E-éicosatétraénoïque
6-céto-Prostaglandine F1 alpha
Diméthylarginine asymétrique
Arginine
PTC-Arginine
Acide aspartique
Carnitine (libre)
Décanoylcarnitine [Caprylcarnitine] (Fumarylcarnitine)
Décénoylcarnitine
Décadiénoylcarnitine
Dodécanoylcarnitine [Laurylcarnitine]
Dodécénoylcarnitine
Dodécanedioylcarnitine
Tétradécanoylcarnitine
Tétradécénoylcarnitine [Myristoléylcarnitine]
3-Hydroxytétradécénoylcarnitine
[3-Hydroxymyristoléylcarnitine]
3-Hydroxytétradécadiénoylcarnitine
3-Hydroxytétradécanoylcarnitine [Hydroxymyristylcarnitine]
Hexadécénoylcarnitine [Palmitoléylcarnitine]
3-Hydroxyhexadécénoylcarnitine [3-Hydroxypalmitoléylcarnitine]
Hexadécadiénoylcarnitine
3-Hydroxyhexadécadiénoylcarnitine
3-Hydroxyhexadécanoylcarnitine [3-Hydroxypalmitoylcarnitine]
Octadécanoylcarnitine [Stéarylcarnitine]
Octadécénoylcarnitine [Oléylcarnitine]
3-Hydroxyoctadécénoylcarnitine [3-Hydroxyoléylcarnitine]
Acétylcarnitine
Propénoylcarnitine
Hydroxypropionylcarnitine
Buténoylcarnitine
3-Hydroxybutyrylcarnitine / Malonylcarnitine
Isovalérylcarnitine / 2-Méthylbutyrylcarnitine / Valérylcarnitine
Tiglylcarnitine / 3-Méthyl-crotonylcarnitine
Glutarylcarnitine / Hydroxycaproylcarnitine
Glutarylcarnitine / Hydroxycaproylcarnitine
Méthylglutarylcarnitine
3-Hydroxyisovalérylcarnitine / 3-Hydroxy-2-méthylbutyryle
Hexanoylcarnitine [Caproylcarnitinel
Hexénoylcarnitine
Pimélylcarnitine
Octanoylcarnitine [Caprylylcarnitine]
Octénoylcarnitine
Nonanoylcarnitine [Pelargonylcarnitine]
Citrulline
Créatinine
Glutamine
PTC-Glutamine
Glutamate
Histidine
PTC-Histidine
Kynurénine
Leucine
Lysophosphatidylcholine avec résidu acyle C14:0
Lysophosphatidylcholine avec résidu acyle C16:0
Lysophosphatidylcholine avec résidu acyle C16:1
Lysophosphatidylcholine avec résidu acyle C18:0
Lysophosphatidylcholine avec résidu acyle C18:1
Lysophosphatidylcholine avec résidu acyle C18:2
Lysophosphatidylcholine avec résidu acyle C20:3
Lysophosphatidylcholine avec résidu acyle C20:4
Lysophosphatidylcholine avec résidu acyle C24:0
Lysophosphatidylcholine avec résidu acyle C26:0
Lysophosphatidylcholine avec résidu acyle C26:1
Lysophosphatidylcholine avec résidu acyle C28:0
Lysophosphatidylcholine avec résidu acyle C28:1
Lysophosphatidylcholine avec résidu acyle C6:0
PTC-Méthionine
Ornithine
PTC-Ornithine
Phosphatidylcholine avec somme des résidus diacyle C24:0
Phosphatidylcholine avec somme des résidus diacyle C26:0
Phosphatidylcholine avec somme des résidus diacyle C28:1
Phosphatidylcholine avec somme des résidus diacyle C30:0
Phosphatidylcholine avec somme des résidus diacyle C30:2
Phosphatidylcholine avec somme des résidus diacyle C32:0
Phosphatidylcholine avec somme des résidus diacyle C32:1
Phosphatidylcholine avec somme des résidus diacyle C32:2
Phosphatidylcholine avec somme des résidus diacyle C32:3
Phosphatidylcholine avec somme des résidus diacyle C34:1
Phosphatidylcholine avec somme des résidus diacyle C34:2
Phosphatidylcholine avec somme des résidus diacyle C34:3
Phosphatidylcholine avec somme des résidus diacyle C34:4
Phosphatidylcholine avec somme des résidus diacyle C36:0
Phosphatidylcholine avec somme des résidus diacyle C36:1
Phosphatidylcholine avec somme des résidus diacyle C36:2
Phosphatidylcholine avec somme des résidus diacyle C36:3
Phosphatidylcholine avec somme des résidus diacyle C36:4
Phosphatidylcholine avec somme des résidus diacyle C36:5
Phosphatidylcholine avec somme des résidus diacyle C36:6
Phosphatidylcholine avec somme des résidus diacyle C38:0
Phosphatidylcholine avec somme des résidus diacyle C38:1
Phosphatidylcholine avec somme des résidus diacyle C38:3
Phosphatidylcholine avec somme des résidus diacyle C38:4
Phosphatidylcholine avec somme des résidus diacyle C38:5
Phosphatidylcholine avec somme des résidus diacyle C38:6
Phosphatidylcholine avec somme des résidus diacyle C42:1
Phosphatidylcholine avec somme des résidus diacyle C40:3
Phosphatidylcholine avec somme des résidus diacyle C40:4
Phosphatidylcholine avec somme des résidus diacyle C40:5
Phosphatidylcholine avec somme des résidus diacyle C40:6
Phosphatidylcholine avec somme des résidus diacyle C42:0
Phosphatidylcholine avec somme des résidus diacyle C42:1
Phosphatidylcholine avec somme des résidus diacyle C42:2
Phosphatidylcholine avec somme des résidus diacyle C42:4
Phosphatidylcholine avec somme des résidus diacyle C42:5
Phosphatidylcholine avec somme des résidus diacyle C42:6
Phosphatidylcholine avec somme des résidus acyl-alkyle C30:0
Phosphatidylcholine avec somme des résidus acyl-alkyle C30:1
Phosphatidylcholine avec somme des résidus acyl-alkyle C34:0
Phosphatidylcholine avec somme des résidus acyl-alkyle C34:1
Phosphatidylcholine avec somme des résidus acyl-alkyle C34:2
Phosphatidylcholine avec somme des résidus acyl-alkyle C34:3
Phosphatidylcholine avec somme des résidus acyl-alkyle C36:0
Phosphatidylcholine avec somme des résidus acyl-alkyle C36:1
Phosphatidylcholine avec somme des résidus acyl-alkyle C36:2
Phosphatidylcholine avec somme des résidus acyl-alkyle C36:3
Phosphatidylcholine avec somme des résidus acyl-alkyle C36:4
Phosphatidylcholine avec somme des résidus acyl-alkyle C38:0
Phosphatidylcholine avec somme des résidus acyl-alkyle C38:1
Phosphatidylcholine avec somme des résidus acyl-alkyle C38:2
Phosphatidylcholine avec somme des résidus acyl-alkyle C38:3
Phosphatidylcholine avec somme des résidus acyl-alkyle C38:4
Phosphatidylcholine avec somme des résidus acyl-alkyle C38:5
Phosphatidylcholine avec somme des résidus acyl-alkyle C38:6
Phosphatidylcholine avec somme des résidus acyl-alkyle C40:0
Phosphatidylcholine avec somme des résidus acyl-alkyle C40:1
Phosphatidylcholine avec somme des résidus acyl-alkyle C40:2
Phosphatidylcholine avec somme des résidus acyl-alkyle C40:3
Phosphatidylcholine avec somme des résidus acyl-alkyle C40:4
Phosphatidylcholine avec somme des résidus acyl-alkyle C40:5
Phosphatidylcholine avec somme des résidus acyl-alkyle C40:6
Phosphatidylcholine avec somme des résidus acyl-alkyle C42:0
Phosphatidylcholine avec somme des résidus acyl-alkyle C42:1
Phosphatidylcholine avec somme des résidus acyl-alkyle C42:3
Phosphatidylcholine avec somme des résidus acyl-alkyle C42:4
Phosphatidylcholine avec somme des résidus acyl-alkyle C42:5
Phosphatidylcholine avec somme des résidus acyl-alkyle C44:5
Phosphatidylcholine avec somme des résidus acyl-alkyle C44:6
Prostaglandine D2
Phénylalanine
PTC-Phénylalanine
Proline
PTC-Proline
Putrescine
Sérine
PTC-Sérine
Hydroxysphingomyéline avec somme des résidus acyle C14:1
Hydroxysphingomyéline avec somme des résidus acyle C16:1
Hydroxysphingomyéline avec somme des résidus acyle C22:1
Hydroxysphingomyéline avec somme des résidus acyle C22:2
Hydroxysphingomyéline avec somme des résidus acyle C24:1
Sphingomyéline avec somme des résidus acyle C16:0
Sphingomyéline avec somme des résidus acyle C16:1
Sphingomyéline avec somme des résidus acyle C18:0
Sphingomyéline avec somme des résidus acyle C18:1
Sphingomyéline avec somme des résidus acyle C20:2
Sphingomyéline avec somme des résidus acyle C22:3
Sphingomyéline avec somme des résidus acyle C24:0
Sphingomyéline avec somme des résidus acyle C24:1
Sphingomyéline avec somme des résidus acyle C26:0
Sphingomyéline avec somme des résidus acyle C26:1
Acide succinique (succinate)
Diméthylarginine totale : somme ADMA + SDMA
Tryptophane
PTC-Tryptophane
Tyrosine
Valine
PTC-Valine
Leucine + Isoleucine
où la désignation « résidu Cn:m » ou « somme des résidus Cn:m » représente la longueur de chaîne du ou des résidus acyle/alkyle, n représente le nombre total d'atomes de carbone dans le ou les résidus acyle/alkyle, et m représente le nombre total de doubles liaisons dans le ou les résidus.

7. Méthode selon l'une quelconque des revendications 1 à 6, où de tels métabolites sont utilisés en tant que métabolites endogènes de référence qui présentent une stabilité selon les mesures de stabilité statistique choisies dans le groupe constitué par le coefficient de variation (CV) des données d'intensité brutes, l'écart-type (SD) des données d'intensité logarithmique, la mesure de stabilité (M) de l'algorithme geNorm ou la valeur de mesure de stabilité (rho) de l'algorithme NormFinder.

8. Méthode selon l'une quelconque des revendications 1 à 7, où lesdits métabolites endogènes de référence présentent une stabilité selon au moins 2 mesures de stabilité statistique choisies dans le groupe constitué par le coefficient de variation (CV) des données d'intensité brutes, l'écart-type (SD) des données d'intensité logarithmique, la mesure de stabilité (M) de l'algorithme geNorm ou la valeur de mesure de stabilité (rho) de l'algorithme NormFinder, *et*/*ou* de tels métabolites endogènes de référence étant en particulier choisis dans le groupe constitué par les suivants :
PTC-Arginine
Carnitine (libre)
Décénoylcarnitine
Décanoylcarnitine [Caprylcarnitine] (Fumarylcarnitine)
Dodécénoylcarnitine
Dodécanedioylcarnitine
Dodécanoylcarnitine [Laurylcarnitine]
Tétradécanoylcarnitine
3-Hydroxytétradécanoylcarnitine [Hydroxymyristylcarnitine]
Hexadécénoylcarnitine [Palmitoléylcarnitine]
3-Hydroxyhexadécénoylcarnitine [3-Hydroxypalmitoléylcarnitine]
3-Hydroxyhexadécadiénoylcarnitine
3-Hydroxyhexadécanoylcarnitine [3-Hydroxypalmitoylcarnitine]
3-Hydroxyoctadécénoylcarnitine [3-Hydroxyoléylcarnitine]
Propénoylcarnitine
Hydroxypropionylcarnitine
3-Hydroxybutyrylcarnitine / Malonylcarnitine
Méthylglutarylcarnitine
3-Hydroxyisovalérylcarnitine / 3-Hydroxy-2-méthylbutyryle Hexénoylcarnitine
Hexanoylcarnitine [Caproylcarnitine]
Pimélylcarnitine
Octénoylcarnitine
Octanoylcarnitine [Caprylylcarnitine]
Glutamine
PTC-Glutamine
PTC-Histidine
Lysophosphatidylcholine avec résidu acyle C14:0
Lysophosphatidylcholine avec résidu acyle C26:0
Lysophosphatidylcholine avec résidu acyle C26:1
Lysophosphatidylcholine avec résidu acyle C28:0
Lysophosphatidylcholine avec résidu acyle C28:1
Phosphatidylcholine avec somme des résidus diacyle C24:0
Phosphatidylcholine avec somme des résidus diacyle C26:0
Phosphatidylcholine avec somme des résidus diacyle C30:0
Phosphatidylcholine avec somme des résidus diacyle C30:2
Phosphatidylcholine avec somme des résidus diacyle C32:2
Phosphatidylcholine avec somme des résidus diacyle C34:2
Phosphatidylcholine avec somme des résidus diacyle C36:0
Phosphatidylcholine avec somme des résidus diacyle C36:2
Phosphatidylcholine avec somme des résidus diacyle C36:4
Phosphatidylcholine avec somme des résidus diacyle C38:0
Phosphatidylcholine avec somme des résidus diacyle C38:1
Phosphatidylcholine avec somme des résidus diacyle C42:1
Phosphatidylcholine avec somme des résidus diacyle C42:0
Phosphatidylcholine avec somme des résidus diacyle C42:5
Phosphatidylcholine avec somme des résidus diacyle C42:6
Phosphatidylcholine avec somme des résidus acyl-alkyle C30:1
Phosphatidylcholine avec somme des résidus acyl-alkyle C34:1
Phosphatidylcholine avec somme des résidus acyl-alkyle C36:0
Phosphatidylcholine avec somme des résidus acyl-alkyle C38:1
Phosphatidylcholine avec somme des résidus acyl-alkyle C38:4
Phosphatidylcholine avec somme des résidus acyl-alkyle C38:6
Phosphatidylcholine avec somme des résidus acyl-alkyle C40:0
Phosphatidylcholine avec somme des résidus acyl-alkyle C40:1
Phosphatidylcholine avec somme des résidus acyl-alkyle C40:5
Phosphatidylcholine avec somme des résidus acyl-alkyle C40:6
Phosphatidylcholine avec somme des résidus acyl-alkyle C42:0
Phosphatidylcholine avec somme des résidus acyl-alkyle C42:5
Phosphatidylcholine avec somme des résidus acyl-alkyle C44:6
Phénylalanine
PTC-Phénylalanine
Proline
Sphingomyéline avec somme des résidus acyle C16:0
Sphingomyéline avec somme des résidus acyle C16:1
Sphingomyéline avec somme des résidus acyle C18:0
Sphingomyéline avec somme des résidus acyle C18:1
Sphingomyéline avec somme des résidus acyle C20:2
Sphingomyéline avec somme des résidus acyle C24:0
Sphingomyéline avec somme des résidus acyle C24:1
Hydroxysphingomyéline avec somme des résidus acyle C14:1
Hydroxysphingomyéline avec somme des résidus acyle C16:1
Hydroxysphingomyéline avec somme des résidus acyle C22:2
Hydroxysphingomyéline avec somme des résidus acyle C24:1
Acide succinique (succinate)
PTC-Tryptophane
Valine
PTC-Valine
Leucine + Isoleucine
où la désignation « résidu Cn:m » ou « somme des résidus Cn:m » représente la longueur de chaîne du ou des résidus acyle/alkyle, n représente le nombre total d'atomes de carbone dans le ou les résidus acyle/alkyle, et m représente le nombre total de doubles liaisons dans le ou les résidus.

9. Méthode selon l'une quelconque des revendications 1 à 8, où lesdits métabolites endogènes de référence présentent une stabilité selon au moins 3 mesures de stabilité statistique choisies dans le groupe constitué par le coefficient de variation (CV) des données d'intensité brutes, l'écart-type (SD) des données d'intensité logarithmique, la mesure de stabilité (M) de l'algorithme geNorm ou la valeur de mesure de stabilité (rho) de l'algorithme NormFinder, *et*/*ou* de tels métabolites endogènes de référence étant en particulier choisis dans le groupe constitué par les suivants :
Carnitine (libre)
Décanoylcarnitine [Caprylcarnitine] (Fumarylcarnitine)
Dodécanedioylcarnitine
Dodécanoylcarnitine [Laurylcarnitine]
Hexadécénoylcarnitine [Palmitoléylcarnitine]
3-Hydroxyhexadécénoylcarnitine [3-Hydroxypalmitoléylcarnitine]
3-Hydroxyhexadécanoylcarnitine [3-Hydroxypalmitoylcarnitine]
Propénoylcarnitine
Hydroxypropionylcarnitine
3-Hydroxybutyrylcarnitine / Malonylcarnitine
Méthylglutarylcarnitine
Hexanoylcarnitine [Caproylcarnitine]
Pimélylcarnitine
Octénoylcarnitine
Octanoylcarnitine [Caprylylcarnitine]
Glutamine
PTC-Glutamine
PTC-Histidine
Lysophosphatidylcholine avec résidu acyle C14:0
Lysophosphatidylcholine avec résidu acyle C26:0
Lysophosphatidylcholine avec résidu acyle C26:1
Lysophosphatidylcholine avec résidu acyle C28:1
Phosphatidylcholine avec somme des résidus diacyle C26:0
Phosphatidylcholine avec somme des résidus diacyle C32:2
Phosphatidylcholine avec somme des résidus diacyle C36:0
Phosphatidylcholine avec somme des résidus diacyle C38:0
Phosphatidylcholine avec somme des résidus diacyle C42:1
Phosphatidylcholine avec somme des résidus diacyle C42:5
Phosphatidylcholine avec somme des résidus diacyle C42:6
Phosphatidylcholine avec somme des résidus acyl-alkyle C38:4
Phosphatidylcholine avec somme des résidus acyl-alkyle C40:0
Phosphatidylcholine avec somme des résidus acyl-alkyle C42:0
Phosphatidylcholine avec somme des résidus acyl-alkyle C42:5
Phosphatidylcholine avec somme des résidus acyl-alkyle C44:6
Sphingomyéline avec somme des résidus acyle C16:0
Sphingomyéline avec somme des résidus acyle C16:1
Sphingomyéline avec somme des résidus acyle C20:2
Sphingomyéline avec somme des résidus acyle C24:0
Hydroxysphingomyéline avec somme des résidus acyle C24:1
Valine
PTC-Valine
où la désignation « résidu Cn:m » ou « somme des résidus Cn:m » représente la longueur de chaîne du ou des résidus acyle/alkyle, n représente le nombre total d'atomes de carbone dans le ou les résidus acyle/alkyle, et m représente le nombre total de doubles liaisons dans le ou les résidus.

10. Méthode selon l'une quelconque des revendications 1 to 8, où lesdits métabolites endogènes de référence présentent une stabilité selon toutes les mesures de stabilité statistique choisies dans le groupe constitué par le coefficient de variation (CV) des données d'intensité brutes, l'écart-type (SD) des données d'intensité logarithmique, la mesure de stabilité (M) de l'algorithme geNorm ou la valeur de mesure de stabilité (rho) de l'algorithme NormFinder, et/ou de tels métabolites endogènes de référence étant en particulier la Phosphatidylcholine avec somme des résidus diacyle C42:6, où la désignation « C42:6 » représente la longueur de chaîne des résidus acyle, 42 représente le nombre total d'atomes de carbone dans les résidus acyle, et 6 représente le nombre total de doubles liaisons dans les résidus.

11. Méthode selon l'une quelconque des revendications 1 à 10, où ladite multitude de métabolites endogènes comprend entre 2 et 80, en particulier entre 2 et 60, préférentiellement entre 2 et 50, préféré entre 2 et 30, plus préféré entre 2 et 10, particulièrement préféré entre 2 et 10, préférentiellement entre 3 et 5 métabolites endogènes.

12. Utilisation d'une multitude de composés ou de leurs dérivés, où lesdits composés présentent une masse moléculaire inférieure à 1500 Da, en tant que métabolites endogènes de référence dans des méthodes d'analyse métabolomique, où lesdites méthodes d'analyse métabolomique comprennent la génération de données d'intensité pour la quantification de métabolites endogènes par spectrométrie de masse (MS), en particulier les technologies MS comme MALDI (désorptionionisation laser assistée par matrice), ESI (ionisation par électrospray), APCI (ionisation chimique à pression atmosphérique), la spectroscopie de résonance magnétique nucléaire (RMN) ¹H, ¹³C et/ou ³¹P, éventuellement couplée à la MS, la détermination de concentrations de métabolites par utilisation de méthodes et/ou de technologies MS couplées à une séparation, en particulier la chromatographie liquide (LC-MS), la chromatographie en phase gazeuse (GC-MS) ou l'électrophorèse capillaire (CE-MS), où lesdits métabolites endogènes de référence sont choisis dans le groupe constitué par les suivants :
acides aminés, en particulier arginine, acide aspartique, citrulline, acide glutamique (glutamate), glutamine, leucine, isoleucine, histidine, ornithine, proline, phénylalanine, sérine, tryptophane, tyrosine, valine, kynurénine ;
acides phénylthiocarbamylaminés (acides PTC-aminés), en particulier PCT-arginine, PTC-glutamine, PTC-histidine, PTC-méthionine, PTC-ornithine, PTC-phénylalanine, PTC-proline, PTC-sérine, PTC-tryptophane, PTC-tyrosine, PTC-valine ; diméthylarginine, en particulier N,N-diméthyl-L-arginine ;
acides carboxyliques, nommément acide 15(S)-hydroxy-5Z, 8Z, 11Z, 13E-éicosatétraénoïque [acide (5Z, 8Z, 11Z, 13E, 15S)-15-hydroxyicosa-5, 8, 11, 13-tétraénoïque], acide succinique (succinate) ;
carnitine ; acylcarnitines comportant entre 1 et 20 atomes de carbone au niveau du résidu acyle ; acylcarnitines comportant entre 3 et 20 atomes de carbone au niveau du résidu acyle et entre 1 et 4 doubles liaisons au niveau du résidu acyle ; acylcarnitines comportant entre 1 et 20 atomes de carbone au niveau du résidu acyle et comportant entre 1 et 3 groupements OH au niveau du résidu acyle ; acylcarnitines comportant entre 3 et 20 atomes de carbone au niveau du résidu acyle avec 1 à 4 doubles liaisons et 1 à 3 groupements OH au niveau du résidu acyle ;
phospholipides, en particulier lysophosphatidylcholines (monoacylphosphatidylcholines) comportant entre 1 et 30 atomes de carbone au niveau du résidu acyle ; lysophosphatidylcholines comportant entre 3 et 30 atomes de carbone au niveau du résidu acyle et entre 1 et 6 doubles liaisons au niveau du résidu acyle ;
phosphatidylcholines (diacylphosphatidylcholines) comportant au total entre 1 et 50 atomes de carbone au niveau des résidus acyle ; phosphatidylcholines comportant au total entre 3 et 50 atomes de carbone au niveau des résidus acyle et au total entre 1 et 8 doubles liaisons au niveau des résidus acyle ;
sphingolipides, en particulier
sphingomyélines comportant un nombre total d'atomes de carbone au niveau des chaînes acyle compris entre 10 et 30 ; sphingomyélines comportant un nombre total d'atomes de carbone au niveau des chaînes acyle compris entre 10 et 30 et entre 1 et 5 doubles liaisons ; hydroxysphingomyélines comportant un nombre total d'atomes de carbone au niveau des résidus acyle compris entre 10 et 30 ;
hydroxysphingomyélines comportant un nombre total d'atomes de carbone au niveau des résidus acyle compris entre 10 et 30 et entre 1 et 5 doubles liaisons ;
prostaglandines, nommément 6-céto-prostaglandine F1 alpha, prostaglandine D2 ;
putrescine.

13. Utilisation selon la revendication 12, où lesdits métabolites endogènes de référence sont choisis dans le groupe constitué par les suivants :
acide 15(S)-hydroxy-5Z, 8Z, 11Z, 13E-éicosatétraénoïque
6-céto-Prostaglandine F1 alpha
Diméthylarginine asymétrique
Arginine
PTC-Arginine
Acide aspartique
Carnitine (libre)
Décanoylcarnitine [Caprylcarnitine] (Fumarylcarnitine)
Décénoylcarnitine
Décadiénoylcarnitine
Dodécanoylcarnitine [Laurylcarnitine]
Dodécénoylcarnitine
Dodécanedioylcarnitine
Tétradécanoylcarnitine
Tétradécénoylcarnitine [Myristoléylcarnitine]
3-Hydroxytétradécénoylcarnitine
[3-Hydroxymyristoléylcarnitine]
3-Hydroxytétradécadiénoylcarnitine
3-Hydroxytétradécanoylcarnitine [Hydroxymyristylcarnitine]
Hexadécénoylcarnitine [Palmitoléylcarnitine]
3-Hydroxyhexadécénoylcarnitine [3-Hydroxypalmitoléylcarnitine]
Hexadécadiénoylcarnitine
3-Hydroxyhexadécadiénoylcarnitine
3-Hydroxyhexadécanoylcarnitine [3-Hydroxypalmitoylcarnitine]
Octadécanoylcarnitine [Stéarylcarnitine]
Octadécénoylcarnitine [Oléylcarnitine]
3-Hydroxyoctadécénoylcarnitine [3-Hydroxyoléylcarnitine]
Acétylcarnitine
Propénoylcarnitine
Hydroxypropionylcarnitine
Buténoylcarnitine
3-Hydroxybutyrylcarnitine / Malonylcarnitine
Isovalérylcarnitine / 2-Méthylbutyrylcarnitine / Valérylcarnitine
Tiglylcarnitine / 3-Méthyl-crotonylcarnitine
Glutarylcarnitine / Hydroxycaproylcarnitine
Glutarylcarnitine / Hydroxycaproylcarnitine
Méthylglutarylcarnitine
3-Hydroxyisovalérylcarnitine / 3-Hydroxy-2-méthylbutyryle Hexanoylcarnitine [Caproylcarnitine]
Hexénoylcarnitine
Pimélylcarnitine
Octanoylcarnitine [Caprylylcarnitine]
Octénoylcarnitine
Nonanoylcarnitine [Pelargonylcarnitine]
Citrulline
Créatinine
Glutamine
PTC-Glutamine
Glutamate
Histidine
PTC-Histidine
Kynurénine
Leucine
Lysophosphatidylcholine avec résidu acyle C14:0
Lysophosphatidylcholine avec résidu acyle C16:0
Lysophosphatidylcholine avec résidu acyle C16:1
Lysophosphatidylcholine avec résidu acyle C18:0
Lysophosphatidylcholine avec résidu acyle C18:1
Lysophosphatidylcholine avec résidu acyle C18:2
Lysophosphatidylcholine avec résidu acyle C20:3
Lysophosphatidylcholine avec résidu acyle C20:4
Lysophosphatidylcholine avec résidu acyle C24:0
Lysophosphatidylcholine avec résidu acyle C26:0
Lysophosphatidylcholine avec résidu acyle C26:1
Lysophosphatidylcholine avec résidu acyle C28:0
Lysophosphatidylcholine avec résidu acyle C28:1
Lysophosphatidylcholine avec résidu acyle C6:0
PTC-Méthionine
Ornithine
PTC-Ornithine
Phosphatidylcholine avec somme des résidus diacyle C24:0
Phosphatidylcholine avec somme des résidus diacyle C26:0
Phosphatidylcholine avec somme des résidus diacyle C28:1
Phosphatidylcholine avec somme des résidus diacyle C30:0
Phosphatidylcholine avec somme des résidus diacyle C30:2
Phosphatidylcholine avec somme des résidus diacyle C32:0
Phosphatidylcholine avec somme des résidus diacyle C32:1
Phosphatidylcholine avec somme des résidus diacyle C32:2
Phosphatidylcholine avec somme des résidus diacyle C32:3
Phosphatidylcholine avec somme des résidus diacyle C34:1
Phosphatidylcholine avec somme des résidus diacyle C34:2
Phosphatidylcholine avec somme des résidus diacyle C34:3
Phosphatidylcholine avec somme des résidus diacyle C34:4
Phosphatidylcholine avec somme des résidus diacyle C36:0
Phosphatidylcholine avec somme des résidus diacyle C36:1
Phosphatidylcholine avec somme des résidus diacyle C36:2
Phosphatidylcholine avec somme des résidus diacyle C36:3
Phosphatidylcholine avec somme des résidus diacyle C36:4
Phosphatidylcholine avec somme des résidus diacyle C36:5
Phosphatidylcholine avec somme des résidus diacyle C36:6
Phosphatidylcholine avec somme des résidus diacyle C38:0
Phosphatidylcholine avec somme des résidus diacyle C38:1
Phosphatidylcholine avec somme des résidus diacyle C38:3
Phosphatidylcholine avec somme des résidus diacyle C38:4
Phosphatidylcholine avec somme des résidus diacyle C38:5
Phosphatidylcholine avec somme des résidus diacyle C38:6
Phosphatidylcholine avec somme des résidus diacyle C42:1
Phosphatidylcholine avec somme des résidus diacyle C40:3
Phosphatidylcholine avec somme des résidus diacyle C40:4
Phosphatidylcholine avec somme des résidus diacyle C40:5
Phosphatidylcholine avec somme des résidus diacyle C40:6
Phosphatidylcholine avec somme des résidus diacyle C42:0
Phosphatidylcholine avec somme des résidus diacyle C42:1
Phosphatidylcholine avec somme des résidus diacyle C42:2
Phosphatidylcholine avec somme des résidus diacyle C42:4
Phosphatidylcholine avec somme des résidus diacyle C42:5
Phosphatidylcholine avec somme des résidus diacyle C42:6
Phosphatidylcholine avec somme des résidus acyl-alkyle C30:0
Phosphatidylcholine avec somme des résidus acyl-alkyle C30:1
Phosphatidylcholine avec somme des résidus acyl-alkyle C34:0
Phosphatidylcholine avec somme des résidus acyl-alkyle C34:1
Phosphatidylcholine avec somme des résidus acyl-alkyle C34:2
Phosphatidylcholine avec somme des résidus acyl-alkyle C34:3
Phosphatidylcholine avec somme des résidus acyl-alkyle C36:0
Phosphatidylcholine avec somme des résidus acyl-alkyle C36:1
Phosphatidylcholine avec somme des résidus acyl-alkyle C36:2
Phosphatidylcholine avec somme des résidus acyl-alkyle C36:3
Phosphatidylcholine avec somme des résidus acyl-alkyle C36:4
Phosphatidylcholine avec somme des résidus acyl-alkyle C38:0
Phosphatidylcholine avec somme des résidus acyl-alkyle C38:1
Phosphatidylcholine avec somme des résidus acyl-alkyle C38:2
Phosphatidylcholine avec somme des résidus acyl-alkyle C38:3
Phosphatidylcholine avec somme des résidus acyl-alkyle C38:4
Phosphatidylcholine avec somme des résidus acyl-alkyle C38:5
Phosphatidylcholine avec somme des résidus acyl-alkyle C38:6
Phosphatidylcholine avec somme des résidus acyl-alkyle C40:0
Phosphatidylcholine avec somme des résidus acyl-alkyle C40:1
Phosphatidylcholine avec somme des résidus acyl-alkyle C40:2
Phosphatidylcholine avec somme des résidus acyl-alkyle C40:3
Phosphatidylcholine avec somme des résidus acyl-alkyle C40:4
Phosphatidylcholine avec somme des résidus acyl-alkyle C40:5
Phosphatidylcholine avec somme des résidus acyl-alkyle C40:6
Phosphatidylcholine avec somme des résidus acyl-alkyle C42:0
Phosphatidylcholine avec somme des résidus acyl-alkyle C42:1
Phosphatidylcholine avec somme des résidus acyl-alkyle C42:3
Phosphatidylcholine avec somme des résidus acyl-alkyle C42:4
Phosphatidylcholine avec somme des résidus acyl-alkyle C42:5
Phosphatidylcholine avec somme des résidus acyl-alkyle C44:5
Phosphatidylcholine avec somme des résidus acyl-alkyle C44:6
Prostaglandine D2
Phénylalanine
PTC-Phénylalanine
Proline
PTC-Proline
Putrescine
Sérine
PTC-Sérine
Hydroxysphingomyéline avec somme des résidus acyle C14:1
Hydroxysphingomyéline avec somme des résidus acyle C16:1
Hydroxysphingomyéline avec somme des résidus acyle C22:1
Hydroxysphingomyéline avec somme des résidus acyle C22:2
Hydroxysphingomyéline avec somme des résidus acyle C24:1
Sphingomyéline avec somme des résidus acyle C16:0
Sphingomyéline avec somme des résidus acyle C16:1 Sphingomyéline avec somme des résidus acyle C18:0 Sphingomyéline avec résidu acyle C18:1
Sphingomyéline avec somme des résidus acyle C20:2
Sphingomyéline avec somme des résidus acyle C22:3
Sphingomyéline avec somme des résidus acyle C24:0
Sphingomyéline avec somme des résidus acyle C24:1
Sphingomyéline avec somme des résidus acyle C26:0
Sphingomyéline avec somme des résidus acyle C26:1
Acide succinique (succinate)
Diméthylarginine totale : somme ADMA + SDMA
Tryptophane
PTC-Tryptophane
Tyrosine
Valine
PTC-Valine
Leucine + Isoleucine
où la désignation « résidu Cn:m » ou « somme des résidus Cn:m » représente la longueur de chaîne du ou des résidus acyle/alkyle, n représente le nombre total d'atomes de carbone dans le ou les résidus acyle/alkyle, et m représente le nombre total de doubles liaisons dans le ou les résidus.

14. Utilisation selon la revendication 13, où lesdits métabolites endogènes de référence présentent une stabilité selon les mesures de stabilité statistique choisies dans le groupe constitué par le coefficient de variation (CV) des données d'intensité brutes, l'écart-type (SD) des données d'intensité logarithmique, la mesure de stabilité (M) de l'algorithme geNorm ou la valeur de mesure de stabilité (rho) de l'algorithme NormFinder.

15. Utilisation selon l'une quelconque des revendications 12 à 14, où lesdits métabolites endogènes de référence présentent une stabilité selon au moins 2 mesures de stabilité statistique choisies dans le groupe constitué par le coefficient de variation (CV) des données d'intensité brutes, l'écart-type (SD) des données d'intensité logarithmique, la mesure de stabilité (M) de l'algorithme geNorm ou la valeur de mesure de stabilité (rho) de l'algorithme NormFinder, *et*/*ou* de tels métabolites endogènes de référence étant en particulier choisis dans le groupe constitué par les suivants :
PTC-Arginine
Carnitine (libre)
Décénoylcarnitine
Décanoylcarnitine [Caprylcarnitine] (Fumarylcarnitine)
Dodécénoylcarnitine
Dodécanedioylcarnitine
Dodécanoylcarnitine [Laurylcarnitine]
Tétradécanoylcarnitine
3-Hydroxytétradécanoylcarnitine [Hydroxymyristylcarnitine]
Hexadécénoylcarnitine [Palmitoléylcarnitine]
3-Hydroxyhexadécénoylcarnitine [3-Hydroxypalmitoléylcarnitine]
3-Hydroxyhexadécadiénoylcarnitine
3-Hydroxyhexadécanoylcarnitine [3-Hydroxypalmitoylcarnitine]
3-Hydroxyoctadécénoylcarnitine [3-Hydroxyoléylcarnitine]
Propénoylcarnitine
Hydroxypropionylcarnitine
3-Hydroxybutyrylcarnitine / Malonylcarnitine
Méthylglutarylcarnitine
3-Hydroxyisovalérylcarnitine / 3-Hydroxy-2-méthylbutyryle
Hexénoylcarnitine
Hexanoylcarnitine [Caproylcarnitine]
Pimélylcarnitine
Octénoylcarnitine
Octanoylcarnitine [Caprylylcarnitine]
Glutamine
PTC-Glutamine
PTC-Histidine
Lysophosphatidylcholine avec résidu acyle C14:0
Lysophosphatidylcholine avec résidu acyle C26:0
Lysophosphatidylcholine avec résidu acyle C26:1
Lysophosphatidylcholine avec résidu acyle C28:0
Lysophosphatidylcholine avec résidu acyle C28:1
Phosphatidylcholine avec somme des résidus diacyle C24:0
Phosphatidylcholine avec somme des résidus diacyle C26:0
Phosphatidylcholine avec somme des résidus diacyle C30:0
Phosphatidylcholine avec somme des résidus diacyle C30:2
Phosphatidylcholine avec somme des résidus diacyle C32:2
Phosphatidylcholine avec somme des résidus diacyle C34:2
Phosphatidylcholine avec somme des résidus diacyle C36:0
Phosphatidylcholine avec somme des résidus diacyle C36:2
Phosphatidylcholine avec somme des résidus diacyle C36:4
Phosphatidylcholine avec somme des résidus diacyle C38:0
Phosphatidylcholine avec somme des résidus diacyle C38:1
Phosphatidylcholine avec somme des résidus diacyle C42:1
Phosphatidylcholine avec somme des résidus diacyle C42:0
Phosphatidylcholine avec somme des résidus diacyle C42:5
Phosphatidylcholine avec somme des résidus diacyle C42:6
Phosphatidylcholine avec somme des résidus acyl-alkyle C30:1
Phosphatidylcholine avec somme des résidus acyl-alkyle C34:1
Phosphatidylcholine avec somme des résidus acyl-alkyle C36:0
Phosphatidylcholine avec somme des résidus acyl-alkyle C38:1
Phosphatidylcholine avec somme des résidus acyl-alkyle C38:4
Phosphatidylcholine avec somme des résidus acyl-alkyle C38:6
Phosphatidylcholine avec somme des résidus acyl-alkyle C40:0
Phosphatidylcholine avec somme des résidus acyl-alkyle C40:1
Phosphatidylcholine avec somme des résidus acyl-alkyle C40:5
Phosphatidylcholine avec somme des résidus acyl-alkyle C40:6
Phosphatidylcholine avec somme des résidus acyl-alkyle C42:0
Phosphatidylcholine avec somme des résidus acyl-alkyle C42:5
Phosphatidylcholine avec somme des résidus acyl-alkyle C44:6
Phénylalanine
PTC-Phénylalanine
Proline
Sphingomyéline avec somme des résidus acyle C16:0
Sphingomyéline avec somme des résidus acyle C16:1
Sphingomyéline avec somme des résidus acyle C18:0
Sphingomyéline avec somme des résidus acyle C18:1
Sphingomyéline avec somme des résidus acyle C20:2
Sphingomyéline avec somme des résidus acyle C24:0
Sphingomyéline avec somme des résidus acyle C24:1
Hydroxysphingomyéline avec somme des résidus acyle C14:1
Hydroxysphingomyéline avec somme des résidus acyle C16:1
Hydroxysphingomyéline avec somme des résidus acyle C22:2
Hydroxysphingomyéline avec somme des résidus acyle C24:1
Acide succinique (succinate)
PTC-Tryptophane
Valine
PTC-Valine
Leucine + Isoleucine
où la désignation « résidu Cn:m » ou « somme des résidus Cn:m » représente la longueur de chaîne du ou des résidus acyle/alkyle, n représente le nombre total d'atomes de carbone dans le ou les résidus acyle/alkyle, et m représente le nombre total de doubles liaisons dans le ou les résidus.

16. Utilisation selon l'une quelconque des revendications 12 à 15, où lesdits métabolites endogènes de référence présentent une stabilité selon au moins 3 mesures de stabilité statistique choisies dans le groupe constitué par le coefficient de variation (CV) des données d'intensité brutes, l'écart-type (SD) des données d'intensité logarithmique, la mesure de stabilité (M) de l'algorithme geNorm ou la valeur de mesure de stabilité (rho) de l'algorithme NormFinder, *et*/*ou* de tels métabolites endogènes de référence étant en particulier choisis dans le groupe constitué par les suivants :
Carnitine (libre)
Décanoylcarnitine [Caprylcarnitine] (Fumarylcarnitine) Dodécanedioylcarnitine
Dodécanoylcarnitine [Laurylcarnitine]
Hexadécénoylcarnitine [Palmitoléylcarnitine]
3-Hydroxyhexadécénoylcarnitine [3-Hydroxypalmitoléylcarnitine]
3-Hydroxyhexadécanoylcarnitine [3-Hydroxypalmitoylcarnitine]
Propénoylcarnitine
Hydroxypropionylcarnitine
3-Hydroxybutyrylcarnitine / Malonylcarnitine
Méthylglutarylcarnitine
Hexanoylcarnitine [Caproylcarnitine]
Pimélylcarnitine
Octénoylcarnitine
Octanoylcarnitine [Caprylylcarnitine]
Glutamine
PTC-Glutamine
PTC-Histidine
Lysophosphatidylcholine avec résidu acyle C14:0
Lysophosphatidylcholine avec résidu acyle C26:0
Lysophosphatidylcholine avec résidu acyle C26:1
Lysophosphatidylcholine avec résidu acyle C28:1
Phosphatidylcholine avec somme des résidus diacyle C26:0
Phosphatidylcholine avec somme des résidus diacyle C32:2
Phosphatidylcholine avec somme des résidus diacyle C36:0
Phosphatidylcholine avec somme des résidus diacyle C38:0
Phosphatidylcholine avec somme des résidus diacyle C42:1
Phosphatidylcholine avec somme des résidus diacyle C42:5
Phosphatidylcholine avec somme des résidus diacyle C42:6
Phosphatidylcholine avec somme des résidus acyl-alkyle C38:4
Phosphatidylcholine avec somme des résidus acyl-alkyle C40:0
Phosphatidylcholine avec somme des résidus acyl-alkyle C42:0
Phosphatidylcholine avec somme des résidus acyl-alkyle C42:5
Phosphatidylcholine avec somme des résidus acyl-alkyle C44:6
Sphingomyéline avec somme des résidus acyle C16:0
Sphingomyéline avec somme des résidus acyle C16:1
Sphingomyéline avec somme des résidus acyle C20:2
Sphingomyéline avec somme des résidus acyle C24:0 Hydroxysphingomyéline avec somme des résidus acyle C24:1 Valine
PTC-Valine
où la désignation « résidu Cn:m » ou « somme des résidus Cn:m » représente la longueur de chaîne du ou des résidus acyle/alkyle, n représente le nombre total d'atomes de carbone dans le ou les résidus acyle/alkyle, et m représente le nombre total de doubles liaisons dans le ou les résidus.

17. Utilisation selon l'une quelconque des revendications 12 to 16, où lesdits métabolites endogènes de référence présentent une stabilité selon toutes les mesures de stabilité statistique choisies dans le groupe constitué par le coefficient de variation (CV) des données d'intensité brutes, l'écart-type (SD) des données d'intensité logarithmique, la mesure de stabilité (M) de l'algorithme geNorm ou la valeur de mesure de stabilité (rho) de l'algorithme NormFinder, et/ou de tels métabolites endogènes de référence étant en particulier la Phosphatidylcholine avec somme des résidus diacyle C42:6, où la désignation « C42:6 » représente la longueur de chaîne des résidus acyle, 42 représente le nombre total d'atomes de carbone dans les résidus acyle, et 6 représente le nombre total de doubles liaisons dans les résidus.

18. Utilisation selon l'une quelconque des revendications 12 à 17, où ladite multitude de métabolites endogènes comprend entre 2 et 80, en particulier entre 2 et 60, préférentiellement entre 2 et 50, préféré entre 2 et 30, plus préféré entre 2 et 10, particulièrement préféré entre 2 et 10, préférentiellement entre 3 et 5 métabolites endogènes.
